# EUROPEAN PATENT SPECIFICATION

(11) **EP 2 837 351 B1**
(45) Date of publication and mention of the grant of the patent: **30.05.2018**
(21) Application number: 14179712.6
(22) Date of filing: 18.04.2008
(51) Int. Cl.: A61B 18/18, A61N 5/02

(54) **Systems for creating an effect using microwave energy to specified tissue**
Systeme zur Erzeugung eines Effekts mit Verwendung von Mikrowellenenergie an spezifischem Gewebe
Systèmes pour créer un effet en utilisant une énergie micro-onde sur un tissu spécifié

(30) Priority: 19.04.2007 US 912899 P; 12.12.2007 US 13274 P; 17.04.2008 US 45937 P
(43) Date of publication of application: 18.02.2015
(62) Divisional of application: 08746358.4
(73) Proprietor: Miramar Labs, Inc., Sunnyvale, CA 94085 (US)
(72) Inventor: Deem, Mark E., Mountain View, CA 94041 (US); Francis, Dan, Mountain View, CA 94040 (US); Johnson, Jessi Ernest, Sunnyvale, CA 94085 (US); Kim, Steven, Los Altos, CA 94024 (US); Salamini, Alexey, San Francisco, CA 94110 (US); Su, Ted, Sunnyvale, CA 94085 (US); Smith, Peter, Kirkcaldy, Fife KY2 5NB (GB); Hallock, Dan, Redwood City, CA 94063 (US)
(74) Representative: Murray, David Craig

(56) References cited:
- WO-A1-99/46005
- WO-A1-2007/038567
- WO-A2-2006/117682
- US-B1- 6 208 903

## Description

### BACKGROUND

### Field of the Invention

The field of the invention relates systems for non-invasive delivery of microwave therapy. In particular, the field of the invention relates to systems for non-invasively delivering microwave energy to the epidermal, dermal and subdermal tissue of a patient to achieve various therapeutic and/or aesthetic results.

WO 2007/038567 (Candela Corporation) relates to a system for delivering a beam of radiation to a subcutaneous fat region.

US 6,208,903 (Richards et al.) relates to an apparatus and method for non-invasive removal of target tissue, used for reducing fatty tissue within the subcutaneous fatty layer.

WO 2006/117682 (Reneware Medical System Sa) relates to RF or microwave device for biological samples or tissue.

WO 99/46005 (Palomar Medical Technologies Inc) relates to a system for treating a selected dermatologic problem.

### SUMMARY

A system for the application of microwave energy to skin according to the appended claims.

### BRIEF DESCRIPTION OF THE DRAWINGS

Figure 1 is an illustration of a cross-section of human tissue structures.
Figure 2 illustrates a system for generating and controlling microwave energy according to one embodiment of the invention.
Figure 3 illustrates a system for delivering microwave energy according to one embodiment of the invention.
Figure 4 is a side perspective view of a microwave applicator of a system according to one embodiment of the invention
Figure 5 is a top perspective view of a microwave applicator of a system according to one embodiment of the invention.
Figure 6 is a front view of a microwave applicator of a system according to one embodiment of the invention.
Figure 7 is a front view of a tissue head for use with a microwave applicator of a system according to one embodiment of the invention.
Figure 8 is a cut away view of a tissue head of a system according to one embodiment of the invention.
Figure 9 is a side cutaway view of a microwave applicator of a system according to one embodiment of the invention.
Figure 10 is a top perspective partial cutaway view of a microwave applicator of a system according to one embodiment of the invention.
Figure 11 is a side partial cutaway view of a microwave applicator of a system according to one embodiment of the invention.
Figure 12 is a cutaway view of a tissue head and antenna of a system according to one embodiment of the invention.
Figure 13 is a cutaway view of a tissue head and antenna of a system according to one embodiment of the invention.
Figure 14 is a cutaway view of a tissue head, antenna and field spreader of a system according to one embodiment of the invention.
Figure 15 is a cutaway view of a tissue head, antenna and field spreader of a system according to one embodiment of the invention.
Figure 16 is a cutaway view of a tissue head, antenna and field spreader according to one embodiment of the invention.
Figure 17 is a cutaway view of a tissue head, antenna and field spreader of a system according to one embodiment of the invention.
Figure 18 is a cutaway view of a tissue head, antenna and field spreader of a system according to one embodiment of the invention.
Figure 19 is a cutaway view of a tissue head, antenna and field spreader, with tissue engaged, of a system according to one embodiment of the invention.
Figure 20 is a cutaway view of a tissue head and antenna, and with tissue engaged, of a system according to one embodiment of the invention.
Figure 21 illustrates a tissue head including a plurality of waveguide antennas of a system according to one embodiment of the invention.
Figure 22 illustrates a tissue head including a plurality of waveguide antennas of a system according to one embodiment of the invention.
Figure 23 illustrates a tissue head including a plurality of waveguide antennas of a system according to one embodiment of the invention.
Figure 24 illustrates a disposable tissue head for use with an applicator of a system according to one embodiment of the invention.
Figure 25 illustrates a disposable tissue head for use with an applicator of a system according to one embodiment of the invention.
Figure 26 illustrates a tissue profile generated using a system according to one embodiment of the invention.
Figure 27 illustrates a tissue profile generated using a system according to one embodiment of the invention.
Figure 28 illustrates a tissue profile generated using a system according to one embodiment of the invention.
Figure 29 illustrates a tissue profile generated using a system according to one embodiment of the invention.
Figure 30 illustrates a tissue profile generated using a system according to one embodiment of the invention.
Figure 31 illustrates a tissue profile generated using a system according to one embodiment of the invention.
Figure 32 illustrates a tissue profile generated using a system according to one embodiment of the invention.
Figure 33 illustrates a tissue profile generated using a system according to one embodiment of the invention.
Figure 34 illustrates a tissue profile generated using a system according to one embodiment of the invention.
Figure 35 illustrates a tissue profile generated using a system according to one embodiment of the invention.
Figure 36 illustrates a tissue profile generated using a system according to one embodiment of the invention.
Figure 37 illustrates a tissue profile generated using a system according to one embodiment of the invention.
Figure 38 illustrates a tissue profile generated using a system according to one embodiment of the invention.
Figure 39 illustrates a tissue profile generated using a system according to one embodiment of the invention.
Figure 40 illustrates a tissue profile generated using a system according to one embodiment of the invention.
Figure 41 illustrates a tissue profile generated using a system according to one embodiment of the invention.
Figure 42 illustrates a tissue profile generated using a system according to one embodiment of the invention.
Figure 43 illustrates a tissue profile generated using a system according to one embodiment of the invention.
Figure 44 illustrates a tissue profile generated using a system according to one embodiment of the invention.
Figure 45 illustrates a tissue profile generated using a system according to one embodiment of the invention.
Figure 46 illustrates a tissue profile generated using a system according to one embodiment of the invention.
Figure 47 illustrates a tissue profile generated using a system according to one embodiment of the invention.
Figure 48 illustrates a tissue profile generated using a system according to one embodiment of the invention.
Figure 49 illustrates a tissue profile generated using a system according to one embodiment of the invention.
Figure 50 illustrates a tissue profile generated using a system according to one embodiment of the invention.
Figure 51 illustrates a tissue profile generated using a system according to one embodiment of the invention.

### DESCRIPTION

When skin is irradiated with electromagnetic radiation, such as, for example, microwave energy, energy is absorbed by each layer of tissue as the electromagnetic radiation passes through the tissue. The amount of energy absorbed by each tissue layer is a function of a number of variables. Some of the variables which control the amount of energy absorbed in each tissue layer include, the power of the electromagnetic radiation which reaches each layer; the amount of time each layer is irradiated; the electrical conductivity or loss tangent of the tissue in each layer and the power deposition pattern from the antenna radiating the electromagnetic energy. The power which reaches a particular layer of tissue is a function of a number of variables. Some of the variables which control the power reaching a particular layer of tissue include the power impinging upon the surface of the skin and the frequency of the electromagnetic radiation. For example, at higher frequencies the penetration depth of the electromagnetic signal is shallow and most power is deposited in the upper layers of tissue, at lower frequencies, larger penetration depths result in power deposition in both upper and lower tissue layers.

Heat may be generated in tissue by a number of mechanisms. Some of the mechanisms for generating heat in tissue include resistive heating, dielectric heating and thermal conduction. Resistive heating occurs when electrical currents are generated in the tissue, resulting in resistive losses. Tissue may be resistively heated using, for example, mono-polar or bi-polar RF energy. Dielectric heating occurs when electromagnetic energy induces an increased physical rotation of polar molecules that converts microwave energy into heat. Tissue may be dielectrically heated by, for example, irradiating the tissue with electromagnetic energy in the microwave frequency band. As used herein, microwave frequency band or microwave frequencies may refer to, for example, electromagnetic energy at frequencies which are suitable for inducing dielectric heating in tissue when that tissue is irradiated using an external antenna to transmit the electromagnetic radiation. Such frequencies may be in the range of, for example, from approximately 100 Megahertz (MHz) to 30 Gigahertz (GHz). Whether tissue is heated by resistive heating or by dielectric heating, heat generated in one region of tissue may be transmitted to adjacent tissue by, for example, thermal conduction, thermal radiation or thermal convection.

When a microwave signal is radiated into a lossy dielectric material such as water, the energy of the signal is absorbed and converted to heat as it penetrates the material. This heat is primarily generated by induced physical rotation of molecules when subjected to the microwave signal. The efficiency of the conversion of microwave energy into heat for a given material can be quantified by the energy dissipation factor, or loss-tangent (tanδ). The loss-tangent varies as a function of material composition and the frequency of the microwave signal. Water has a large loss-tangent and heats efficiently when irradiated with microwave energy. Since all biological tissue has some water content, and thus is lossy, it can be heated using microwave energy. Different tissue types have varying amounts of water content, with muscle and skin having a relatively high water content and fat and bone having significantly less water content. Microwave heating is generally more efficient in high water content tissues.

The application of RF energy, which is conducted through the surface of the skin, or microwave energy, which is radiated through the surface of the skin, to heat tissue below the skin surface generally results in a temperature gradient having a peak at the surface of the skin and decreasing with increasing depth into the tissue. That is, the hottest point is generally found at or near the surface of the skin. The temperature gradient results from the power lost by the electromagnetic radiation as it moves through the tissue. The power loss density profile generally peaks in tissue at the skin surface and declines as the electromagnetic radiation moves through the tissue, regardless of the radiated power or frequency of the electromagnetic radiation. Power loss density is measured in watts per cubic meter. An equivalent characterization of power deposition in tissue is the Specific Absorption Rate (SAR) which is measured in watts per kilogram. Specific absorption rate in tissue may be, for example, proportional to the square of the magnitude of electric field in tissue. For a fixed radiated power level the penetration depth will generally decrease as the frequency increases. Thus, as a general principal to heat tissue near the skin surface, such as, for example, the epidermis, without damage to deeper tissue layers one would generally select a higher frequency. Further, as a general principal, to heat tissue deep within the skin, such as, for example, in the hypodermis, or below the skin surface, such as, for example, in muscle tissue, one would generally select a lower frequency to ensure that sufficient energy reached the deeper tissue.

Where electromagnetic energy is being used to heat structures in tissue below the skin surface and it is desirable to limit or prevent damage to the skin surface or tissue adjacent the skin surface, it is possible to modify the temperature gradient to move the peak temperature deeper into the tissue. Temperature gradients within tissue may be modified to move the peak temperature into tissue below the skin surface by, for example, using external mechanisms to remove heat from tissue close to the skin surface. External mechanisms which remove heat from the surface of the skin may include, for example, heat sinks which cool the skin surface and adjacent layers. When external mechanisms are used to remove heat from the surface of the skin, the heat may be removed as the electromagnetic radiation deposits energy into that tissue. Thus, where external mechanisms are used to remove heat from the surface of the skin and adjoining layers, energy is still absorbed at substantially the same rate by tissue adjacent the skin surface (and the power loss density and SAR in the cooled tissue remain substantially constant and are not effected by cooling) but damage is prevented by removing heat faster than it can build up, preventing the temperature of the cooled tissue, for example, tissue adjacent the skin surface, from reaching a temperature where tissue damage occurs or a lesion could form.

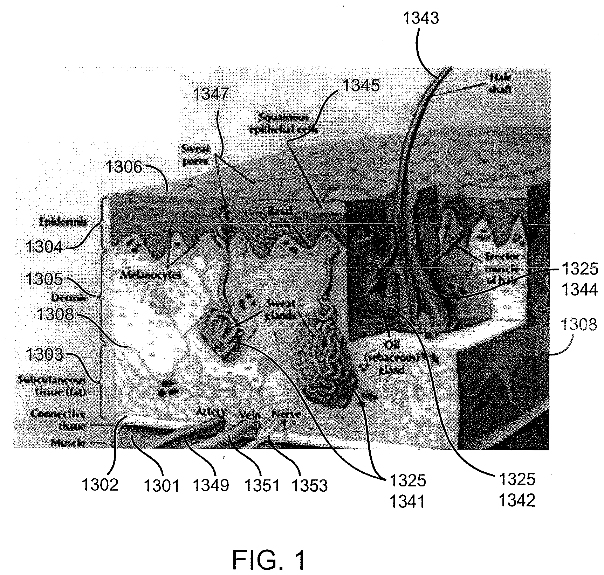

Figure 1 is an illustration of human tissue structure. In Figure 1, muscle tissue 1301 is connected to hypodermis 1303 by connective tissue 1302. Hypodermis 1303 is connected to dermis 1305 at interface 1308. Epidermis 1304 lies over dermis 1305. Skin surface 1306 lies over epidermis 1304 and includes squamous epithelial cells 1345 and sweat pores 1347. Tissue structures 1325 such as, for example, sweat glands 1341, sebaceous glands 1342 and hair follicles 1344, may be positioned throughout dermis 1305 and hypodermis 1303. Further, tissue structures 1325 and may be positioned such that they cross or interrupt interface 1308. Figure 1 further includes artery 1349, vein 1351 and nerve 1353. Hair follicle 1344 is attached to hair shaft 1343. Tissue structures such as, for example, apocrine glands, eccrine glands or hair follicles may be expected to have sizes in the range of, for example, between approximately 0.1 millimeter and two millimeters and may group together to form groups or structures having even larger sizes. As illustrated in Figure 1, interface 1308 may be expected to be a non-linear, non-continuous, rough interface which also includes many tissue structures and groups of tissue structures which cross and interrupt interface 1308. When modeling tissue layers such as, for example the dermis, it is difficult to accurately model the dielectric and/or conductivity characteristics of the tissue layers because of the variability from person to person and within body regions of individuals. In addition, the presence of tissue structures and of groups of tissue structures creates additional complexities. Assuming an average dielectric constant for a particular layer does not generally reflect the complexity of the actual tissue, however, it may be used as a starting point. For example, assuming a dielectric constant of, for example, approximately 66 for dermal tissue at 100 MHz, electromagnetic energy at the low end of the microwave range, would have a wavelength of approximately 370 millimeters. Assuming a dielectric constant of, for example, approximately 38 for dermal tissue at 6.0 GHz, electromagnetic energy would have a wavelength of approximately 8 millimeters in dermal tissue. Assuming a dielectric constant of, for example, approximately 18 for dermal tissue at 30 GHz, electromagnetic energy at the high end of the microwave range would have a wavelength of approximately 2.5 millimeters in dermal tissue. Thus, as frequency increases, the presence of rough, discontinuous interfaces between tissue regions and the presence of tissue structures will result in scattering of at least some of the signal when it encounters tissue structures or discontinuous tissue interfaces. For a fixed size tissue structure or discontinuity, scattering generally increases as wavelength decreases and becomes more pronounced when wavelength is within an order of magnitude of the size of tissue structures, groups of tissue structures or discontinuities in the interface.

When electromagnetic energy is transmitted through a medium having structures and interfaces, including interfaces between tissue layers, the electromagnetic energy will, depending upon the electrical and physical characteristics of the structures and interfaces, and the differences between electrical and physical characteristics of such structures and interfaces and surrounding tissue, be scattered and/or reflected by the structures and/or interfaces. When reflected electromagnetic waves interact with incident electromagnetic waves they may, under certain circumstances, combine to form a standing wave having one or more constructive interference peaks, such as, for example an E-field peak, and one or more interference minimums (also referred to as regions of destructive interference).

In modeling tissue for the purposes of the present discussion dermal tissue may be modeled to include a dermis and an epidermis. In modeling tissue for the purposes of the present discussion dermal tissue may be modeled to have a conductivity of approximately 4.5 siemens per meter at approximately 6 GHz. In modeling tissue for the purposes of the present discussion dermal tissue may be modeled to have a dielectric constant of approximately 40 at approximately 6 GHz. In modeling tissue for the purposes of the present discussion hypodermal tissue may be modeled to have a conductivity of approximately 0.3 siemens per meter at approximately 6 GHz. In modeling tissue for the purposes of the present discussion hypodermal tissue may be modeled to have a dielectric constant of approximately 5 at approximately 6 GHz.

### SYSTEMS AND APPARATUSES

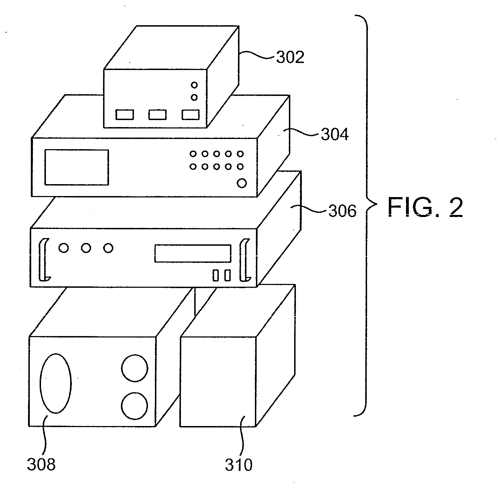

Figures 2 through 25 and 48 through 51 illustrate systems and components of systems which may be used to generate heat in selected tissue regions.

Figures 2 through 25 and 48 through 51 illustrate systems and components of systems which may be used to generate predetermined specific absorption rate profiles in selected tissue regions.

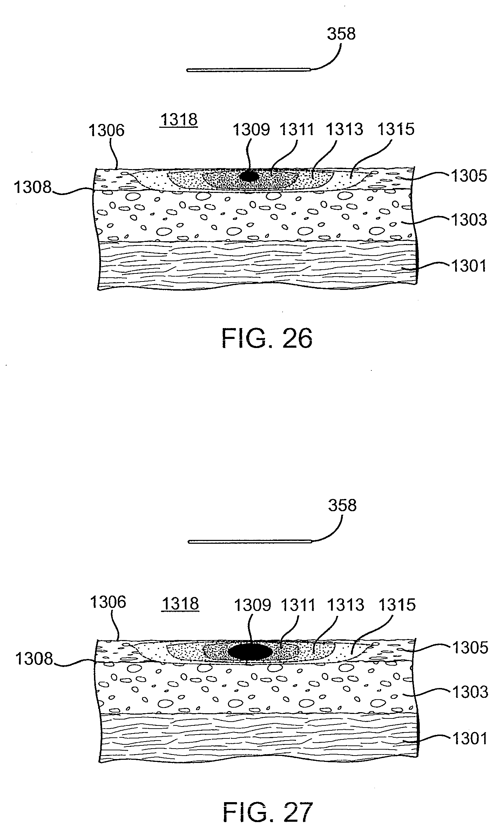

Figures 2 through 25 and 48 through 51 illustrate systems and components of systems which may be used to generate predetermined specific absorption rate profiles such as, for example, the specific absorption rate profiles illustrated in Figures 26 through 51.

Figures 2 through 25 and 48 through 51 illustrate systems and components of systems which may be used to generate predetermined power loss density profiles in selected tissue regions.

Figures 2 through 25 and 48 through 51 illustrate systems and components of systems which may be used to generate predetermined power loss density profiles such as, for example, the power loss density profiles illustrated in Figures 26 through 51.

Figures 2 through 25 and 48 through 51 illustrate systems and components of systems which may be used to generate predetermined temperature profiles in selected tissue regions.

Figures 2 through 25 and 48 through 51 illustrate systems and components of systems which may be used to generate predetermined temperature profiles such as, for example, the temperature profiles illustrated in Figures 26 through 51.

Figures 2 through 25 and 48 through 51 illustrate systems and components of systems which may be used to create lesions in selected tissue regions.

Figures 2 through 25 and 48 through 51 illustrate systems and components of systems which may be used to create lesions in selected regions by generating specific absorption rate profiles with a peak in the selected tissue regions.

Figures 2 through 25 and 48 through 51 illustrate systems and components of systems which may be used to create lesions in selected tissue by generating specific absorption rate profiles such as, for example, the specific absorption rate profiles illustrated in Figures 26 through 51 wherein the lesion is created in region of the tissue corresponding to the peak specific absorption rate.

Figures 2 through 25 and 48 through 51 illustrate systems and components of systems which may be used to create lesions in selected regions by generating power loss density profiles with a peak in the selected tissue regions.

Figures 2 through 25 and 48 through 51 illustrate systems and components of systems which may be used to create lesions in selected tissue by generating power loss density profiles such as, for example, the power loss density profiles illustrated in Figures 26 through 51 wherein the lesion is created in region of the tissue corresponding to the peak power loss density.

Figures 2 through 25 and 48 through 51 illustrate systems and components of systems which may be used to create lesions in selected regions by generating temperature profiles with a peak in the selected tissue regions.

Figures 2 through 25 and 48 through 51 illustrate systems and components of systems which may be used to create lesions in selected tissue by generating temperature profiles such as, for example, the temperature profiles illustrated in Figures 26 through 51 wherein the lesion is created in region of the tissue corresponding to the peak temperature.

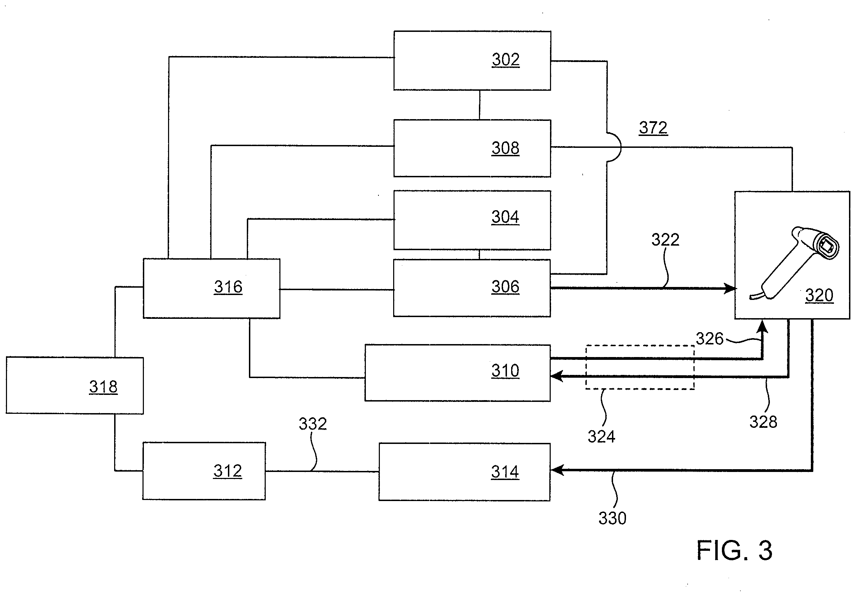

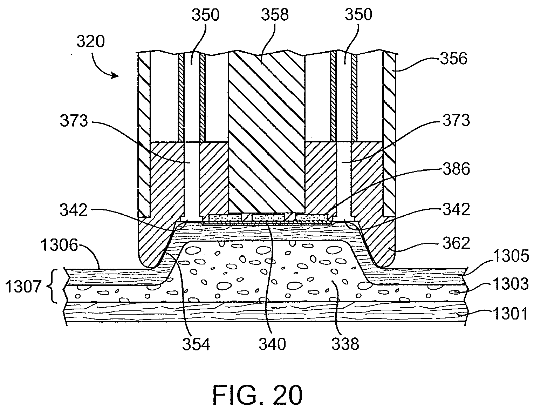

Further non-limiting examples of microwave systems and apparatuses that may be used and configured as described above can be found, for example, at Figs. 3-7C and pp. 8-13 of U.S. Provisional App. No. 60/912,899; and Figs. 3-9 and 20-26 and pp. 34-48 and Figs. 20-26 of U.S. Provisional App. No. 61/013,274

Figure 2 illustrates a system for generating and controlling microwave energy according to one embodiment of the invention. In the system illustrated in Figure 2, controller 302 may be, for example, a custom digital logic timer controller module that controls the delivery of microwave energy generated by signal generator 304 and amplified by amplifier 306. Controller 302 may also control a solenoid valve to control application of vacuum from vacuum source 308. The signal generator 304 may be, for example, a Model N5181A MXG Analog Signal Generator 100 KHz-6 GHz, available from Agilent Technologies. The amplifier 306 may be, for example, a Model HD18288SP High Power TWT Amplifier 5.7-18 GHz, available from HD Communications Corporation. The vacuum source 308 may be, for example, a Model 0371224 Basic 30 Portable Vacuum Pump, available from Medela. The coolant source 310 may be, for example, a 0P9TNAN001 NanoTherm Industrial Recirculating Chiller available from ThermoTek, Inc.

Figure 3 illustrates a system for delivering microwave energy according to one embodiment of the invention. In the system illustrated in Figure 3, power is supplied by power source 318, which may be, for example an AC mains power line. In the system illustrated in Figure 3, isolation transformer 316 isolates the mains power provided by power source 318 and supplies isolated power to controller 302, vacuum source 308, signal generator 304, amplifier 306, temperature data acquisition system 314 and coolant source 310. Vacuum cable 372 connects vacuum source 308 to applicator 320. In the system illustrated in Figure 3, signal generator 304 generates a signal, which may be, for example, a continuous wave (CW) signal having a frequency in the range of, for example, 5.8 GHz and that signal is supplied to amplifier 306, which is controlled by controller 302. In the system illustrated in Figure 3, an output signal from amplifier 306 may be transmitted to an applicator 320 by signal cable 322. The signal cable 322 may be, for example, a fiber optic link. The applicator 320 may be, for example, a microwave energy device. In the system illustrated in Figure 3, coolant source 310 may supply a coolant, such as, for example, chilled de-ionized water, to applicator 320 through coolant tubing 324, and, more particularly, coolant may be supplied to applicator 320 through inflow tubing 326 and returned to coolant source 310 through outflow tubing 328. In the system illustrated in Figure 3, applicator 320 includes temperature measurement devices which relay temperature signals 330 to the temperature data acquisition system 314, which, in turn, relays temperature signals by a fiber optic link 332 to the temperature display computer 312. The isolation transformer 316 may be ISB-100W Isobox, available from Toroid Corporation of Maryland. The temperature display computer 312 may be, for example, a custom timer controller developed from a number of off-the-shelf timer relay components and custom control circuitry. The temperature data acquisition system 314 may be, for example, a Thermes-USB Temperature Data Acquisition System with OPT-1 Optical Link available from Physitemp Instruments Inc.

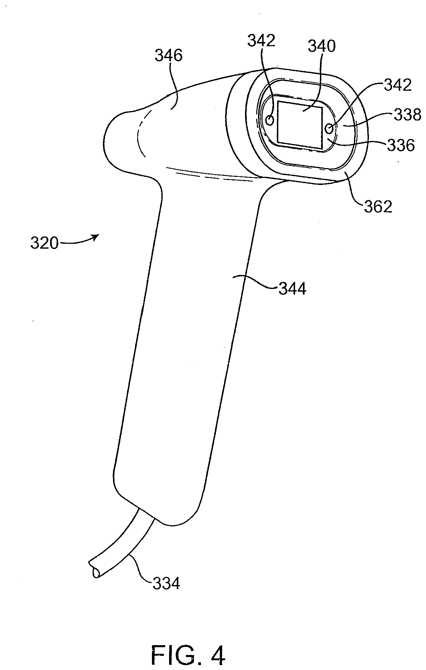

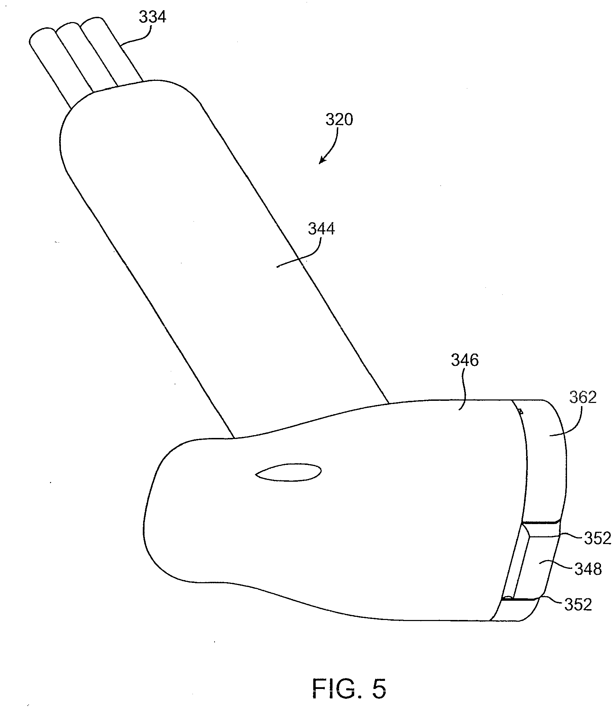

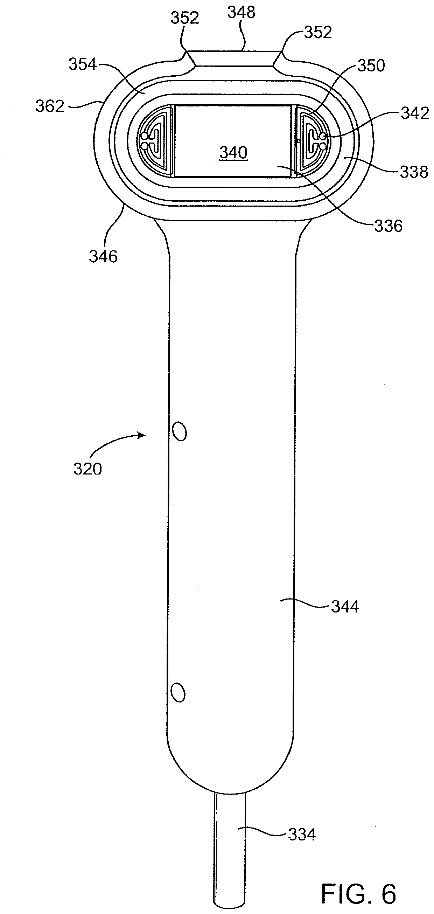

Figure 4 is a side perspective view of a microwave applicator of a system according to one embodiment of the invention. Figure 5 is a top perspective view of a microwave applicator of a system according to one embodiment of the invention. Figure 6 is a front view of a microwave applicator of a system according to one embodiment of the invention. In the microwave applicator illustrated in Figures 4 through 6, applicator 320 includes applicator cable 334, applicator handle 344, applicator head 346 and tissue head 362. In the microwave applicatorillustrated in Figures 4 through 6, tissue head 362 includes vacuum ports 342, cooling plate 340, tissue chamber 338 and tissue interface 336. In the microwave applicator illustrated in Figure 5, tissue head 362 includes alignment guide 348, which includes alignment features 352. In the microwave applicator illustrated in Figure 6, tissue head 362 is mounted on applicator head 346 of applicator 320. In the microwave applicatorillustrated in Figure 6, tissue head 362 includes alignment guide 348, alignment features 352 and tissue chamber 338. In the microwave applicator illustrated in Figure 6, tissue chamber 338 includes tissue wall 354 and tissue interface 336. In the microwave applicator illustrated in Figure 6, tissue interface 336 includes cooling plate 340, vacuum ports 342 and vacuum channel 350.

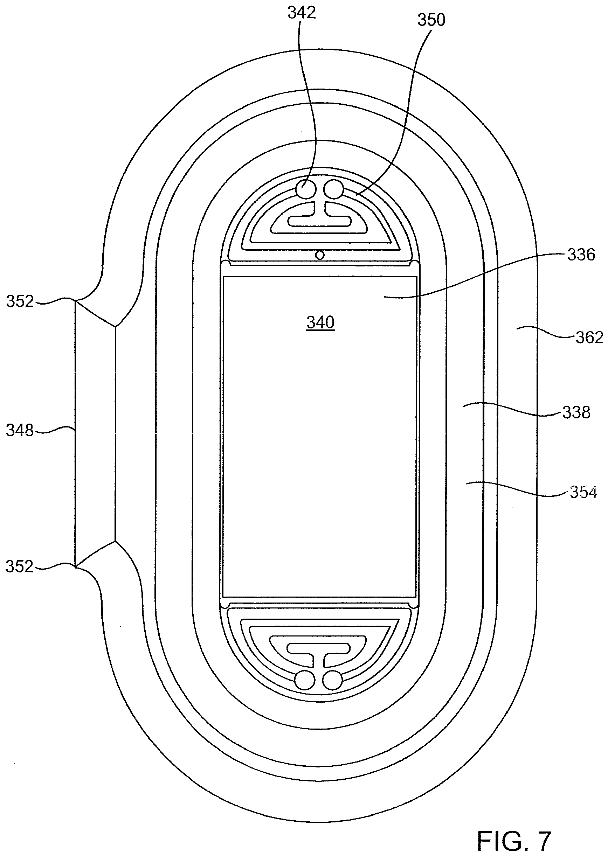

Figure 7 is a front view of a tissue head for use with a microwave applicator of a system according to one embodiment of the invention. In the tissue head illustrated in Figure 7, tissue head 362 includes alignment guide 348, alignment features 352 and tissue chamber 338. In the tissue head illustrated in Figure 7, tissue chamber 338 includes tissue wall 354 and tissue interface 336. In the tissue head illustrated in Figure 7, tissue interface 336 includes cooling plate 340, vacuum ports 342 and vacuum channel 350. In one embodiment of the invention, tissue head 362 is detachable and may be used as a disposable element of a microwave applicator such as, for example, applicator 320.

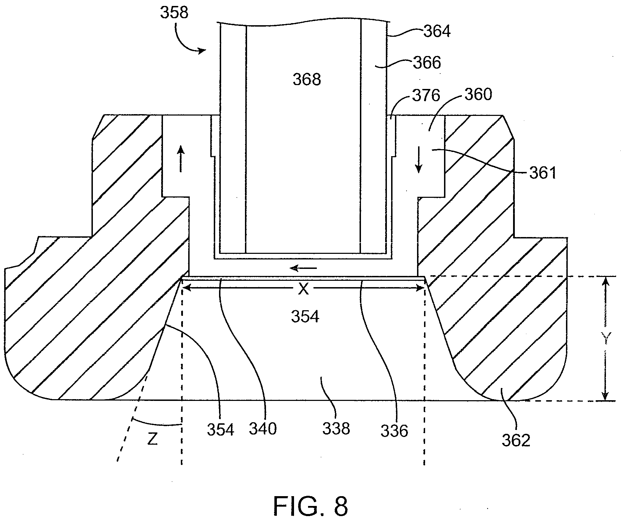

Figure 8 is a cut away view of a tissue head of a system according to one embodiment of the invention. Figure 8 is a cutaway view of a tissue head 362 and antenna 358 of a system according to one embodiment of the invention. Antenna 358 may be, for example, a waveguide 364 which may include, for example, waveguide tubing 366 and dielectric filler 368. In the tissue head illustrated in Figure 8 antenna 358 is isolated from cooling fluid 361 in coolant chamber 360 by standoff 376. In the tissue head illustrated in Figure 8, chamber wall 354 has a chamber angle Z which facilitates the acquisition of tissue. In the tissue head illustrated in Figure 8 tissue interface 336, which may include cooling plate 340, has a minimum dimension X and tissue chamber 338 has a depth Y.

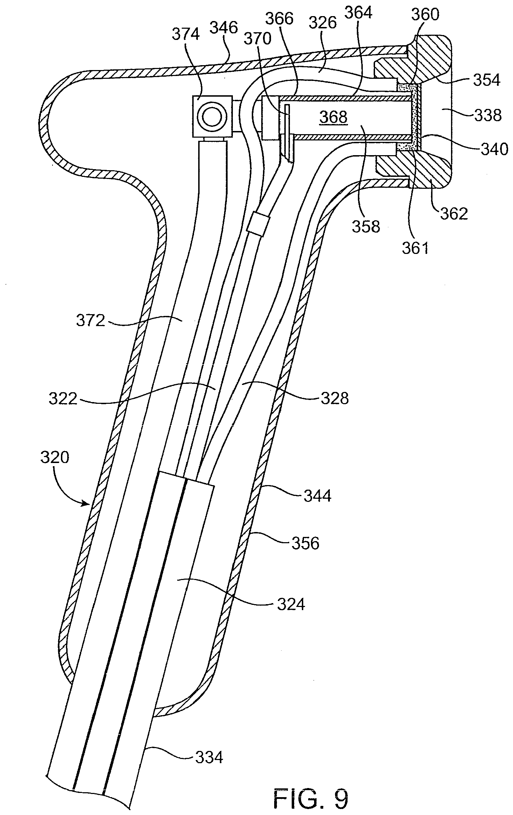

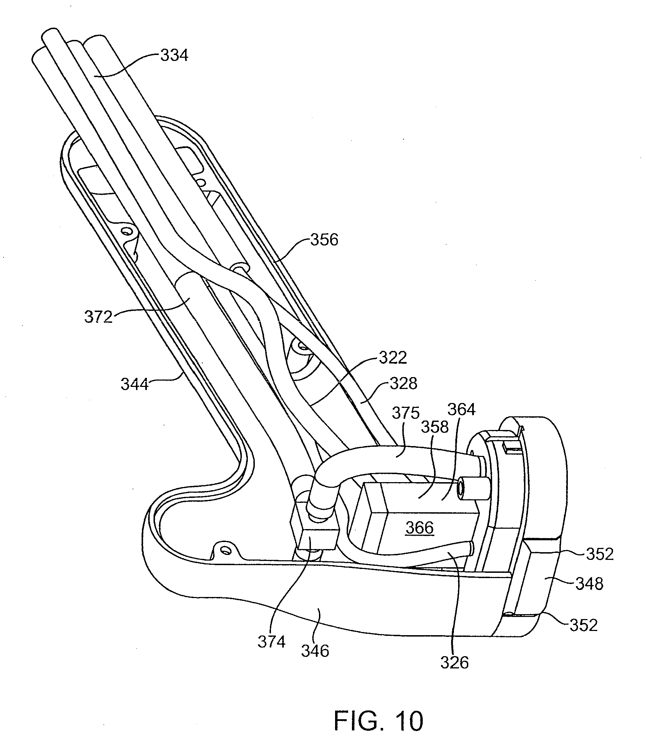

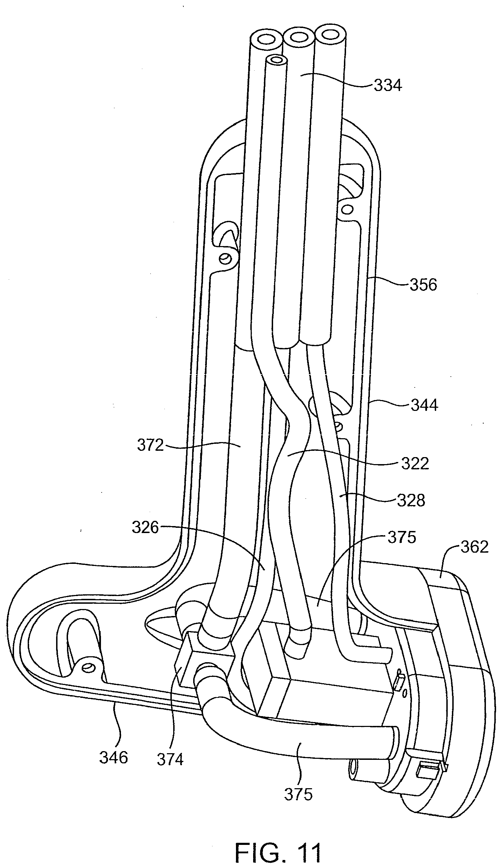

Figure 9 is a side cutaway view of a microwave applicator of a system according to one embodiment of the invention. Figure 10 is a top perspective partial cutaway view of a microwave applicator of a system according to one embodiment of the invention. Figure 11 is a side partial cutaway view of a microwave applicator of a system according to one embodiment of the invention. In the microwave applicator illustrated in Figures 9 through 11, applicator 320 includes applicator housing 356 and tissue head 362. In the microwave applicatorillustrated in Figures 9 through 11, applicator housing 356 encloses applicator handle 344 and at least a portion of applicator head 346. In the microwave applicatorillustrated in Figures 9 through 11 applicator cable 334 includes coolant tubing 324, inflow tubing 326, outflow tubing 328, signal cable 322 and vacuum cable 372. In the microwave applicator illustrated in Figures 9 through 11, vacuum cable 372 is connected to vacuum splitter 374. In the microwave applicatorillustrated in Figures 9 through 11, applicator 320 includes antenna 358. In the microwave applicatorillustrated in Figures 9 through 11, antenna 358 may include waveguide antenna 364. In the microwave applicatorillustrated in Figures 9 through 11, waveguide antenna 364 may include dielectric filler 368 and waveguide tubing 366. Cooling chamber 360 may be configured to facilitate the continuous flow of cooling fluid 361 across one surface of cooling plate 340. In the microwave applicator illustrated in Figures 9 through 11, signal cable 322 is connected to antenna 358 by antenna feed 370, which may be, for example a distal end of a semi-rigid coaxial cable or a panel mount connector and includes the center conductor of the cable or connector. In the microwave applicatorillustrated in Figures 9 through 11, applicator 320 includes tissue head 362. In the microwave applicator illustrated in Figures 9 through 11, tissue head 362 includes tissue chamber 338, chamber wall 354, cooling plate 340 and cooling chamber 360. In the microwave applicator illustrated in Figures 9 through 11, cooling chamber 360 is connected to inflow tubing 326 and outflow tubing 328. In the microwave applicator illustrated in Figure 10, vacuum cable 372 is connected to secondary vacuum cables 375. In the microwave applicator illustrated in Figure 10, secondary vacuum cables 375 are connected to vacuum ports 342 (not shown) in tissue head 362.

In the microwave applicator illustrated in Figure 11, vacuum cable 372 is connected to secondary vacuum cables 375. In the microwave applicator illustrated in Figure 11, secondary vacuum cables 375 are connected to vacuum ports 342 (not shown) in tissue head 362.

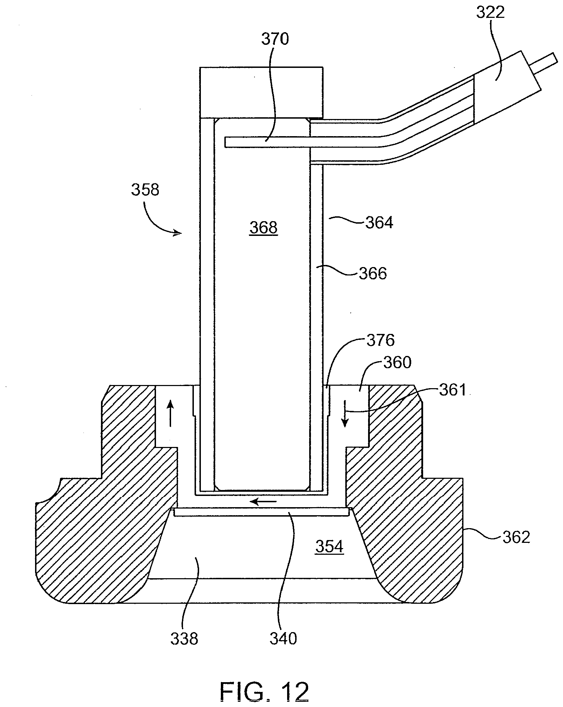

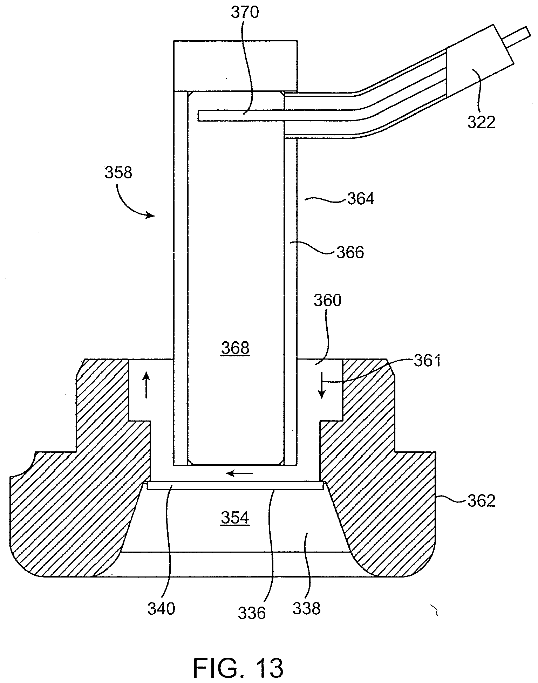

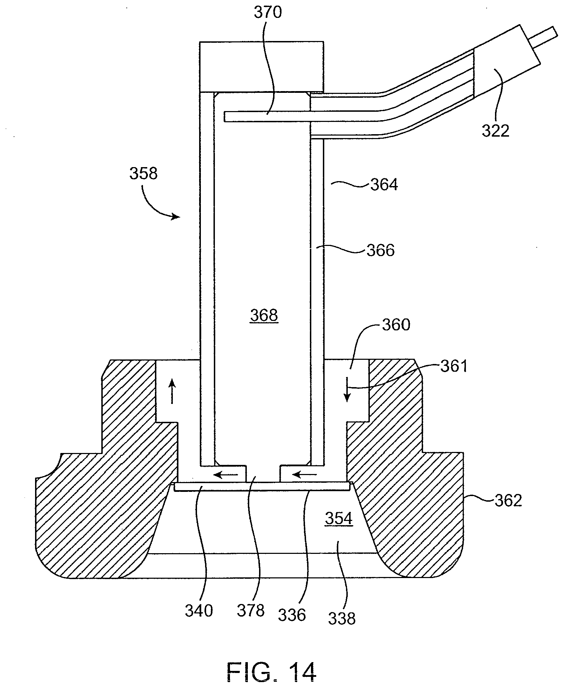

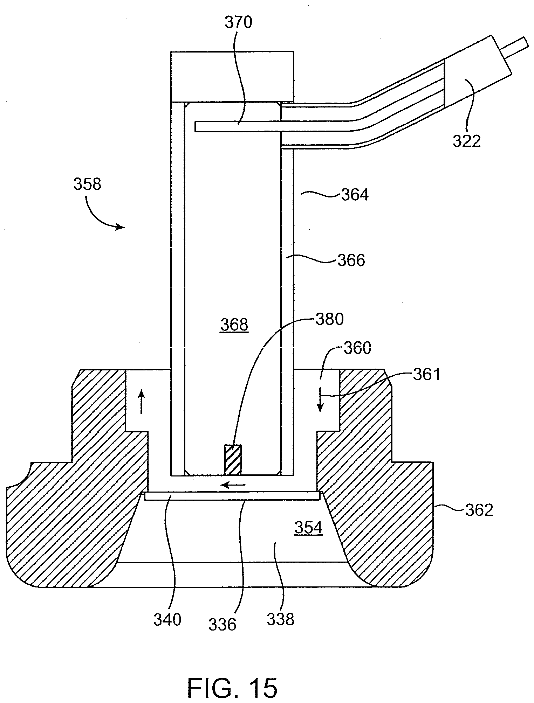

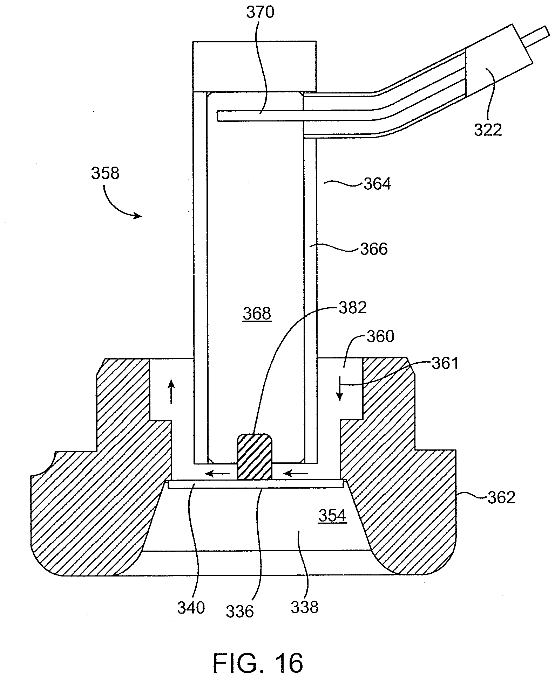

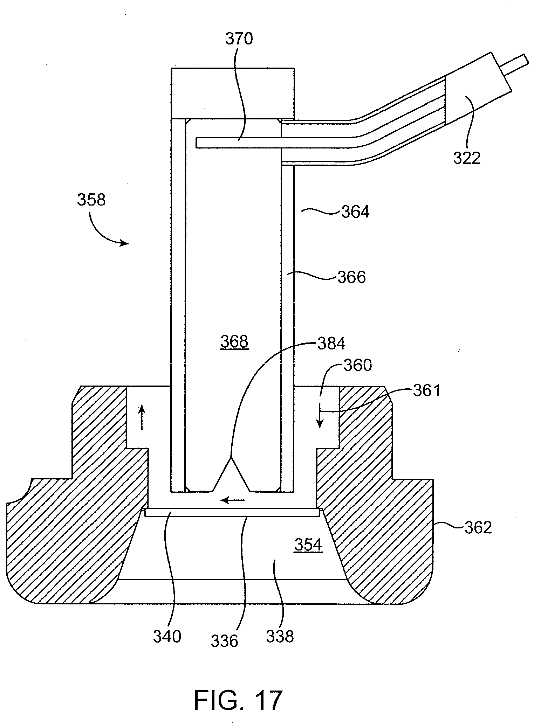

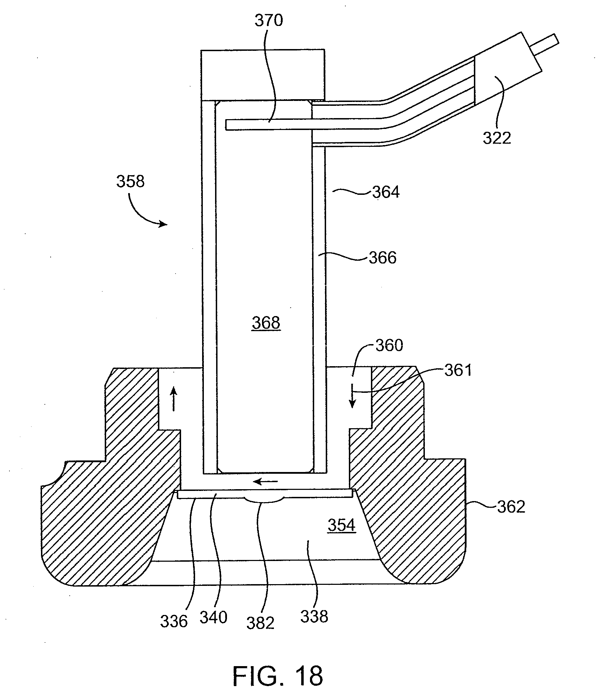

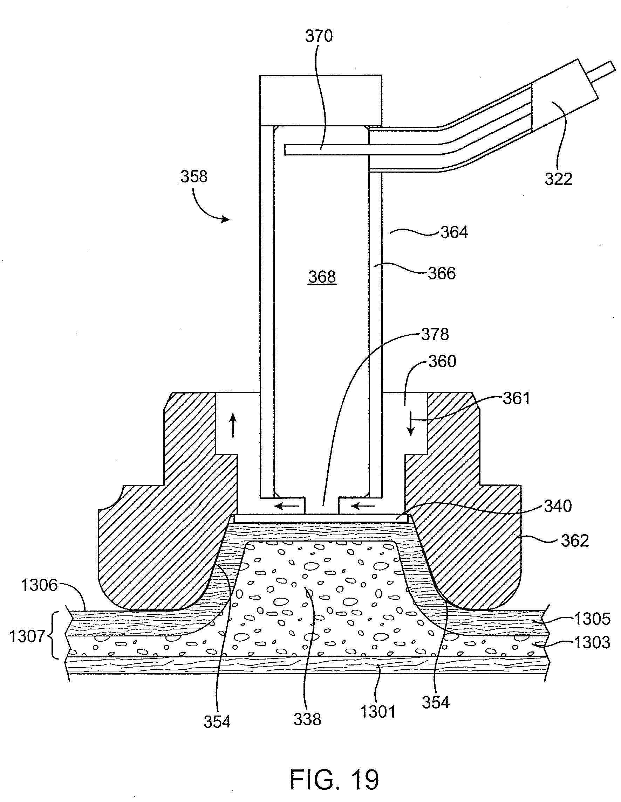

Figure 12 is a cutaway view of a tissue head and antenna of a system according to one embodiment of the invention. Figure 13 is a cutaway view of a tissue head and antenna of a system according to one embodiment of the invention. Figure 14 is a cutaway view of a tissue head, antenna and field spreader of a system according to one embodiment of the invention. Figure 15 is a cutaway view of a tissue head, antenna and field spreader of a system according to one embodiment of the invention. Figure 16 is a cutaway view of a tissue head, antenna and field spreader of a system according to one embodiment of the invention. Figure 17 is a cutaway view of a tissue head, antenna and field spreader of a system according to one embodiment of the invention. Figure 18 is a cutaway view of a tissue head, antenna and field spreader of a system according to one embodiment of the invention. Figure 19 is a cutaway view of a tissue head, antenna and field spreader, with tissue engaged, of a system according to one embodiment of the invention. In the tissue head and illustrated in Figures 12 through 19 antenna 358 may be, for example, a waveguide antenna 364. The waveguide antenna 364 may comprise, for example, waveguide tubing 366, waveguide filler 368 and may be connected to signal cable 322 by, for example, antenna feed 370. In the tissue head and antenna illustrated in Figures 12 through 19 tissue head 362 may comprise, for example, tissue chamber 338, chamber wall 354, cooling plate 340 and cooling chamber 360. The cooling chamber 360 may include cooling fluid 361.

In the tissue head and antenna illustrated in Figure 12 antenna 358 is isolated from cooling fluid 361 in coolant chamber 360 by standoff 376. In the tissue head and antenna illustrated in Figure 13 at least a portion of antenna 358 is positioned in coolant chamber 360. In the tissue head and antenna illustrated in Figure 13 at least a portion of waveguide antenna 364 is positioned in coolant chamber 360. In the tissue head and antenna illustrated in Figure 13 waveguide antenna 364 is positioned in coolant chamber 360 such that at least a portion of waveguide tubing 366 and dielectric filler 368 contact cooling fluid 361 in coolant chamber 360.

In the tissue head, antenna and field spreaderillustrated in Figure 14 field spreader 378 is positioned at an output of waveguide antenna 364. In the tissue head, antenna and field spreader illustrated in Figure 14 field spreader 378 is an extension of dielectric filler 368 and is positioned at an output of waveguide antenna 364. In the tissue head, antenna and field spreader illustrated in Figure 14 field spreader 378 is an extension of dielectric filler 368 extending into coolant chamber 360. In the tissue head, antenna and field spreader illustrated in Figure 14 field spreader 378 is an extension of dielectric filler 368 extending through coolant chamber 360 to cooling plate 340.

In the tissue head, antenna and field spreader illustrated in Figure 15 field spreader 380 is integrated into dielectric filler 368 of waveguide antenna 364. In the tissue head, antenna and field spreader illustrated in Figure 15 field spreader 380 is a region of dielectric filler 368 having a dielectric constant which differs from the dielectric constant of the remainder of dielectric filler 368. In the tissue head, antenna and field spreader illustrated in Figure 15 field spreader 380 is a region having a dielectric constant which is in the range of approximately 1 to 15.

In the tissue head, antenna and field spreader illustrated in Figure 16 field spreader 382 is integrated into dielectric filler 368 of waveguide antenna 364 and extends into coolant chamber 360. In the tissue head, antenna and field spreaderillustrated in Figure 16 field spreader 382 is integrated into dielectric filler 368 of waveguide antenna 364 and extends through coolant chamber 360 to cooling plate 340. In the tissue head, antenna and field spreader illustrated in Figure 16 field spreader 382 has a dielectric constant which differs from the dielectric constant of dielectric filler 368. In the tissue head, antenna and field spreader illustrated in Figure 15 field spreader 380 is a region having a dielectric constant which is in the range of approximately 1 to 15. In the tissue head, antenna and field spreader illustrated in Figure 17, a field spreader may be comprised of a notch 384 in dielectric filler 368. In the tissue head, antenna and field spreader illustrated in Figure 17, notch 384 is a cone shaped notch in dielectric filler 368. In the tissue head, antenna and field spreader illustrated in Figure 17, notch 384 is connected to cooling chamber 360 such that cooling fluid 361 in cooling chamber 360 at least partially fills notch 384. In the tissue head, antenna and field spreader illustrated in Figure 17, notch 384 is connected to cooling chamber 360 such that cooling fluid 361 in cooling chamber 360 fills notch 384.

In the tissue head, antenna and field spreader illustrated in Figure 18 field spreader 382 is integrated into or protrudes from cooling plate 340. In the tissue head, antenna and field spreader illustrated in Figure 18 field spreader 382 is integrated into or protrudes from cooling plate 340 at tissue interface 336. In the tissue head, antenna and field spreader illustrated in Figure 18 field spreader 382 is integrated into or protrudes from cooling plate 340 into tissue chamber 338.

In the tissue head, antenna and field spreaderillustrated in Figure 19 skin 1307 is engaged in tissue chamber 338. In the the tissue head, antenna and field spreader illustrated in Figure 19 dermis 1305 and hypodermis 1303 are engaged in tissue chamber 338. In the the tissue head, antenna and field spreader illustrated in Figure 19, skin surface 1306 is engaged in tissue chamber 338 such that skin surface 1306 is in contact with at least a portion of chamber wall 354 and cooling plate 340. As illustrated in Figure 19, a vacuum pressure may be used to elevate dermis 1305 and hypodermis 1303, separating dermis 1305 and hypodermis 1303 from muscle 1301. As illustrated in Figure 19, a vacuum pressure may be used to elevate dermis 1305 and hypodermis 1303, separating dermis 1305 and hypodermis 1303 from muscle 1301 to, for example, protect muscle 1301 by limiting or eliminating the electromagnetic energy which reaches muscle 1301.

Figure 20 is a cutaway view of a tissue head and antenna, with tissue engaged, of a system according to one embodiment of the invention. In the tissue head and antenna illustrated in Figure 20 applicator 320 includes applicator housing 356, antenna 358, vacuum channels 350 and tissue head 362. In the tissue head and antenna illustrated in Figure 20 tissue head 362 includes vacuum conduit 373, cooling elements 386 and cooling plate 340. In embodiments of the invention, cooling elements 386 may be, for example: solid coolants; heat sinks; liquid spray, gaseous spray, cooling plates, thermo-electric coolers; or and combinations thereof. In the tissue head and antenna illustrated in Figure 20 vacuum channels 350 are connected to vacuum conduit 373 and vacuum port 342. In the tissue head and antenna illustrated in Figure 20 skin surface 1306 is engaged in tissue chamber 338 by, for example a vacuum pressure at vacuum ports 342, such that skin surface 1306 is in contact with at least a portion of chamber wall 354 and cooling plate 340. As illustrated in Figure 20, a vacuum pressure at vacuum ports 342 may be used to elevate dermis 1305 and hypodermis 1303, separating dermis 1305 and hypodermis 1303 from muscle 1301. As illustrated in Figure 20, a vacuum pressure at vacuum ports 342 may be used to elevate dermis 1305 and hypodermis 1303, separating dermis 1305 and hypodermis 1303 from muscle 1301 to, for example, protect muscle 1301 by limiting or eliminating the electromagnetic energy which reaches muscle 1301.

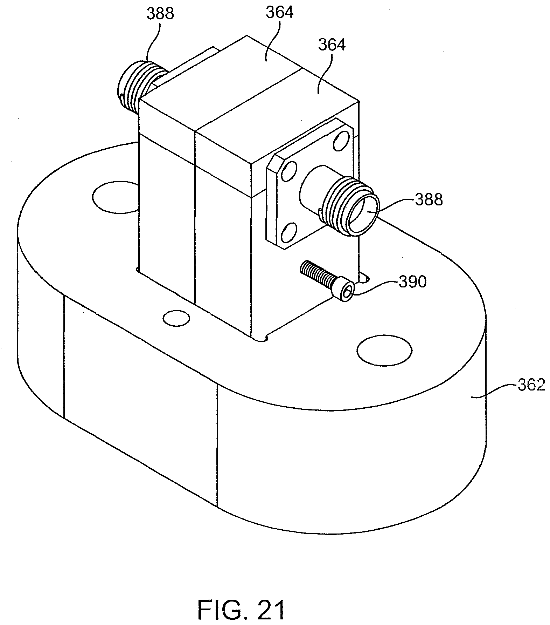

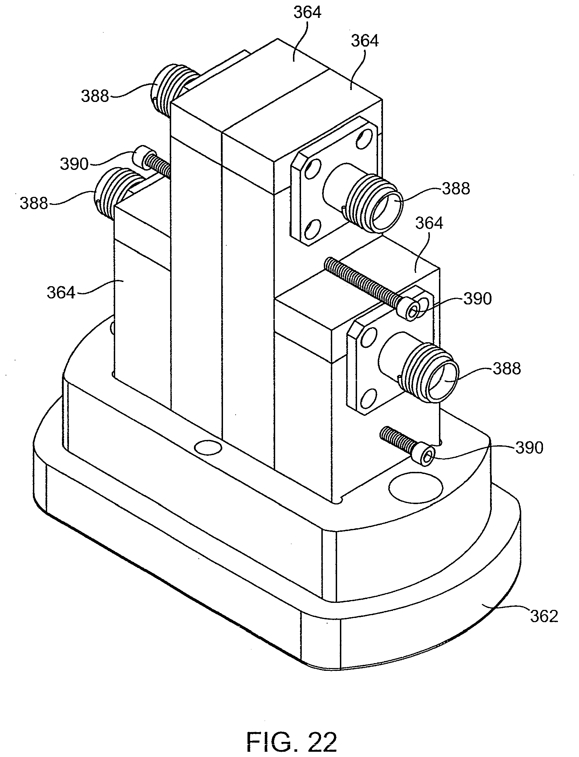

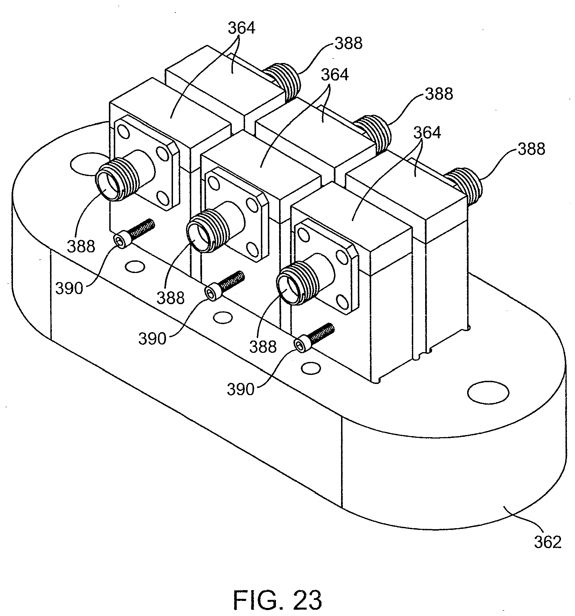

Figures 21 through 23 illustrate tissue heads 362 including a plurality of waveguide antennas 364 of systems according to embodiments of the invention. In the tissue head 362 illustrated in Figure 21, two waveguide antennas 364 are positioned in tissue head 362. In the tissue heads 362illustrated in Figures 21 through 23 waveguides 364 include feed connectors 388 and tuning screws 390. In the tissue head 362 illustrated in Figure 22, four waveguide antennas 364 are positioned in tissue head 362. In the tissue head 362 illustrated in Figure 23, six waveguide antennas 364 are positioned in tissue head 362.

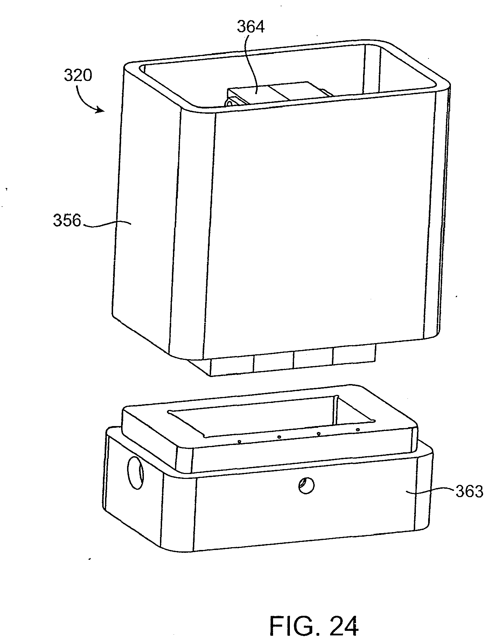

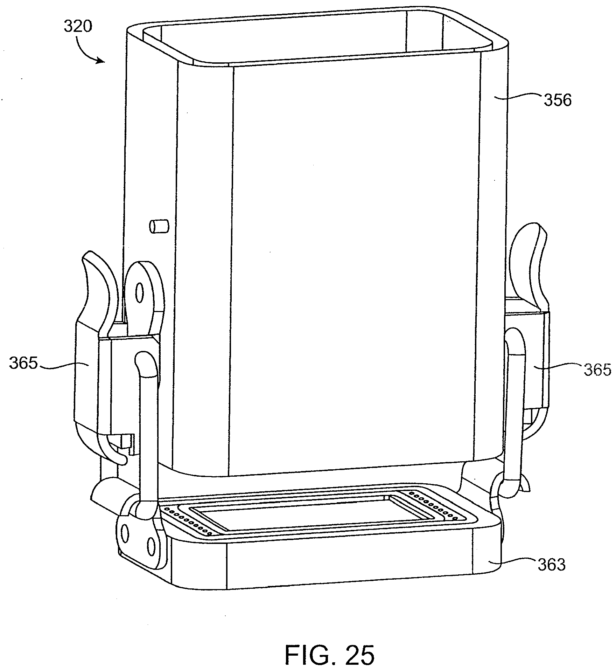

Figure 24 illustrates a disposable tissue head 363 for use with an applicator 320 of a system according to one embodiment of the invention. In the disposable tissue head 363 illustrated in Figure 24 disposable tissue head 363 engages with applicator housing 356, positioning antennas 364 in disposable tissue head 363. Figure 25 illustrates a disposable tissue head 363 for use with an applicator 320 of a system according to one embodiment of the invention. In the disposable tissue head 363 illustrated in Figure 25 disposable tissue head 363 engages with applicator housing 356 and is held in place with latches 365.

### Tissue Profiles

Figures 26 through 51 illustrate a series of tissue profiles, such as, for example, power deposition profiles in tissue, generated by systems according to embodiments of the invention. The illustrated tissue profiles may be representative of, for example, SAR profiles, power loss density profiles or temperature profiles. The systems and components of systems illustrated in Figures 2 through 25 as well as, e.g., those illustrated and described at Figs. 3-7C and pp. 8-13 of U.S. Provisional App. No. 60/912,899; and Figs. 3-9 and 20-26 and pp. 34-48 and Figs. 20-26 of U.S. Provisional App. No. 61/013,274, may be used to generate the tissue profiles illustrated in Figures 26 through 51.

Figures 26 through 35 illustrate a series of tissue profiles generated using systems according to embodiments of the invention. In the tissue profilesillustrated in Figures 26 through 35 antenna 358 may be, for example, a simple dipole antenna or a waveguide antenna. In the tissue profilesillustrated in Figures 26 through 35 antenna 358 may be positioned in a medium1318. In the tissue profiles illustrated in Figures 26 through 35 antenna 358 radiates an electromagnetic signal through medium 1318 and into tissue, generating the patterns illustrated in Figures 26 through 35. The medium 1318 may be, for example, a dielectric material having a dielectric constant (which may also be referred to as permittivity) of approximately 10.

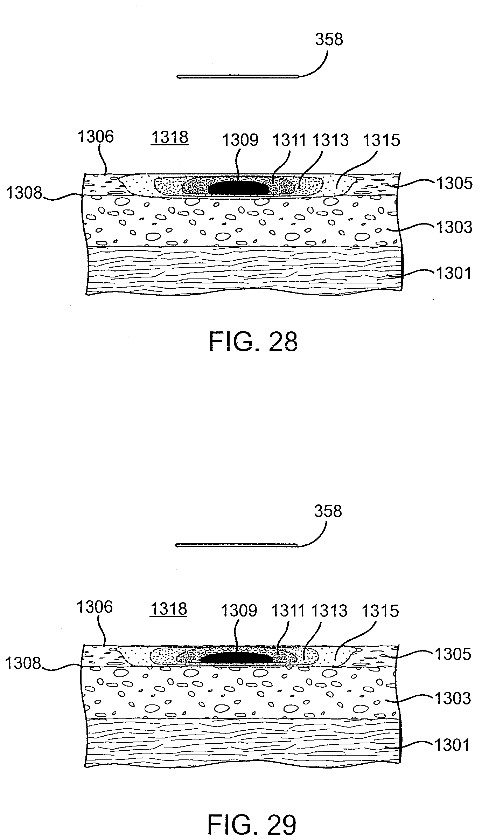

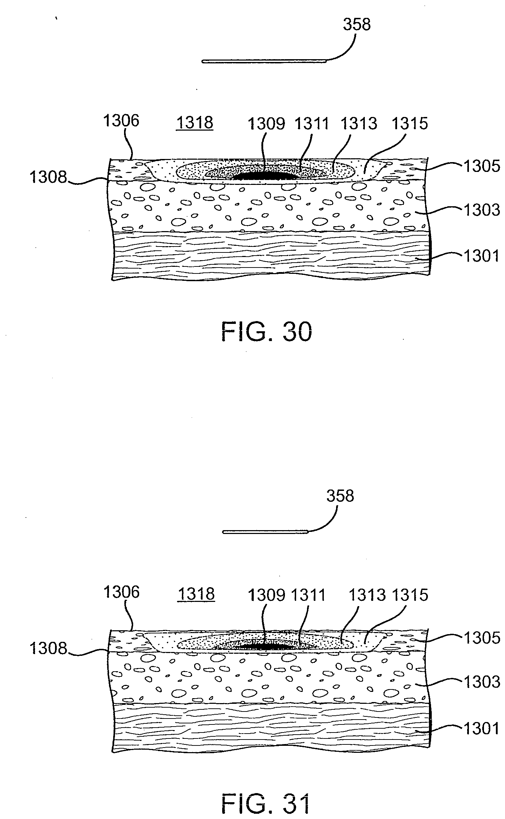

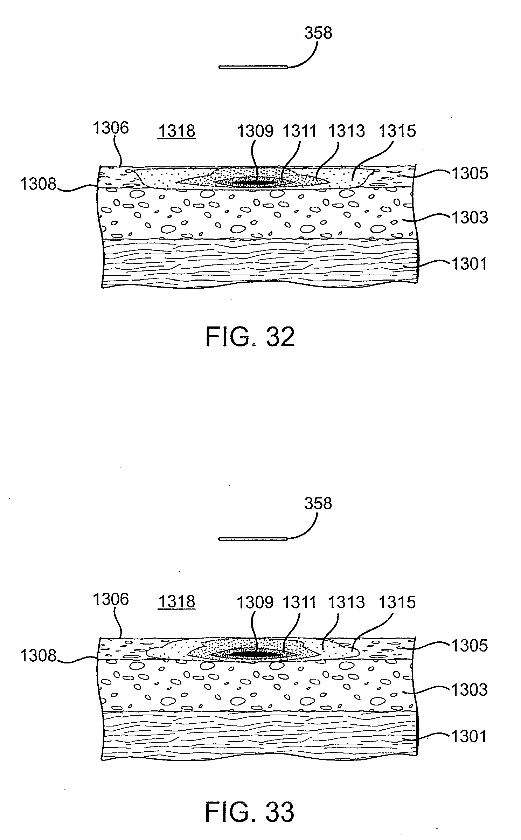

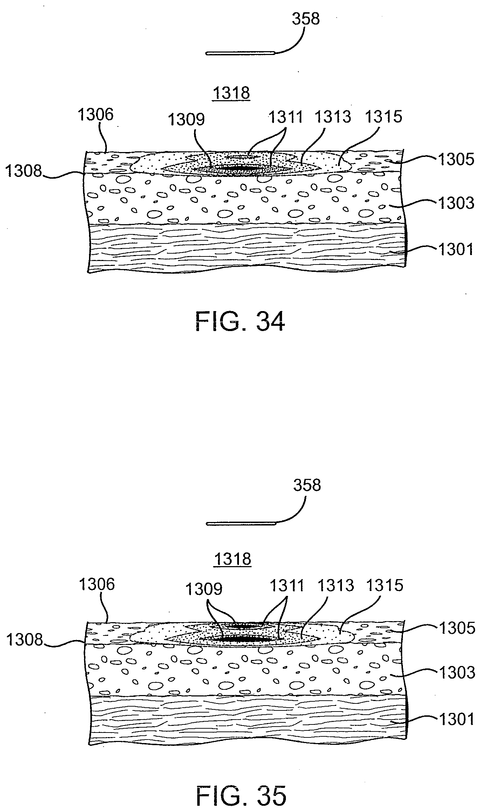

In the tissue profileillustrated in Figure 26 antenna 358 may radiate energy at a frequency of, for example, approximately 3.0 GHz. In the tissue profile illustrated in Figure 27 antenna 358 may radiate energy at a frequency of, for example, approximately 3.5 GHz. In the tissue profile illustrated in Figure 28 antenna 358 may radiate energy at a frequency of, for example, approximately 4.0 GHz. In the tissue profile illustrated in Figure 29 antenna 358 may radiate energy at a frequency of, for example, approximately 4.5 GHz. In the tissue profile illustrated in Figure 30 antenna 358 may radiate energy at a frequency of, for example, approximately 5.0 GHz. In the tissue profile illustrated in Figure 31 antenna 358 may radiate energy at a frequency of, for example, approximately 5.8 GHz. In the tissue profile illustrated in Figure 32 antenna 358 may radiate energy at a frequency of, for example, approximately 6.5 GHz. In the tissue profileillustrated in Figure 33 antenna 358 may radiate energy at a frequency of, for example, approximately 7.5 GHz. In the tissue profile illustrated in Figure 34 antenna 358 may radiate energy at a frequency of, for example, approximately 8.5 GHz. In the tissue profile illustrated in Figure 35 antenna 358 may radiate energy at a frequency of, for example, approximately 9.0 GHz. A tissue profile, such as the profile illustrated in Figures 34 and 35 may include at least two constructive interference peaks, where in a first constructive interference peak is positioned in tissue below a second constructive interference peak. A tissue profile, such as the profile illustrated in Figures 34 and 35 may include at least two constructive interference peaks, where in a second constructive interference peak is positioned near a skin surface.

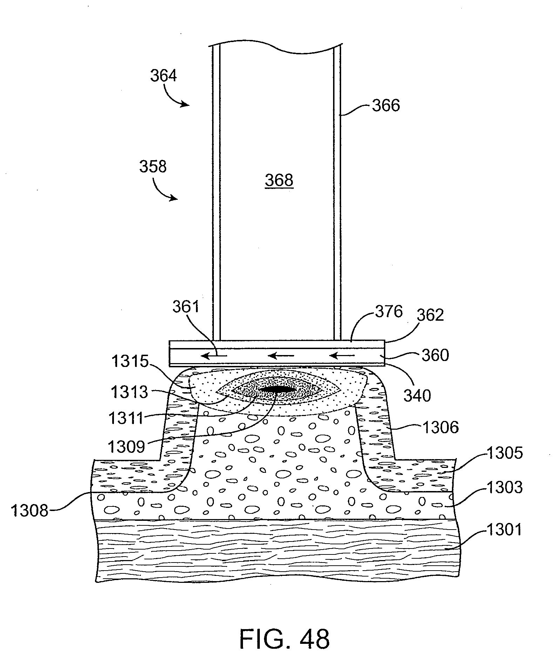

Wherein antenna 358 is representative of a wave guide antenna such as, for example, the waveguide antenna illustrated in Figure 48 radiating through, for example, at least a portion of a tissue head including a tissue interface, the frequencies at which particular tissue profiles, such as, for example, SAR profiles, power loss profiles or temperature profiles are created may vary from the frequencies at which that such profiles are generated by a dipole antenna. A tissue head positioned between a waveguide antenna and a skin surface may comprise, for example, for example, a standoff 376, a cooling chamber 360 filled with cooling fluid 361, such as, for example de-ionized water and a cooling plate 340. Wherein antenna 358 is a waveguide, antenna 358 may be positioned a distance of approximately 1.5 millimeters from skin surface 1306. Figure 34 illustrates a resulting profile where antenna 358 is a waveguide antenna radiating energy through a tissue head at a frequency of, for example, approximately 10 GHz. In one embodiment of the invention, Figure 35 illustrates a resulting profile where antenna 358 is a waveguide antenna radiating energy through a tissue head at a frequency of, for example, approximately 12 GHz.

In the tissue profiles illustrated in Figures 26 through 35, antenna 358 may have a length of, for example, approximately one half wavelength (measured at the operational frequency). In the tissue profiles illustrated in Figures 26 through 35, antenna 358 may be positioned in, for example, a radiating near field region with respect to skin surface 1306. In the tissue profiles illustrated in Figures 26 through 35 antenna 358 may be positioned at a distance of, for example, approximately 10 millimeters from skin surface 1306. In the tissue profiles illustrated in Figures 26 through 30 antenna 358 may be a dipole antenna having an antenna height of, for example, approximately 12 millimeters. In the tissue profiles illustrated in Figure 31 antenna 358 may be a dipole antenna having an antenna height of, for example, approximately 8.5 millimeters. In the tissue profiles illustrated in Figures 27 through 35 antenna 358 may be a dipole antenna having an antenna height of, for example, approximately 7 millimeters.

In the tissue profiles illustrated in Figures 26 through 35 power from antenna 358 is transmitted through skin surface 1306, generating a profile, such as, for example, a SAR profile, a power loss density profile or a temperature profile, in dermis 1305. In the tissue profiles illustrated in Figures 26 through 35 power transmitted from antenna 358 though skin surface 1306 generates a profile having a peak in first tissue region 1309. In the tissue profiles illustrated in Figures 26 through 35 power transmitted from antenna 358 though skin surface 1306 generates a profile wherein the magnitude decreases from first tissue region 1309 to second tissue region 1311. In the tissue profiles illustrated in Figures 26 through 35 power transmitted from antenna 358 though skin surface 1306 generates a SAR profile wherein the magnitude decreases from second tissue region 1311 to third tissue region 1313. In the tissue profiles illustrated in Figures 26 through 35 power transmitted from antenna 358 though skin surface 1306 generates a profile wherein the magnitude decreases from third tissue region 1313 to fourth tissue region 1315.

In the tissue profiles, illustrated in, for example, Figures 26 through 39, power transmitted from antenna 358 through skin surface 1306 is at least partially reflected off of interface 1308 such that a peak magnitude of, for example, SAR, power loss density or temperature is generated in first tissue region 1309 below skin surface 1306. In the tissue profiles illustrated in Figure 26 through 39, interface 1308 is idealized as a substantially straight line, however, in actual tissue, interface 1308 may be expected to be a non-linear, non continuous, rough interface which also includes tissue structures and groups of tissue structures which cross and interrupt interface 1308. In one embodiment of the invention, a peak magnitude of, for example, SAR, power loss density or temperature is formed as a result of constructive interference between incident and reflected power is positioned at first tissue region 1309 below a first layer of dermal tissue. In one embodiment of the invention, a minimum magnitude of, for example, SAR, power loss density or temperature is formed as a result of destructive interference between incident and reflected power is positioned in a first layer of dermal tissue near skin surface 1306. In one embodiment of the invention, interface 1308 may be, for example, an interface between dermis 1305 and hypodermis 1303. In one embodiment of the invention, first tissue region 1309 may be formed in the lower half of the dermis. In one embodiment of the invention, interface 1308 may be, for example, an interface between a high dielectric / high conductivity tissue layer and a low dielectric / low conductivity tissue. In one embodiment of the invention, interface 1308 may be, for example, an interface between a glandular layer and a hypodermis.

In one embodiment of the invention, energy transmitted through skin surface 1306 creates a peak temperature in first region 1309. In one embodiment of the invention, energy transmitted through skin surface 1306 raises a temperature in first region 1309 to a temperature sufficient to induce hyperthermia in tissue in region 1309. In one embodiment of the invention, energy transmitted through skin surface 1306 raises a temperature in first region 1309 to a temperature sufficient to ablate tissue in region 1309. In one embodiment of the invention, energy transmitted through skin surface 1306 raises a temperature in first region 1309 to a temperature sufficient to cause cell death in tissue in region 1309. In one embodiment of the invention, energy transmitted through skin surface 1306 raises a temperature in first region 1309 to a temperature sufficient to form a lesion core in first region 1309. In one embodiment of the invention, energy transmitted through skin surface 1306 raises a temperature in first region 1309 to a temperature sufficient to create a lesion in tissue in region 1309. In one embodiment of the invention, energy transmitted through skin surface 1306 raises the temperature of tissue in region 1309 by dielectric heating. In one embodiment of the invention, energy transmitted through skin surface 1306 preferentially raises the temperature of tissue in region 1309 above the temperature of surrounding regions.

In one embodiment of the invention, energy transmitted through skin surface 1306 generates a temperature in first region 1309 sufficient to heat tissue around first region 1309, by, for example, conductive heating. In one embodiment of the invention, energy transmitted through skin surface 1306 generates a temperature in first region 1309 sufficient to heat tissue structures, such as, for example, sweat glands or hair follicles, in tissue around first region 1309, by, for example, conductive heating. In one embodiment of the invention, energy transmitted through skin surface 1306 generates a temperature in first region 1309 sufficient to cause hyperthermia in tissue around first region 1309, by, for example, conductive heating. In one embodiment of the invention, energy transmitted through skin surface 1306 generates a temperature in first region 1309 sufficient to cause ablate tissue around first region 1309, by, for example, conductive heating. In one embodiment of the invention, energy transmitted through skin surface 1306 generates a temperature in first region 1309 sufficient to create a lesion in tissue around first region 1309, by, for example, conductive heating. In one embodiment of the invention, energy transmitted through skin surface 1306 generates a temperature in first region 1309 sufficient to expand a lesion into tissue around first region 1309, by, for example, conductive heating.

### Near Field

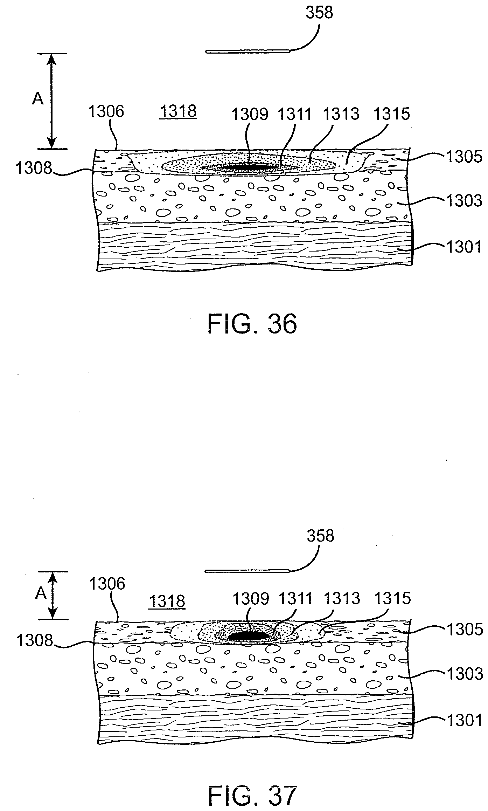

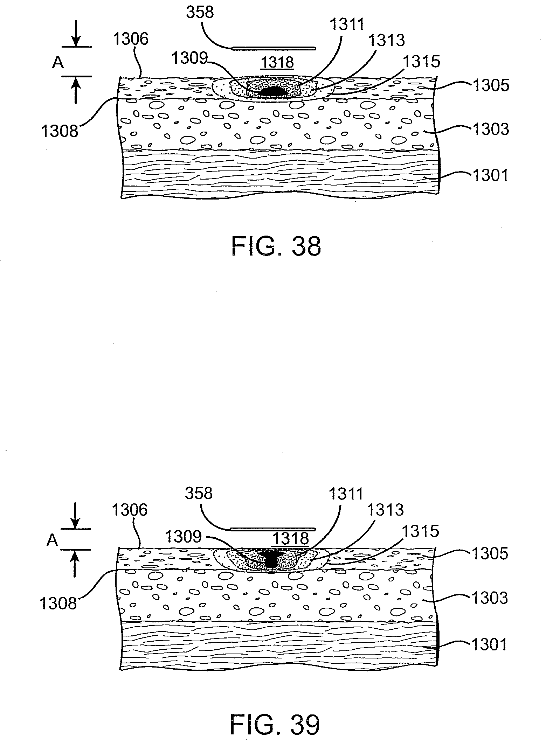

Figures 36 through 39 illustrate a series of tissue profiles generated using systems according to embodiments of the invention. In the tissue profiles illustrated in Figures 36 through 39 antenna 358 may be, for example, a simple dipole antenna or a waveguide antenna. In the tissue profiles illustrated in Figure 36 through 39 antenna 358 may be excited at a predetermined frequencies such as, for example, approximately 5.8 GHz. In the tissue profiles illustrated in Figures 36 through 38, antenna 358 may be positioned in, for example, a radiating near field region with respect to skin surface 1306. In the tissue profile illustrated in Figure 39, antenna 358 may be positioned in, for example, a reactive near field region with respect to skin surface 1306. In the tissue profiles illustrated in Figures 36 through 39 antenna 358 may be positioned at a distance A of, for example, between approximately 10 millimeters and approximately 2 millimeters from skin surface 1306. In the tissue profiles illustrated in Figures 36 through 39 antenna 358 may be positioned in a medium1318. In the tissue profiles illustrated in Figures 36 through 39 antenna 358 maybe a dipole antenna having an antenna height of approximately 8.5 millimeters. In the tissue profiles illustrated in Figures 36 through 39 antenna 358 may radiate energy at a frequency of, for example, approximately 5.8 GHz.

In the tissue profiles illustrated in Figures 36 through 39 power from antenna 358 is transmitted through skin surface 1306, generating a SAR profile in dermis 1305. In the tissue profiles illustrated in Figures 36 through 39 power transmitted from antenna 358 though skin surface 1306 generates a SAR profile having a peak in first tissue region 1309. In the tissue profiles illustrated in Figures 36 through 39 power transmitted from antenna 358 though skin surface 1306 generates a tissue profile which may represent, for example, SAR, power loss density or temperature, the magnitude of, for example, SAR, power loss density or temperature, decreases from first tissue region 1309 to second tissue region 1311, from second tissue region 1311 to third tissue region 1313 and from third tissue region 1313 to fourth tissue region 1315.

In the tissue profile, illustrated in, for example, Figure 36, power transmitted from antenna 358 through skin surface 1306 is at least partially reflected off of interface 1308 such that a peak of, for example, SAR, power loss density or temperature is generated in first tissue region 1309 below skin surface 1306. In the tissue profile illustrated in, for example, Figure 36, a peak of, for example, SAR, power loss density or temperature formed as a result of constructive interference between incident and reflected power is positioned at first tissue region 1309 below a first layer of dermal tissue. In the tissue profile illustrated in, for example, Figure 36, a peak of, for example, SAR, power loss density or temperature formed as a result of constructive interference between incident and reflected power is positioned at first tissue region 1309 in a lower half of the dermis. In the tissue profile illustrated in Figure 36 antenna 358 may be positioned at a distance A of, for example, approximately 10 millimeters from skin surface 1306. In the tissue profile illustrated in Figure 37 antenna 358 may be positioned at a distance A of, for example, approximately 5 millimeters from skin surface 1306. In the tissue profile illustrated in Figure 38 antenna 358 may be positioned at a distance A of, for example, approximately 3 millimeters from skin surface 1306. In the tissue profile illustrated in Figure 39 antenna 358 may be positioned at a distance A of, for example, approximately 2 millimeters from skin surface 1306. In the tissue profile illustrated in Figures 36 through 38, tissue in region 1309 is preferentially heated with respect to tissue in layers above first tissue region 1309.

In the tissue profile illustrated in Figure 36 antenna 358 may be positioned at a distance A within a radiating near field of skin surface 1306. In the tissue profile illustrated in Figure 37 antenna 358 may be positioned at a distance A within a radiating near field of skin surface 1306. In the tissue profile illustrated in Figure 38 antenna 358 may be positioned at a distance A within a radiating near field of skin surface 1306. In the tissue profile illustrated in Figure 39 antenna 358 may be positioned at a distance A within a reactive near field of skin surface 1306. As illustrated in Figure 39, positioning an antenna in a reactive near field results in substantial reactive coupling, which increases power deposition at the upper skin layer and destroys the preferential heating profiles illustrated in Figures 36 thorough 38.

### Preferential Heating - Dermis

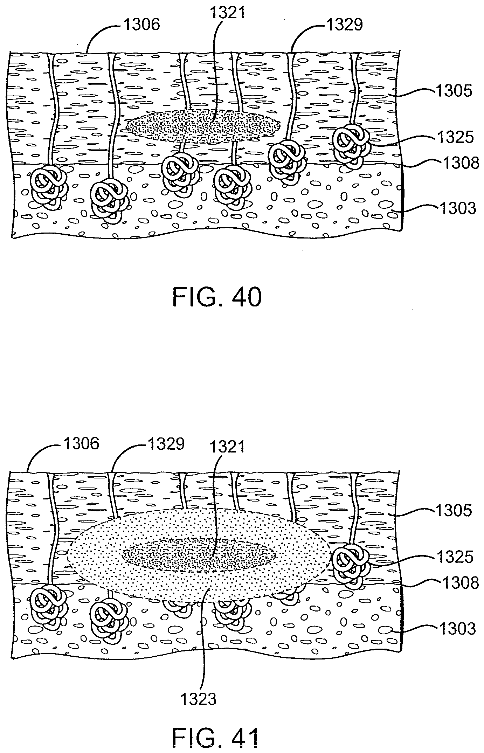

Figures 40 through 43 illustrate tissue profiles. In the tissue profiles illustrated in Figures 40 through 43 dermis 1305 and hypodermis 1303 may contain tissue structures 1325 which may be, for example, sweat glands, including, for example, eccrine glands, apocrine glands or apoeccrine glands. In the tissue profiles illustrated in Figures 40 through 43 dermis 1305 and hypodermis 1303 may contain tissue structures 1325 which may be, for example, sweat glands, including, for example, eccrine glands, apocrine glands or apoeccrine glands. In the tissue profiles illustrated in Figures 40 through 43 dermis 1305 and hypodermis 1303 may contain tissue structures 1325 which may be, for example, hair follicles. In the tissue profiles illustrated in Figures 40 through 43 tissue structures 1325 may include ducts 1329 extending from tissue structures 1325 to skin surface 1306.

Figure 40 illustrates a tissue profile according to one embodiment of the invention. In the tissue profile illustrated in Figure 40 a lesion core 1321 is created in a predetermined portion of dermis 1305 by, for example, irradiating dermis 1305 with electromagnetic radiation to generate dielectric heating in tissue at lesion core 1321. The lesion core 1321 may be, for example, a point or region within a tissue layer where a lesion starts to grow. In the tissue profile illustrated in Figure 40 lesion core 1321 is created by heat generated in dermal tissue by dielectric heating of lesion core 1321. In the the tissue profile illustrated in Figure 40 lesion core 1321 expands as energy is added to dermis 1305. In the the tissue profile illustrated in Figure 40 lesion core 1321 may be located in a region of dermis 1305 where a constructive interference peak is generated by electromagnetic energy transmitted through skin surface 1306. In the the tissue profile illustrated in Figure 40 lesion core 1321 may be located in a region of dermis 1305 where a constructive interference peak is generated by electromagnetic energy transmitted through skin surface 1306 wherein at least a portion of the electromagnetic energy transmitted through skin surface 1306 reflects off of interface 1308 which may be, for example, an interface between high dielectric / high conductivity tissue and low dielectric / low conductivity tissue. In the the tissue profileillustrated in Figure 40 lesion core 1321 may be located in a region of dermis 1305 where a constructive interference peak is generated by electromagnetic energy transmitted through skin surface 1306 wherein at least a portion of the electromagnetic energy transmitted through skin surface 1306 reflects off of interface 1308 which may be, for example, an interface between dermis 1305 and hypodermis 1303.

Figure 41 illustrates a tissue profile. In the the tissue profileillustrated in Figure 41 lesion core 1321 expands as energy is added to dermis 1305, generating heat which is conducted into surrounding tissue creating expanded lesion 1323. In the the tissue profile illustrated in Figure 41 heat conducted from lesion core 1321 into expanded lesion 1323 damages tissue, including tissue structures 1325. In the the tissue profile illustrated in Figure 41 heat conducted from lesion core 1321 into expanded lesion 1323 crosses interface 1308 and damages tissue below interface 1308, including tissue structures 1325.

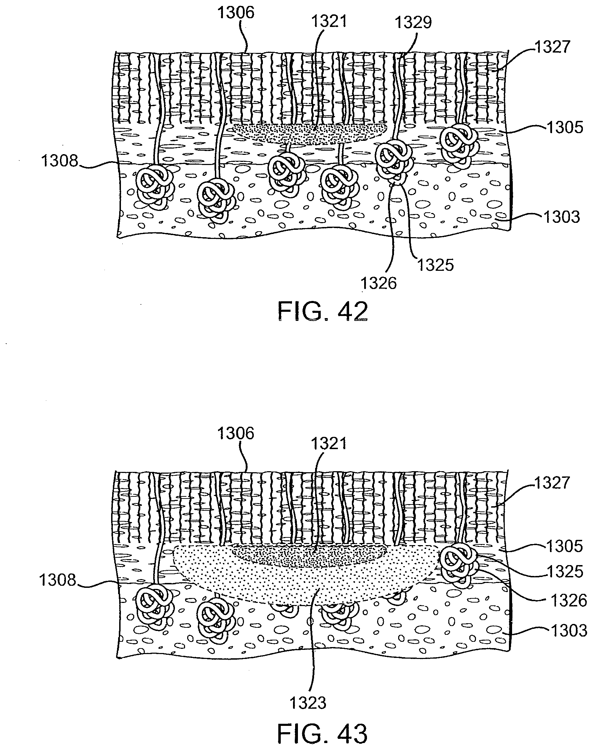

Figure 42 illustrates a tissue profile. In the the tissue profiles illustrated in Figure 42 a lesion core 1321 is created in a predetermined portion of dermis 1305 by, for example, irradiating dermis 1305 with electromagnetic radiation to generate dielectric heating in tissue at lesion core 1321. In the the tissue profile illustrated in Figure 42 lesion core 1321 expands as energy is added to dermis 1305. In the the tissue profile illustrated in Figure 42 heat is removed from skin surface 1306. In the the tissue profileillustrated in Figure 42 heat is removed from dermis 1305 through skin surface 1306. In the the tissue profileillustrated in Figure 42 heat is removed from dermis 1305 through skin surface 1306 by cooling skin surface 1306. In the the tissue profile illustrated in Figure 42 heat removed from dermis 1305 through skin surface 1306 prevents lesion core 1321 from growing in the direction of skin surface 1306. In the the tissue profile illustrated in Figure 42 removed from dermis 1305 through skin surface 1306 prevents lesion core 1321 from growing into cooled region 1327.

Figure 43 illustrates a tissue profile. In the the tissue profile illustrated in Figure 43 a lesion core 1321 is created in a predetermined portion of dermis 1305 by, for example, irradiating dermis 1305 with electromagnetic radiation to generate dielectric heating in tissue at lesion core 1321 and expanded lesion 1323 is created by heat conducted from lesion core 1321. In the the tissue profile illustrated in Figure 43 lesion core 1321 expands as energy is added to dermis 1305 and expanded lesion 1323 expands as heat is conducted from lesion core 1321. In the the tissue profileillustrated in Figure 43 heat is removed from skin surface 1306. In the the tissue profiles illustrated in Figure 43 heat is removed from dermis 1305 through skin surface 1306. In the the tissue profileillustrated in Figure 43 heat is removed from dermis 1305 through skin surface 1306 by cooling skin surface 1306. In the the tissue profile illustrated in Figure 43 heat removed from dermis 1305 through skin surface 1306 prevents lesion core 1321 and expanded lesion 1323 from growing in the direction of skin surface 1306. In the the tissue profile illustrated in Figure 43 removed from dermis 1305 through skin surface 1306 prevents lesion core 1321 and expanded lesion 1323 from growing into cooled region 1327.

### Preferential Heating - Glandular Layer

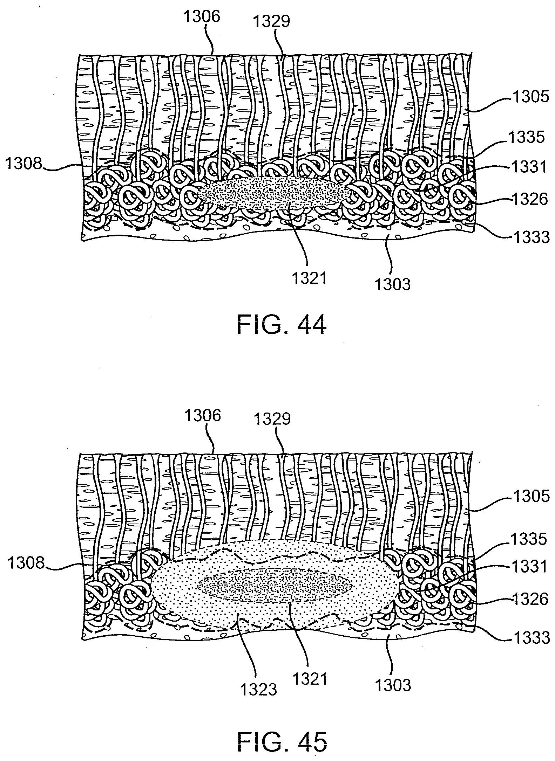

Figures 44 through 47 illustrate tissue profiles. In the tissue profiles illustrated in Figures 44 through 47 dermis 1305 and hypodermis 1303 may contain tissue structures 1325 which may be, for example, sweat glands, including, for example, eccrine glands, apocrine glands or apoeccrine glands. In the tissue illustrated in Figures 44 through 47 dermis 1305 and hypodermis 1303 may contain tissue structures 1325 which maybe, for example, sweat glands, including, for example, eccrine glands, apocrine glands or apoeccrine glands. In the tissue profiles illustrated in Figures 44 through 47 dermis 1305 and hypodermis 1303 may contain tissue structures 1325 which may be, for example, hair follicles. In the tissue profiles illustrated in Figures 44 through 47 tissue structures 1325 may include ducts 1329 extending from tissue structures 1325 to skin surface 1306. In the tissue profiles illustrated in Figures 44 through 47 tissue structures 1325 may be concentrated in a glandular layer 1331. In the tissue profiles illustrated in Figures 44 through 47 tissue structures 1325 may be concentrated in a glandular layer 1331 wherein glandular layer 1331 has an upper interface 1335 and a lower interface 1333. In the tissue profiles illustrated in Figures 44 through 47 glandular layer 1331 may have an upper interface 1335 between glandular layer 1331 and dermis 1305. In the tissue profiles illustrated in Figures 44 through 47 glandular layer 1331 may have a lower interface 1333 between glandular layer 1331 and hypodermis 1303. In the tissue profiles illustrated in Figures 44 through 47 interface 1333 may be, in actual tissue a non-linear, non continuous, rough interface which also includes many tissue structures and groups of tissue structures which add to the roughness and nonlinearity of tissue interface 1333.

In the tissue profiles illustrated in Figures 44 through 47 tissue structures 1325 may be composed, at least in part of high dielectric / high conductivity tissue such as, for example, sweat glands. In the tissue profiles illustrated in Figures 44 through 47 tissue structures 1325 may be composed, at least in part of tissue having a high water content, such as, for example, sweat glands. In the tissue profiles illustrated in Figures 44 through 47 glandular layer 1331 may be composed, at least in part of high dielectric / high conductivity tissue. In the tissue profiles illustrated in Figures 44 through 47 glandular layer 1331 may have an upper interface 1335 between glandular layer 1331 and high dielectric / high conductivity tissue, such as, for example, dermis 1305. In the tissue profiles illustrated in Figures 44 through 47 glandular layer 1331 may have a lower interface 1333 between glandular layer 1331 and low dielectric / low conductivity tissue, such as, for example, hypodermis 1303. In the tissue profiles illustrated in Figures 44 through 47 glandular layer 1331 may have a lower interface 1333 between glandular layer 1331 and low dielectric tissue.

Figure 44 illustrates a tissue profile. In the tissue profile illustrated in Figure 44 a lesion core 1321 is created in a predetermined portion of glandular layer 1331 by, for example, irradiating glandular layer 1331 with electromagnetic radiation to generate dielectric heating in tissue at lesion core 1321. In the tissue profile illustrated in Figure 44 lesion core 1321 is created by heat generated in glandular layer 1331 by dielectric heating of lesion core 1321. In the tissue profile illustrated in Figure 44 lesion core 1321 expands as energy is added to glandular layer 1331. In the tissue profile illustrated in Figure 44 lesion core 1321 may be located in a region of glandular layer 1331 where a constructive interference peak of, for example, SAR, power loss density or temperature, is generated by electromagnetic energy transmitted through skin surface 1306. In the tissue profile illustrated in Figure 44 lesion core 1321 may be located in a region of glandular layer 1331 where a constructive interference peak of, for example, SAR, power loss density or temperature, is generated by electromagnetic energy transmitted through skin surface 1306 wherein at least a portion of the electromagnetic energy transmitted through skin surface 1306 reflects off of lower interface 1333. In the tissue profile illustrated in Figure 44 lesion core 1321 may be located in a region of glandular layer 1331 where a constructive interference peak of, for example, SAR, power loss density or temperature, is generated by electromagnetic energy transmitted through skin surface 1306 wherein at least a portion of the electromagnetic energy transmitted through skin surface 1306 reflects off of lower interface 1333 which may be, for example, an interface between glandular layer 1331 and hypodermis 1303.

Figure 45 illustrates a tissue profile. In the tissue profile illustrated in Figure 45 lesion core 1321 expands as energy is added to glandular layer 1331, generating heat which is conducted into surrounding tissue, creating expanded lesion 1323. In the tissue profile illustrated in Figure 45 heat conducted from lesion core 1321 into expanded lesion 1323 damages tissue, including tissue structures 1325. In the tissue profile illustrated in Figure 45 heat conducted from lesion core 1321 into expanded lesion 1323 crosses lower interface 1333 and damages tissue below lower interface 1333.

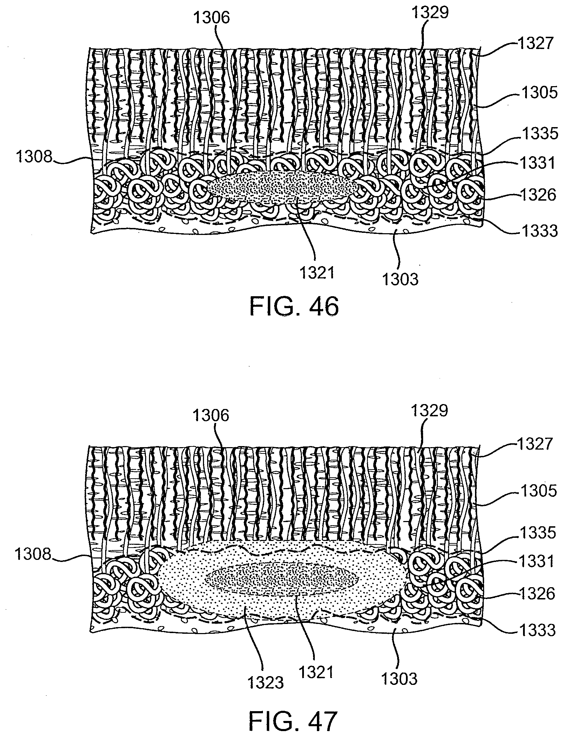

Figures 46 and 47 illustrate tissue profiles. In the tissue profile illustrated in Figures 46 and 47 a lesion core 1321 is created in a portion of glandular layer 1331 by, for example, irradiating glandular layer 1331 with electromagnetic radiation to generate dielectric heating in tissue at lesion core 1321. In the tissue profile illustrated in Figures 46 and 47 lesion core 1321 expands as energy is added to glandular layer 1331 and expanded lesion 1323 is created by heat conducted from lesion core 1323. In the tissue profile illustrated in Figures 46 and 47 heat is removed from skin surface 1306. In the tissue profile illustrated in Figures 46 and 47 heat is removed from dermal layer 1305 through skin surface 1306. In the tissue profile illustrated in Figures 46 and 47 heat is removed from dermal layer 1305 through skin surface 1306 by cooling skin surface 1306, creating cooled region 1307 in dermis 1305. In the tissue profile illustrated in Figure 47 heat removed from dermal layer 1305 through skin surface 1306 prevents expanded lesion 1323 from growing in the direction of skin surface 1306. In the tissue profile illustrated in Figure 46 heat removed from glandular layer 1331 through skin surface 1306 prevents expanded lesion 1323 from growing into cooled region 1327.

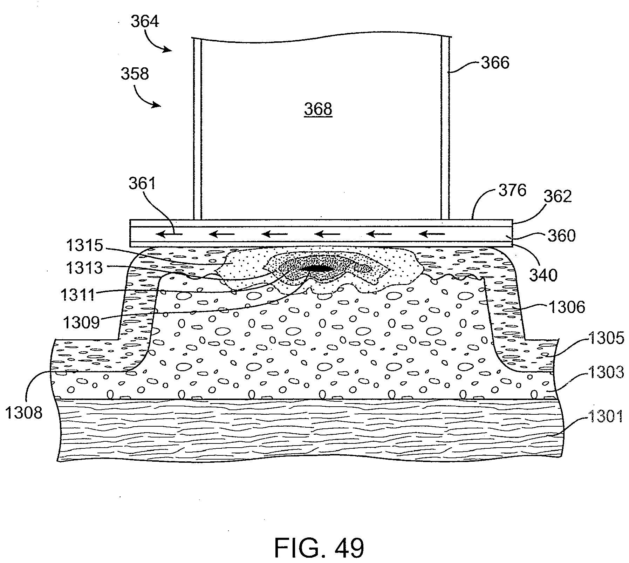

Figures 48 through 51 illustrate tissue profiles and apparatuses according to embodiments of the invention. In Figures 48 through 51, antenna 358 may be, for example, waveguide antenna 364. In the tissue profiles and apparatuses illustrated in Figures 48 and 49, waveguide antenna 364 may include, for example, waveguide tubing 366 and dielectric filler 368. In the tissue profiles and apparatuses illustrated in Figure 48 and 49 electromagnetic energy may be radiated into dermis 1305 through a tissue head 362 which may include, for example, standoff 376, coolant chamber 360 and cooling plate 340. In the tissue profile and apparatus illustrated in Figure 48 a peak which may be, for example, a peak SAR, peak power loss density or peak temperature, is generated in first tissue region 1309. In the tissue profile and apparatus illustrated in Figure 48 a reduced level which may be, for example, a reduced SAR, reduced power loss density or reduced temperature, is generated in second tissue region 1311 with further reduced levels in third tissue region 1313 and fourth tissue region 1315. In the tissue profile and apparatus illustrated in Figure 48 dermis 1305 is separated from hypodermis 1303 by interface 1308. In the tissue profile and apparatus illustrated in Figure 48 interface 1308 is idealized as a substantially straight line for the purposes of this discussion, however, in actual tissue, interface 1308 is a non-linear, non continuous, rough interface which also includes many tissue structures which cross and interrupt the tissue interface. In the tissue profile and apparatus illustrated in Figure 48 hypodermis 1303 lies over muscle tissue 1301. In the tissue profile and apparatus illustrated in Figure 48 electromagnetic radiation may be radiated at a frequency of, for example, 5.8 GHz. In the tissue profile and apparatus illustrated in Figure 48 dermis 1305 may be assumed have a dielectric constant of, for example, 38.4 and a conductivity of, for example 4.54 siemens per meter. In the tissue profile and apparatus illustrated in Figure 48 hypodermis 1303 may be assumed to have, for example, a dielectric constant of, for example, 4.9 and a conductivity of, for example, .31 siemens per meter. In the tissue profile and apparatus illustrated in Figure 48 muscle tissue 1301 may be assumed to have, for example, a dielectric constant of, for example, 42.22 and a conductivity of, for example, 5.2 siemens per meter. In the tissue profile and apparatus illustrated in Figure 48 standoff 376 may be, for example, polycarbonate and may have, for example, a dielectric constant of, for example, 3.4 and a conductivity of, for example, .0051 siemens per meter. In the tissue profile and apparatus illustrated in Figure 48 cooling plate 340 may be, for example, alumina (99.5%) and may have, for example, a dielectric constant of, for example, 9.9 and a conductivity of, for example, 3x10-4 siemens per meter. In the tissue profile and apparatus illustrated in Figure 48 cooling fluid 361 may be, for example, de-ionized water and may have, for example, a dielectric constant of, for example, 81 and a conductivity of, for example, .0001 siemens per meter.

In the tissue profile and apparatus illustrated in Figure 49 a peak, which may be, for example, a peak SAR, peak power loss density or peak temperature, is generated in first tissue region 1309. In the tissue profile and apparatus illustrated in Figure 48 a reduced level which may be, for example, a reduced SAR, reduced power loss density or reduced temperature, is generated in second tissue region 1311 with further reduced levels in third tissue region 1313 and fourth tissue region 1315. In the tissue profile and apparatus illustrated in Figure 49 dermis 1305 is separated from hypodermis 1303 by interface 1308. In the tissue profile and apparatus illustrated in Figure 49 interface 1308 is modeled as a nonlinear interface, to more closely resemble an actual interface between dermal and hypodermal tissue. In the tissue profile and apparatus illustrated in Figure 49 hypodermis 1303 lies over muscle tissue 1301. In the tissue profile and apparatus illustrated in Figure 49 electromagnetic radiation may be radiated at a frequency of, for example, 5.8 GHz. In the tissue profile and apparatus illustrated in Figure 49 dermis 1305 may be assumed to have a dielectric constant of, for example, 38.4 and a conductivity of, for example 4.54 siemens per meter. In the tissue profile and apparatus illustrated in Figure 49 hypodermis 1303 may be assumed to have, for example, a dielectric constant of, for example, 4.9 and a conductivity of, for example, .31 siemens per meter. In the tissue profile and apparatus illustrated in Figure 49 muscle tissue 1301 may be assumed to have, for example, a dielectric constant of, for example, 42.22 and a conductivity of, for example, 5.2 siemens per meter. In the tissue profile and apparatusillustrated in Figure 49 standoff 376 may be, for example, Plexiglas (RTM) and may have, for example, a dielectric constant of, for example, 3.4 and a conductivity of, for example, .0051 siemens per meter. In the tissue profile and apparatus illustrated in Figure 49 cooling plate 340 may be, for example, alumina (99.5%) and may have, for example, a dielectric constant of, for example, 9.9 and a conductivity of, for example, 3x10⁻⁴ siemens per meter. In the tissue profile and apparatus illustrated in Figure 49 cooling fluid 361 may be, for example, de-ionized water and may have, for example, a dielectric constant of, for example, 81 and a conductivity of, for example, .0001 siemens per meter.

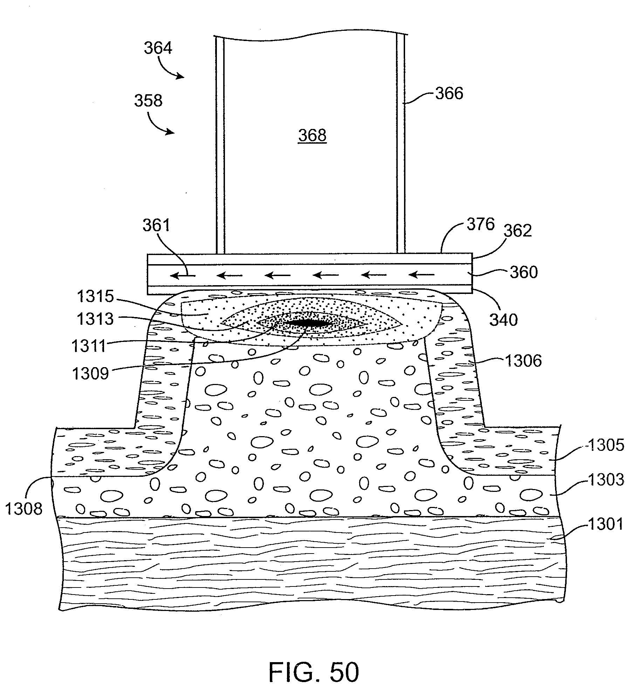

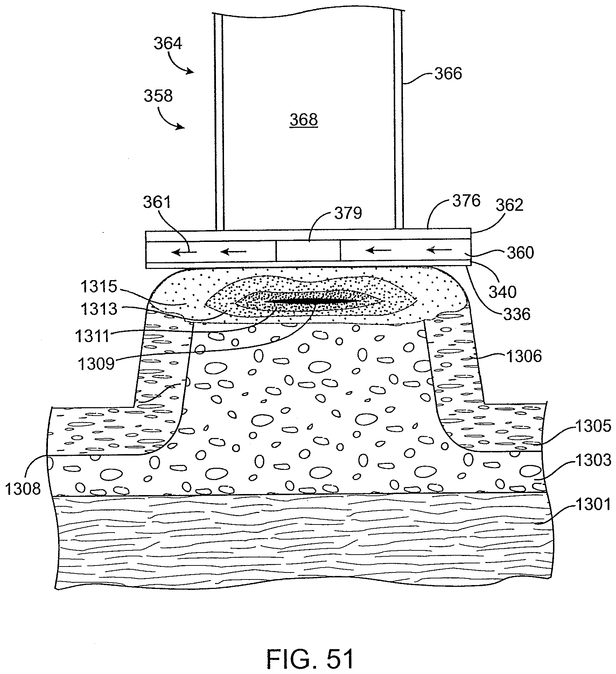

Figure 50 illustrates a tissue profile. Figure 51 illustrates a tissue profile. In the tissue profiles illustrated in Figures 50 and 51, antenna 358 may be, for example, a waveguide antenna 364. Waveguide antenna 364 may have a dielectric filler 368. Antenna 358 may be positioned on, for example, a tissue head 362 comprising, for example, standoff 376, coolant chamber 360 and cooling plate 340. Cooling chamber 340 may contain cooling fluid 361, which may be, for example de-ionized water. A tissue head 362 may include a tissue chamber (not shown) adapted to position tissue against tissue interface 336. In one system, antenna 358 is adapted to transmit electromagnetic radiation through skin surface 1306 creating a tissue profile which may be representative of, for example, a SAR profile, a power loss density profile or a temperature profile. In one system, the tissue profile includes first tissue region 1309, second tissue region 1311, third tissue region 1313 and fourth tissue region 1315. The first tissue region 1309 may represent, for example, a peak SAR, peak power loss density or peak temperature. The first tissue region 1309 may be located in, for example, dermis 1305, near an interface 1308 between dermis 1305 and hypodermis 1303, which overlies muscle 1301. In the tissue profile illustrated in Figure 51, field spreader 379 is located in coolant chamber 360. In the tissue profile illustrated in Figure 51, field spreader 379 may be used to, for example, spread and flatten first tissue region 1309. In the tissue profile illustrated in Figure 51 field spreader 379 may be used to, for example, spread and flatten lesions formed in first tissue region 1309.

### Procedure

In one procedure, electromagnetic power is delivered to the skin for a predetermined period of time. In one procedure skin is engaged in, for example, a tissue chamber prior to the delivery of energy. In one procedure, skin is cooled prior to the application of electromagnetic energy. In one procedure, skin is cooled during the application of electromagnetic energy. In one procedure, skin is cooled after the application of electromagnetic energy. In one procedure, energy is delivered to the skin by applying a predetermined amount of power to an antenna positioned proximal to the surface of the skin. In one procedure, skin is positioned proximal to an electromagnetic energy device. In one procedure, skin is positioned proximal to an electromagnetic energy delivery device using vacuum pressure to hold the skin in position. In one procedure the region to be treated is anesthetized prior to treatment. In one procedure the anesthetized region changes the dielectric properties of the skin. In one procedure, characteristics of the electromagnetic radiation irradiated through the skin are modified to account for variables, such as, for example the dielectric properties of the anesthesia, which determine anesthesia's influence on the treatment. Variables that may determine anesthesia's influence on treatment may include, for example: time from administration; vasodilatation characteristics of anesthetic; volume of anesthesia administered; anesthesia type (liquid injected, topical); location/depth in tissue anesthesia is administered; method of administration, such as, for example, one place or lots of little places. In one procedure, a template may be used to align a handpiece adapted to deliver electromagnetic energy to tissue. In one procedure, a template is used to align a handpiece as the handpiece is moved from position to position in, for example, the axilia. In one procedure, a template is used to align an injection site for the delivery of, for example, anesthesia which may be, for example, lidocaine. In one procedure, a template is used to facilitate treatment by indicating regions which have been previously treated. In one procedure, a template may be aligned by, for example, using henna, sharpie marks or tattoos.

### Tissue Structure

### Regions

Tissue may be made up of layers having particular dielectric and conductivity characteristics. Tissue having a high dielectric constant, also referred to as high dielectric tissue, may have a dielectric constant greater than approximately 25. Tissue having a low dielectric constant, also referred to as low dielectric tissue, may have a dielectric constant less than approximately 10. Tissue having a high conductivity, also referred to as high conductivity tissue, may have a conductivity greater than approximately 1.0 siemens per meter. Tissue having a low conductivity, also referred to as high dielectric tissue, may have a conductivity of less than approximately 1.0 siemens per meter.

### Low/Low

Low dielectric/low conductivity tissue may be, for example the hypodermis. Low dielectric tissue, low conductivity tissue may be tissue found in the hypodermis, such as, for example, fat. Low dielectric/low conductivity tissue may be, for example a region of the hypodermis below a glandular layer.

### High/High

High dielectric, high conductivity tissue may be, for example tissue found in the dermis. High dielectric, high conductivity tissue may be, for example tissue found in the dermis. High dielectric, high conductivity tissue may be, for example, tissue found in a glandular layer. High dielectric, high conductivity tissue may be, for example, muscle tissue.

### Glandular

A glandular layer may be, for example a layer of high dielectric, high conductivity tissue. A glandular layer may be a layer of tissue with high water content. A glandular layer may be a tissue layer in the region of an interface between the dermis and hypodermis which contains sufficient glandular tissue to raise the dielectric constant and conductivity of the glandular layer to a level sufficient to create a standing wave pattern having a peak E-field in the glandular layer. Glandular tissue may occupy an average thickness of three to five millimeters in a five millimeter thick piece of human skin. A glandular layer may include both apocrine gland lobules and eccrine gland lobules within the glandular layer. A glandular layer may be a layer in the human axilla where substantially all of the sweat glands are localized. Wherein a glandular layer includes both apocrine and eccrine gland lobules, the apocrine gland modules may be more numerous and larger than the eccrine gland lobules. A glandular layer may be a layer of tissue which includes a concentration of glands, such as, for example, eccrine, apoeccrine and/or apocrine sweat glands, sufficient to raise the conductivity of tissue surrounding the glands. A glandular layer may be a layer of tissue which includes a concentration of glands, such as, for example, eccrine, apoeccrine and/or apocrine sweat glands, sufficient to raise the dielectric constant of tissue surrounding the glands. A glandular layer may be a region of the hypodermis with sufficient glandular tissue to raise dielectric constant to match the dielectric constant of the adjoining dermis. A glandular layer may be a region of the hypodermis with sufficient glandular tissue to raise dielectric constant of the glandular layer to match the dielectric constant of surrounding hypodermis. A glandular layer may be a region of the hypodermis with sufficient glandular tissue to raise dielectric constant of the glandular layer to exceed the dielectric constant of surrounding hypodermis. A glandular layer may have a dielectric constant of greater than approximately 20. A glandular layer may have a conductivity of greater than approximately 2.5 siemens per meter.

### Interface

A critical interface, which may also be referred to as a dielectric interface or a dielectric discontinuity, may be an interface between a layer of tissue having a high dielectric constant and high conductivity and a layer of tissue having a low dielectric constant. A dielectric interface may be an interface between a layer of tissue having a high dielectric constant and high conductivity and a layer of tissue having a low dielectric constant and low conductivity. A critical interface exists at the interface between the dermis and a glandular layer. A critical interface may be an interface between the dermis and the hypodermis. A critical interface may be an interface between the dermis and a portion of the hypodermis having a limited number of sweat glands. A critical interface may be an interface between the dermis and a region of the hypodermis which does not include a glandular region. A critical interface may be an interface between the dermis and a region of the hypodermis which does not include a significant number of tissue structures.

### Treatment

In some treatments, tissue to be treated may be treated by, for example, raising the temperature of the tissue. In some treatments, tissue to be treated may be treated by, for example, raising the temperature of the tissue to a temperature sufficient to cause a change in the tissue. In some treatments, tissue to be treated may be treated by, for example, raising the temperature of the tissue to a temperature sufficient to damage the tissue. In some treatments, tissue to be treated may be treated by, for example, raising the temperature of the tissue to a temperature sufficient to destroy the tissue. In some treatments, electromagnetic radiation is used to heat tissue to create a lesion where the lesion starts as a result of damage from heat generated by dielectric heating of tissue and the lesion is enlarged at least in part as consequence of thermal conduction of heat generated by the dielectric heating. In some treatments electromagnetic radiation may be used to heat the contents, such as, for example, sebum of a tissue structure, such as, for example a hair follicle. In some treatments electromagnetic radiation may be used to heat the contents, such as, for example, sebum of a tissue structure, such as, for example a hair follicle to a temperature sufficient to damage or destroy, for example, bacteria in the contents. In some treatments electromagnetic radiation may be used to heat the contents, such as, for example, sebum of a tissue structure, such as, for example a hair follicle. In some treatments, electromagnetic radiation may be used to heat tissue to a temperature sufficient to cause secondary effects in surrounding tissue or tissue structures.

### Target Tissue

Tissue to be treated, for example, by raising the temperature of the tissue, may be referred to as target tissue.

### Tissue to be Treated

### Tissue Layers

Target tissue may be tissue adjacent to a dermal / hypodermal interface. Target tissue may be tissue in a dermal layer, proximal to the dermal / hypodermal interface. Target tissue may be in deep dermis tissue. Target tissue may be tissue adjacent to a skin / fat interface.

### Physical Structures

Target tissue may be axillary tissue. Target tissue may be tissue in a hair bearing area. Target tissue may be tissue located in a region having at least 30 sweat glands per square centimeter. Target tissue may be tissue located in a region having an average of 100 sweat glands per square centimeter. Target tissue may be tissue located approximately. 5mm to 6mm below the surface of the skin Target tissue may be tissue located in a region where sweat glands, including, for example, apocrine and eccrine glands.

### Tissue Properties

Target tissue may be tissue subject to dielectric heating. Target tissue may be tissue having a high dipole moment. Tissue to be treated may be, for example, tissue containing exogenous materials. Target tissue may include tissue with bacteria.

### Tissue Types

Target tissue may be human tissue. Target tissue may be porcine tissue. Target tissue may be, for example, collagen, hair follicles, cellulite, eccrine glands, apocrine glands, sebaceous glands or spider veins. Target tissue may be, for example, hair follicles. Target tissue may be, for example, regions of a hair follicle, including the lower segment (bulb and suprabulb), the middle segment (isthmus), and the upper segment (infundibulum). Target tissue may be, for example, structures associated with a hair follicle, such as, for example, stem cells. Target tissue may be, for example, wound tissue. Target tissue may be, for example, tissue to be insulted, as for example, skin tissue prior to surgery. Target tissue may be, for example, blood supply to tissue structures.

### Effect

Target tissue may be, for example, a volume of tissue defined by a region with a SAR equal to fifty percent of peak SAR, at least about 30, 40, 50, 60, 70, 80, or more percent of peak SAR, or no more than about 90, 80, 70, 60, or 50 percent of peak SAR.

### Methods

### Tissue & Structures

In one method a method of treating target tissue is described. In one method a method of damaging glands is described. In one method a method of damaging hair follicles is described. In one method a method of destroying tissue is described. In one method a method of treating skin tissue is described. In one methoda method of preventing damage to tissue is described. In one methoda method of preventing the growth of a lesion toward a skin surface is described. In one method, a method of damaging or destroying stem cells associated with hair follicles is described. In one method, a method of aligning electromagnetic fields to preferentially treat tissue is described. In one method, a method of aligning electromagnetic fields to preferentially treat tissue having a high water content is described. In some methods, electromagnetic energy is used to heat sebum.

### Radiation

In one method, a method of controlling power deposition in tissue is described. In one method, a method of controlling E-field pattern in tissue is described. In one methoda method of creating a volume of high power deposition in tissue is described. In one methoda method of controlling output of a microwave device is described.

### Lesion

In one methoda method of creating a lesion in tissue is described. In one method a method of creating a subdermal lesion in tissue is described.

### Gradients

In one method a method of creating a temperature gradient within tissue is described. In one methoda method of creating a temperature gradient having a peak at the dermal / hypodermal interface is described. In one methoda method of creating an inverse power gradient in tissue is described.

### Clinical Indications

In one method a method of reducing sweat is described. In one method a method of reducing sweat production in a patient is described. In one method a method of treating axillary hyperhidrosis is described. In one method a method of treating hyperhidrosis is described. In one method a method of removing hair is described. In one method a method of preventing the re-growth of hair is described. In one method, a method of treating osmidrosis is described. In one method, a method of denervating tissue is described. In one method, a method of treating port wine stains is described. In one method, a method of treating hemangiomas is described. In one method, a method of treating psoriasis is described. In one method, a method of reducing sweat is described. In one method, a method of reducing sweat is described. In some methods, electromagnetic energy is used to treat acne. In some methods, electromagnetic energy is used to treat sebaceous glands. In some methods, electromagnetic energy is used to destroy bacteria. In some methods, electromagnetic energy is used to destroy propionibacterium. In some methods, electromagnetic energy is used to clear sebum from a hair follicle. In some methods, electromagnetic energy is used to clear obstructed hair follicles. In some methods, electromagnetic energy is used to reverse comedogenesis. In some methods, electromagnetic energy is used to clear blackheads. In some methods, electromagnetic energy is used to clear whiteheads. In some methods, electromagnetic energy is used to reducing inflammation. Further conditions and structures that can be treated in some methods are described in, for example, pp. 3-7 of U.S. Provisional App. No. 60/912,899; and pp. 1-10 of U.S. Provisional App. No. 61/013,274.

### Positioning

In one method a method of positioning skin is described. In one method a method of positioning a skin/fat interface is described.

### POWER LOSS DENSITY

### Skin

In one method, irradiating tissue through the surface of skin with electromagnetic radiation results in a region of localized high power loss density below the skin surface. In one method, irradiating tissue through the surface of skin with electromagnetic radiation results in a region of localized high power loss density in a region of the skin below an upper layer of the skin. In one method, irradiating tissue through the surface of skin with electromagnetic radiation generates a region of localized high power loss density in a layer of the skin adjacent a critical interface. In one method, irradiating tissue through the surface of skin with electromagnetic radiation generates a region of localized high power loss density in a layer of the skin adjacent a critical interface and between the skin surface and the critical interface.

### Dermis

In one method, irradiating tissue through the surface of skin with electromagnetic radiation generates a region of localized high power loss density in a region of the dermis. In one method, radiating the surface of skin with electromagnetic radiation generates a region of localized high power loss density in a region of the dermis below an upper layer of the dermis. In one method, irradiating tissue through the surface of skin with electromagnetic radiation generates a region of localized high power loss density in a region of the dermis adjacent an interface between the dermis and the epidermis. In one method, irradiating tissue through the surface of skin with electromagnetic radiation generates a region of localized high power loss density in a region of the dermis adjacent a critical interface.

### Glandular layer

In one method, irradiating tissue through the surface of skin with electromagnetic radiation generates a region of localized high power loss density in a glandular layer. In one method, irradiating tissue through the surface of skin with electromagnetic radiation generates a region of localized high power loss density in a glandular layer adjacent a critical interface. In one method, irradiating tissue through the surface of skin with electromagnetic radiation generates a region of localized high power loss density in a glandular layer adjacent a critical interface and below a first layer of skin. In one method, irradiating tissue through the surface of skin with electromagnetic radiation generates a region of localized high power loss density in a glandular layer adjacent a critical interface and below at least a portion of the dermis.

### TEMPERATURE GRADIENT

### Skin

In one method, irradiating tissue through the surface of skin with electromagnetic radiation generates a temperature gradient having a peak in a region below the skin surface. In one method, irradiating tissue through the surface of skin with electromagnetic radiation generates a temperature gradient having a peak in a region of the skin below an upper layer of the skin. In one method, irradiating tissue through the surface of skin with electromagnetic radiation generates a temperature gradient having a peak in a layer of the skin adjacent to a critical interface. In one method, irradiating tissue through the surface of skin with electromagnetic radiation generates a temperature gradient having a peak in a layer of the skin adjacent to a critical interface and between a critical interface and the surface of the skin.

### Dermis

In one method, electromagnetic radiation generates a temperature gradient where the temperature gradient has a peak in a layer of the dermis below the surface of the skin. In one method, electromagnetic radiation generates a temperature gradient where the temperature gradient has a peak in a layer of the dermis below an upper layer of the dermis. In one method, electromagnetic radiation generates a temperature gradient where the temperature gradient has a peak in a region of the dermis adjacent an interface between the dermis and the hypodermis. In one method, electromagnetic radiation generates a temperature gradient where the temperature gradient has a peak in a region of the dermis adjacent a critical interface.

### Glandular Layer

In one methodirradiating tissue through the surface of skin with electromagnetic radiation generates a temperature gradient having a peak in a glandular layer below the skin surface. In one method, irradiating tissue through the surface of skin with electromagnetic radiation generates a temperature gradient having a peak in a glandular layer adjacent a critical interface. In one method, irradiating tissue through the surface of skin with electromagnetic radiation generates a temperature gradient having a peak in a glandular layer adjacent a critical interface and below a first layer of skin.

### INVERSE POWER GRADIENT

### Skin

In one method, irradiating tissue through the surface of skin with electromagnetic radiation generates an inverse power gradient having a peak in a region below the skin surface. In one method, irradiating tissue through the surface of skin with electromagnetic radiation generates an inverse power gradient having a peak in a region of the skin below an upper layer of the skin. In one method, irradiating tissue through the surface of skin with electromagnetic radiation generates an inverse power gradient having a peak in a layer of the skin adjacent to a critical interface. In one method, irradiating tissue through the surface of skin with electromagnetic radiation generates an inverse power gradient having a peak in a layer of the skin adjacent to a critical interface and between a critical interface and the surface of the skin.

### Dermis

In one method, electromagnetic radiation generates an inverse power gradient where the inverse power gradient has a peak in a layer of the dermis below the surface of the skin. In one method, electromagnetic radiation generates an inverse power gradient where the inverse power gradient has a peak in a layer of the dermis below an upper layer of the dermis. In one method, electromagnetic radiation generates an inverse power gradient where the inverse power gradient has a peak in a region of the dermis adjacent an interface between the dermis and the hypodermis. In one method, electromagnetic radiation generates an inverse power gradient where the inverse power gradient has a peak in a region of the dermis adjacent a critical interface.

### Glandular Layer

In one methodirradiating tissue through the surface of skin with electromagnetic radiation generates an inverse power gradient having a peak in a glandular layer below the skin surface. In one method, irradiating tissue through the surface of skin with electromagnetic radiation generates an inverse power gradient having a peak in a glandular layer adjacent a critical interface. In one method, irradiating tissue through the surface of skin with electromagnetic radiation generates an inverse power gradient having a peak in a glandular layer adjacent a critical interface and below a first layer of skin.

### LESION

### Skin

In one method, electromagnetic radiation is used to create a lesion in a region below the skin surface. In one method, electromagnetic radiation is used to create a lesion in a region below the skin surface where the lesion starts in a layer below an upper layer of the skin. In one method, electromagnetic radiation is used to create a lesion in skin where the lesion starts in a layer of the skin adjacent a critical interface. In one method, electromagnetic radiation is used to create a lesion in skin where the lesion starts in a layer of the skin adjacent a critical interface and between the skin surface and the critical interface.

### Dermis

In one method, electromagnetic radiation is used to create a lesion in skin where the lesion starts in a layer of the dermis below the surface of the skin. In one method, electromagnetic radiation is used to create a lesion in skin where the lesion starts in a layer of the dermis below an upper layer of the dermis. In one method, electromagnetic radiation is used to create a lesion in skin, where the lesion starts in a region of the dermis proximal to the interface between the dermis and the hypodermis. In one method, electromagnetic radiation is used to create a lesion in skin, where the lesion starts in a region of the dermis adjacent a critical interface.

### Glandular Layer

In one method, electromagnetic radiation is used to create a lesion which starts in a glandular layer. In one method, electromagnetic radiation is used to create a lesion which starts in a glandular layer adjacent a critical interface. In one method, electromagnetic radiation is used to create a lesion which starts in a glandular layer adjacent a critical interface and below a first layer of skin.

### Skin

In one method, electromagnetic radiation is used to create a lesion in a region below the skin surface in the absence of any external mechanism for removing heat from the surface of the skin. In one method, electromagnetic radiation is used to create a lesion in a region below the skin surface where the lesion starts in a layer below an upper layer of the skin in the absence of any external mechanism for removing heat from the surface of the skin. In one method, electromagnetic radiation is used to create a lesion in skin where the lesion starts in a layer of the skin adjacent a critical interface in the absence of any external mechanism for removing heat from the surface of the skin. In one method, electromagnetic radiation is used to create a lesion in skin where the lesion starts in a layer of the skin adjacent a critical interface and between the skin surface and the critical interface in the absence of any external mechanism for removing heat from the surface of the skin.

### Dermis

In one method, electromagnetic radiation is used to create a lesion in skin where the lesion starts in a layer of the dermis below the surface of the skin in the absence of any external mechanism for removing heat from the surface of the skin. In one method, electromagnetic radiation is used to create a lesion in skin where the lesion starts in a layer of the dermis below an upper layer of the dermis in the absence of any external mechanism for removing heat from the surface of the skin. In one method, electromagnetic radiation is used to create a lesion in skin, where the lesion starts in a region of the dermis proximal to the interface between the dermis and the hypodermis in the absence of any external mechanism for removing heat from the surface of the skin. In one method, electromagnetic radiation is used to create a lesion in skin, where the lesion starts in a region of the dermis adjacent a critical interface in the absence of any external mechanism for removing heat from the surface of the skin.

### Glandular Layer

In one method, electromagnetic radiation is used to create a lesion which starts in a glandular layer in the absence of any external mechanism for removing heat from the surface of the skin. In one method, electromagnetic radiation is used to create a lesion which starts in a glandular layer adjacent a critical interface in the absence of any external mechanism for removing heat from the surface of the skin. In one method, electromagnetic radiation is used to create a lesion which starts in a glandular layer adjacent a critical interface and below a first layer of skin in the absence of any external mechanism for removing heat from the surface of the skin.

### Lesion Origin

In one method, a lesion origin may be located at a point or region in high dielectric, high conductivity tissue adjacent low dielectric tissue. In one method, a lesion origin may be located at a point or region in high dielectric, high conductivity tissue adjacent a critical interface. In one method, the lesion origin may be located at a point or region where microwave energy radiated through the surface of the skin generates a standing wave pattern having a peak E-field. In one method, the lesion origin may be located in high dielectric/high conductivity tissue near a critical interface where microwave energy radiated through the surface of the skin generates constructive interference.

### ELECTROMAGNETIC RADIATION CHARACTERISTICS

In one method, skin is irradiated by electromagnetic radiation having specific characteristics and, more particularly, specific E-field characteristics. In one method, skin is irradiated with electromagnetic radiation wherein the E-field component of the electromagnetic radiation is substantially parallel to the skin's outer surface. In one method, skin is irradiated with electromagnetic radiation wherein the E-field component of the electromagnetic radiation is substantially parallel to at least one interface between tissue layers within the skin. In one method, skin is irradiated with electromagnetic radiation wherein the E-field component of the electromagnetic radiation is substantially parallel to a critical interface. In one method, skin is irradiated with electromagnetic radiation wherein the E-field component of the electromagnetic radiation is substantially parallel to the interface between the dermis and the hypodermis. In one method, skin is irradiated with electromagnetic radiation wherein the E-field component of the electromagnetic radiation is substantially parallel to the interface between a glandular layer and a portion of the hypodermis.

In one method, skin is irradiated by electromagnetic radiation having specific characteristics and, more particularly, specific polarization characteristics. In one method, skin is irradiated with electromagnetic radiation wherein the electromagnetic radiation is polarized such that the E-field component of the electromagnetic radiation is substantially parallel to the skin's outer surface. In one method, skin is irradiated with electromagnetic radiation wherein the electromagnetic radiation is polarized such that the E-field component of the electromagnetic radiation is substantially parallel to at least one interface between tissue layers within the skin. In one method, skin is irradiated with electromagnetic radiation wherein the electromagnetic radiation is polarized such that the E-field component of the electromagnetic radiation is substantially parallel to the interface between the dermis and the hypodermis. In one method, skin is irradiated with electromagnetic radiation wherein the electromagnetic radiation is polarized such that the E-field component of the electromagnetic radiation is substantially parallel to an interface between a glandular layer and the hypodermis.

In one method, skin is irradiated by electromagnetic radiation having specific characteristics and, more particularly, specific frequency characteristics. In one method, skin is irradiated by electromagnetic radiation having a frequency of approximately 5.8 GHz. In one method, skin is irradiated by electromagnetic radiation having a frequency of between 5 GHz and 6.5 GHz. In one method, skin is irradiated by electromagnetic radiation having a frequency of between 4.0 GHz and 10 GHz.

In one method, skin is irradiated by electromagnetic radiation having specific characteristics and, more particularly, specific characteristics within tissue. In one method, skin is irradiated by electromagnetic radiation which generates a constructive interference pattern having a peak within the skin. In one method, skin is irradiated by electromagnetic radiation which generates a constructive interference pattern in the dermis, wherein the constructive interference pattern has a peak in a region of the dermis which is below a first layer of the dermis and where destructive interference occurs in the first layer of the dermis. In one method, skin is irradiated by electromagnetic radiation which generates a constructive interference pattern, wherein the constructive interference pattern has a peak adjacent a critical interface. In one method, skin is irradiated by electromagnetic radiation which generates a constructive interference pattern having a peak in a glandular layer. In one method, skin is irradiated by electromagnetic radiation having specific characteristics and, more particularly, specific characteristics within skin. In one method, skin is irradiated by electromagnetic radiation which generates a destructive interference pattern within the skin. In one method, skin is irradiated by electromagnetic radiation which generates a destructive interference pattern in the dermis, wherein the destructive interference pattern has a peak in a region of the dermis which is above a deep layer of the dermis. In one method, skin is irradiated by electromagnetic radiation which generates a destructive interference pattern, wherein the destructive interference pattern has a peak adjacent a critical interface. In one method, skin is irradiated by electromagnetic radiation which generates a destructive interference pattern having a peak in a glandular layer. In one method, skin irradiated with electromagnetic radiation generates a constructive interference pattern that generates a peak electric field in a tissue layer. In one method, skin irradiated with electromagnetic radiation generates a constructive interference pattern that generates a region of localized high power loss density. In one method, skin irradiated with electromagnetic radiation generates a destructive interference pattern that generates a minimum electric field in a tissue layer. In one method, skin irradiated with electromagnetic radiation generates a destructive interference pattern that generates a region of localized low power loss density.

In one method, skin is irradiated by electromagnetic radiation having specific characteristics and, more particularly, specific characteristics within tissue. In one method, skin is irradiated by electromagnetic radiation which generates a standing wave pattern within the skin. In one method, skin is irradiated by electromagnetic radiation which generates a standing wave pattern having a peak in the dermis below a first layer of the dermis. In one method, skin is irradiated by electromagnetic radiation which generates a standing wave pattern having a peak adjacent a critical interface. In one method, skin is irradiated by electromagnetic radiation which generates a standing wave pattern having a peak in a glandular layer. In one method, skin irradiated with electromagnetic radiation generates a standing wave pattern that generates a peak electric field. In one method, skin irradiated with electromagnetic radiation generates a standing wave pattern that generates a region of localized high power loss density.

### ANTENNA

In one method, skin is irradiated by electromagnetic radiation having specific characteristics and, more particularly, specific characteristics resulting from the position of the antenna radiating the electromagnetic radiation. In one method, skin is irradiated by electromagnetic radiation generated by an antenna positioned proximal to the skin surface. In one method, skin is irradiated by an antenna located in the radiating near field region with respect to the surface of adjacent skin. In one method, skin is irradiated by an antenna located substantially in the radiating near field region with respect to the surface of adjacent skin. In one method, skin is irradiated by an antenna located less than one half of one wavelength from the surface of adjacent skin. In one method, skin is irradiated by an antenna located less than one half of one wavelength from the surface of adjacent skin, wherein a wavelength is measured in dielectric material separating the antenna from the skin surface. In one method, skin is irradiated by an antenna located less than one half of one wavelength from the surface of adjacent skin, wherein a wavelength is measured in cooling fluid separating the antenna from the skin surface. In one method, skin is irradiated by an antenna located less approximately 2.65 millimeters from the skin surface. In one method, wavelength of a radiated signal is the wavelength in air divided by the square root of the dielectric constant of materials separating the antenna from the skin surface. In one method, wavelength of a radiated signal is the wavelength in air divided by the square root of the dielectric constant of cooling fluid separating the antenna from the skin surface.

In one method, skin is irradiated by electromagnetic radiation having specific characteristics and, more particularly, specific characteristics resulting from the position of the output of the antenna radiating the electromagnetic radiation. In one method, skin is irradiated by an antenna having an output in the radiating near field region with respect to the surface of adjacent skin. In one method, skin is irradiated by an antenna having an output outside the reactive near field region with respect to the surface of adjacent skin. In one method, skin is irradiated by an antenna having an output which is not in the far field region with respect to the surface of adjacent skin.

In one method, skin is irradiated by electromagnetic radiation having specific characteristics and, more particularly, specific characteristics related to the position of a radiating aperture in the antenna radiating the electromagnetic radiation. In one method, skin is irradiated by an antenna having a radiating aperture in the radiating near field region with respect to the surface of adjacent skin. In one method, skin is irradiated by an antenna having a radiating aperture outside the reactive near field with respect to the surface of adjacent skin. In one method, skin is irradiated by an antenna having a radiating aperture which is not in the far field region with respect to the surface of adjacent skin.

In one method, a reactive near field region may be, for example, that portion of the near field region immediately surrounding the antenna where the near reactive field predominates. In one method, an antenna may be located a distance from a skin surface which may be approximately 0.62 times the square root of D²/Lambda, where D is the largest physical dimension of the antenna aperture and Lambda is the wavelength of the electromagnetic radiation transmitted by the antenna measured in the medium positioned between the antenna output and skin surface. In one method, a radiating near field region may be, for example, that that region of the field of an antenna between the reactive near field region and the far field region wherein radiation fields predominate. In one method, an antenna may be located a maximum distance from a skin surface which may be approximately 2 times D²/Lambda, where D is the largest physical dimension of the antenna aperture and Lambda is the wavelength of the electromagnetic radiation transmitted by the antenna measured in the medium positioned between the antenna output and skin surface. In one method, a far field region may be, for example, that region of the field of an antenna where the angular field distribution is essentially independent of the distance from the antenna.

In one method, skin is irradiated by electromagnetic radiation having specific characteristics and, more particularly, specific characteristics resulting from the configuration of the antenna which radiates the electromagnetic radiation. In one method, skin is irradiated by an antenna configured to radiate a field pattern primarily in the TE₁₀ mode. In one method, skin is irradiated by an antenna configured to radiate a field pattern solely in TE₁₀ mode. In one method, skin is irradiated by an antenna configured to radiate a field pattern in the TEM mode. In one method, skin is irradiated by an antenna configured to radiate a field pattern solely in TEM mode. In some methods, TEM and TE₁₀ are particularly useful as they are modes in which radiated electromagnetic energy includes E-Fields in the transverse direction. Thus, where an antenna is positioned appropriately, an antenna transmitting electromagnetic energy in a TEM or TE₁₀ mode will generate an E-field which may be parallel or substantially parallel to a skin surface adjacent the antenna or to a critical interface, such as, for example, an interface between the dermis and the hypodermis.

In one method, skin is irradiated by electromagnetic radiation having specific characteristics and, more particularly, specific characteristics resulting from the configuration of the antenna which radiates the electromagnetic radiation. In one method, skin is irradiated by an antenna configured to radiate a field pattern primarily in the TEM mode. In one method, skin is irradiated by an antenna configured to radiate a field pattern solely in TEM mode.

In one method, skin is irradiated by electromagnetic radiation having specific characteristics and, more particularly, specific characteristics resulting from the configuration of the antenna which radiates the electromagnetic radiation. In one method, skin is irradiated by an antenna configured to radiate electromagnetic energy having an E-field component which is substantially parallel to the surface of the skin. In one method, skin is irradiated by an antenna configured to radiate electromagnetic energy having an E-field component which is substantially parallel to a critical interface. In one method, skin is irradiated by an antenna configured to radiate electromagnetic energy having an E-field component which is substantially parallel to the interface between the dermis and the hypodermis. In one method, skin is irradiated by an antenna configured to radiate electromagnetic energy having an E-field component which is substantially parallel to an interface between a glandular region and a portion of the hypodermis.

In one method, skin is irradiated by electromagnetic radiation having specific characteristics and, more particularly, specific characteristics resulting from the configuration of the antenna which radiates the electromagnetic radiation. In one method, skin is irradiated by an antenna configured to generate a standing wave in adjacent tissue. In one method, skin is irradiated by an antenna configured to generate a standing wave in adjacent tissue wherein the standing wave has a peak adjacent a critical interface.

In one method, skin is irradiated by electromagnetic radiation having specific characteristics and, more particularly, specific characteristics resulting from the configuration of the antenna which radiates the electromagnetic radiation. In one method, skin is irradiated by an antenna configured to generate constructive interference in adjacent tissue. In one method, skin is irradiated by an antenna configured to generate constructive interference in adjacent tissue wherein the constructive interference has a peak adjacent a critical interface.

### HEATING TISSUE / TISSUE STRUCTURES

In one method, tissue is heated by conducting heat generated in a lesion to specified tissue. In one method, tissue is heated by conducting heat generated in a lesion through intermediate tissue wherein heat in the lesion is generated primarily by dielectric heating. In one method, tissue located below a critical interface is heated by conducting heat generated in a lesion across the critical interface. In one method, a method is described for heating tissue located below a critical interface by conducting heat generated in a lesion located above the critical interface wherein heat generated in the lesion is generated primarily by dielectric heating and heat below the critical interface is generated primarily by conduction of heat from the lesion through intermediate tissue to tissue located below a dielectric barrier.

In one method, tissue structures, such as, for example sweat glands or hair follicles, located in the region of the skin near a critical interface are heated. In one method, tissue structures located near a critical interface are heated by conduction of heat from a lesion, wherein the lesion is created by dielectric heating. In one method, tissue structures located in a first tissue layer are heated by heat generated in the first tissue layer as a result of a standing wave generated in the first tissue layer by reflections off a critical interface

In one method, tissue structures located in the region of the skin where the dermis and hypodermis layer meet are heated. In one method, tissue structures located in a glandular layer are heated. In one method, tissue structures located in the region of the skin where the dermis and hypodermis layer meet are damaged. In one method, tissue structures located in a glandular layer are damaged. In one method, tissue structures located in the region of the skin where the dermis and hypodermis layer meet are destroyed. In one method, tissue structures located in a glandular layer are destroyed. In one method, tissue elements are heated by conducting heat generated in a lesion through intermediate tissue to the tissue elements, wherein heat in the lesion is generated primarily by dielectric heating. In one method, tissue structures located below a critical interface are heated by conducting heat generated in a lesion above the critical interface primarily by dielectric heating through intermediate tissue to tissue structures located below a the critical interface.

In one method a region adjacent a critical interface may be heated by depositing more energy into that region than into surrounding tissue.

In one method, tissue in the dermal layer, proximal to the interface between the dermal layer and the subdermal layer is preferentially heated.

### COOLING

In one method, heat generated in tissue below the surface of the skin is prevented from damaging tissue adjacent the surface of the skin by removing heat from the surface of the skin. In one method, heat generated in tissue below the surface of the skin is prevented from damaging tissue adjacent the surface of the skin by cooling the surface of the skin.

One method is described for preventing heat generated in a lesion by dielectric heating from damaging tissue in a skin layer positioned between the lesion and the surface of the skin. One method is described for preventing heat generated in a lesion by dielectric heating from damaging tissue in a skin layer positioned between the lesion and the surface of the skin by removing heat from a skin surface. One method is described for preventing heat generated in a lesion by dielectric heating from damaging tissue in a skin layer positioned between the lesion and the surface of the skin by cooling a skin surface.

One method is described for preventing heat generated in a lesion having an origin in a layer of tissue from damaging tissue in a layer of tissue positioned between the lesion origin and the surface of the skin. One method is described for preventing heat generated in a lesion having an origin in a layer of tissue from damaging tissue in a skin layer positioned between the lesion and the surface of the skin by removing heat from a skin surface. One method is described for preventing heat generated in a lesion having an origin in a layer of tissue from damaging tissue in a skin layer positioned between the lesion and the surface of the skin by cooling a skin surface.

One method is described for preventing lesion from growing toward the surface of the skin. One method is described for preventing lesion from growing toward the surface of the skin by removing heat from the skin surface. One method is described for preventing lesion from growing toward the surface of the skin by cooling the skin surface.

In one method, cooling may be turned off for a period after energy is delivered and resumed thereafter. In one method, cooling may be turned off for a period of for example, approximately 2 seconds after energy is delivered. In one method, cooling turned on and off in a pulsed manner to control the amount of heat removed through the skin surface.

### Antenna System

### Antenna Types

In the antenna system, antenna 358 may be, for example: a coaxial single slot antenna; a coaxial multiple slot antenna; a printed slot antenna; a waveguide antenna; a horn antenna; a patch antenna; a patch trace antenna; a Vivaldi antenna; or a waveguide antenna. In the antenna system, an antenna may be, for example an array of antennas. In the antenna system, an antenna may be, for example an array of antennas wherein one or more of the antennas radiate electromagnetic energy at the same time. In the antenna system, an antenna may be, for example an array of antennas wherein at least one but not all of the antennas radiate electromagnetic energy at the same time. In the antenna system, an antenna may be, for example, two or more different types of antennas. In the antenna system, specific antennas in an array may be selectively activated or deactivated.

### Return Loss / Bandwidth

In one antenna system, an antenna has an optimized return loss (S11) profile centered on 5.8GHz. A scattering parameter or return loss (the magnitude of S11 in dB) is a measure of reflected power measured at antenna feed divided by the power into antenna feed, which it may be used as an efficiency measurement. In one antenna system, an antenna has an optimal coupling value may be, for example, -15dB or below, which corresponds to 97% power coupling. At 97% power coupling, 97% of the input power available to the antenna (e.g. from a microwave generator) is coupled into the antenna's input port. Alternatively, in one antenna system, an antenna has an optimal coupling value of, for example, -10dB or below, which corresponds to 90% power coupling. Alternatively, in one antenna system, an antenna has an optimal coupling value which may be, for example, -7dB or below, which corresponds to 80% power coupling. In one antenna system, an antenna, such as, for example, a wave guide antenna may include tuning screws. In one antenna system, tuning screws may be used to, for example, match return loss (magnitude of S₁₁) for the expected load.

In one antenna system, an antenna is optimized to maintain the power coupled into the antenna with a return of - 10dB or better over an optimal frequency band. An optimal bandwidth may be, for example, approximately .25 GHz (.125GHz on either side of the center frequency), at frequencies of interest, such as, for example, 5.8GHz. An optimal bandwidth may be, for example, approximately 1.0 GHz (0.5 GHz on either side of the center frequency), at frequencies of interest, such as, for example, 5.8GHz.

### Dielectric Filler

In the systems, dielectric filler 368 may have a dielectric constant of approximately 10. In the systems, dielectric filler may have a dielectric constant of between approximately 9.7 and 10.3. In the system, dielectric filler may be impervious to fluid, including cooling fluid in cooling chamber. In the system, dielectric filler may be configured to prevent liquid from entering waveguide tubing. In the system, dielectric filler may be configured to efficiently couple energy from an antenna feed into tissue. In the system, dielectric filler may be configured to match a waveguide tubing, coolant chamber, including cooling fluid and skin at a predetermined frequency of, for example frequencies in the range of: between approximately 4 GHz and 10 GHz; between approximately 5 GHz and 6.5 GHZ; or frequencies of approximately 5.8GHz. In the system, dielectric filler may be configured to generate a field having minimal electric field perpendicular to a tissue surface. In the system, dielectric filler may be configured to generate a TE₁₀ field in target tissue frequencies in the range of: between approximately 4 GHz and 10 GHz; between approximately 5 GHz and 6.5 GHZ; or frequencies of approximately 5.8GHz.

In one system, a waveguide cross sectional inner geometry, such as, for example, a WR62, having a width of 15.8 millimeters and a height of 7.9 millimeters is optimized at a predetermined frequency by selecting an appropriate dielectric filler. In one system, an antenna, such as a WR62 is optimized at a predetermined frequency by selecting an appropriate filler material. In one system, an antenna, such as a WR62 is optimized at a predetermined frequency by selecting a filler material having a dielectric constant in the range of between 3 and 12. In one system, an antenna, such as a WR62 is optimized at a predetermined frequency by selecting a filler material having a dielectric constant of approximately 10. In one system, an antenna, such as a WR62, is optimized at a predetermined frequency by selecting a dielectric filler material which is impervious to fluids, such as, for example cooling fluids. In one system, an antenna, such as a WR62, is optimized at a predetermined frequency by selecting a dielectric filler material which is, for example, Eccostock. In one system, an antenna, may be optimized at a predetermined frequency by selecting a dielectric filler material which is, for example, polycarbonate, Teflon, plastic or air.

### Field Spreader

In one system, an antenna may include a dielectric element, which may be referred to as a field spreader, at the antenna output that perturbs or scatters the microwave signal in such a way that the E-field is applied to tissue over a wider area. In one field spreader system, the field spreader causes the E-field to diverge as it exits the antenna. In one field spreader system, a field spreader may have a dielectric constant of between 1 and 80. In one field spreader system, a field spreader may have a dielectric constant of between 1 and 15. In the field spreader systems, field spreaders may be used to, for example, spread and flatten peak SAR regions, peak temperature regions or peak power loss density regions in tissue. In the field spreader system, field spreaders may be used to, for example, spread and flatten lesions in tissue.

In one field spreader system, a field spreader may be a dielectric element. In one field spreader system, a field spreader may be configured to spread an E-field. In one field spreader system, a field spreader may be configured to extend from an output of an antenna to a cooling plate. In one field spreader system, a field spreader may be configured to extend from a dielectric filler to a cooling plate. In one field spreader system, a field spreader may be positioned at least partially in a cooling chamber. In one field spreader system, a field spreader may be positioned at least partially in a cooling fluid. In one field spreader system, a field spreader may be configured to have rounded features. In one field spreader system, a field spreader may be oval. In one field spreader system, a field spreader positioned at least partially in a cooling fluid may have a countered shape. In one field spreader system, a field spreader positioned at least partially in a cooling fluid may be configured to prevent eddy currents in the cooling fluid. In one field spreader system, a field spreader positioned at least partially in a cooling fluid may be configured to prevent air bubbles from forming in the cooling fluid. In one field spreader system, a system may have multiple field spreaders.

In one field spreader system, a field spreader may be configured to have a dielectric constant which matches a dielectric filler. In one field spreader system, a field spreader may be configured to have a dielectric constant which differs from a dielectric filler. In one field spreader system, a field spreader may be configured to increase the effective field size (EFS) by reducing field strength in center of a waveguide. In one field spreader system, a field spreader may be configured to increase the ratio of 50% SAR contour area at depth in the target tissue and the surface area of the radiating aperture by reducing field strength in center of a waveguide. In one field spreader system, a field spreader may be configured to cause a signal emitted from an antenna to diverge around the field spreader creating local E-field peaks that re-combine to form larger SAR regions. In one field spreader system, a field spreader may have a cross sectional which is between approximately two percent and 50 percent of the inner face of a waveguide antenna. In one field spreader system the field spreader may have a rectangular cross section. In one field spreader system, the field spreader may have a rectangular cross section of 6 millimeters by 10 millimeters. In one field spreader system, the field spreader may have a rectangular cross section of 6 millimeters by 10 millimeters when used with a waveguide having an inner face of 15.8 millimeters by 7.9 millimeters. In one field spreader system, the field spreader may have a rectangular cross section of approximately 60 square millimeters. In one field spreader system, the field spreader may have a rectangular cross section of approximately 60 square millimeters when used with a waveguide having an inner face with an area of approximately 124 square millimeters. In one field spreader system, the field spreader may be comprised of, for example, alumina, having a dielectric constant of, for example 10. In one field spreader system, a field spreader may be configured to consist of a dielectric region embedded in a waveguide. In one field spreader system, a field spreader may be configured to consist of a dielectric region positioned in a cooling chamber. In one field spreader system, a field spreader may be configured to consist of a notch in dielectric filler. In one field spreader system, a field spreader may be configured to consist of a notch in dielectric filler which is configured to allow cooling fluid, such as, for example, water to flow in the notch. In one field spreader system, a field spreader may be configured to consist of cooling fluid. In one field spreader system, a field spreader may be configured to consist of one or more air gaps.

### Efficiency / Fringing

In one system, an antenna, such as, for example, a waveguide antenna, is optimized to reduce or eliminate free space radiation due to fringing fields. In one system, an antenna, such as, for example, a waveguide antenna, is optimized to redirect fringing fields towards tissue. In one system, an antenna, such as, for example, a waveguide antenna is optimized to improve the efficiency of the antenna, which efficiency may be measured by, for example comparing the energy available at the input of the antenna to the energy which is coupled into adjacent tissue. In one system, an antenna, such as, for example, a waveguide antenna is optimized to improve the efficiency of the antenna such that at least seventy percent of the energy available at the input of the antenna is deposited in tissue adjacent to an output of the antenna. In one system, an antenna, such as, for example, a waveguide antenna, may be optimized by positioning the output of the antenna such that an outer edge of the waveguide antenna is in contact with fluid. In one system, an antenna, such as, for example, a waveguide antenna, may be optimized by positioning the output of the antenna such that the output of the antenna is in contact with fluid. In one system, an antenna, such as, for example, a waveguide antenna, may be optimized by positioning the output of the antenna such that an output of the antenna is covered by an insulator which separates the output from a fluid, the insulator having a thickness which reduces the free space radiation due to the fringing fields at the output of the waveguide. In one system, an antenna, such as, for example, a waveguide antenna, may be optimized by positioning the output of the antenna such that an output of the antenna is covered by an insulator which separates the output from a fluid, such as, for example, a cooling fluid the insulator having a thickness of less than .005". In one system power transfer from an antenna, such as, for example, a waveguide antenna, through a cooling fluid and into adjacent tissue is optimized by reducing the thickness of an isolation layer between the output of the antenna and the cooling fluid. In one systempower transfer from an antenna, such as, for example, a waveguide antenna, through a cooling fluid and into adjacent tissue is optimized by placing the output of the antenna into the cooling fluid. In one systeman antenna, such as, for example, a waveguide antenna, may be optimized by covering the output of the antenna with an insulator, such as, for example, polycarbonate having a dielectric constant which is less than a dielectric constant of an antenna filler material. In one systeman antenna, such as, for example, a waveguide antenna, may be optimized by covering the output of the antenna with an insulator, such as, for example, polycarbonate having a dielectric constant which is less than a dielectric constant of an antenna filler material, the thickness of the insulator being between approximately .0001" and .006". In one system an antenna, such as, for example, a waveguide antenna, may be optimized by covering the output of the antenna with an insulator the thickness of the insulator being between approximately .015". In one systeman antenna, such as, for example, a waveguide antenna, may be optimized by covering the output of the antenna with an insulator, such as, for example, polycarbonate having a dielectric constant which is less than a dielectric constant of an antenna filler material, the thickness of the insulator being between approximately .0001" and .004". In one systeman antenna, such as, for example, a waveguide antenna, may be optimized by covering the output of the antenna with an insulator, such as, for example, polycarbonate having a dielectric constant which is less than a dielectric constant of an antenna filler material, the thickness of the insulator is approximately .002". In one systeman antenna, such as, for example, a waveguide antenna, may be optimized by covering the output of the antenna with an insulator, such as, for example, alumina having a dielectric constant which is substantially equal to the a dielectric constant of an antenna filler material.

### Polarization / TE₁₀

In one system, an antenna, such as, for example, a waveguide antenna may be optimized by, for example, optimizing the design of the antenna to ensure that the antenna broadcasts in a substantially pure TE₁₀ mode.

### Cooling System

In one cooling system, a cooling system is placed between a device adapted to emit electromagnetic radiation and skin. In one cooling system, a cooling system includes a cooling fluid and a cooling plate. In one cooling system, a cooling system includes a cooling fluid flowing past a cooling plate. In one cooling system, a cooling fluid flows through a cooling chamber. In one cooling system, a cooling fluid flows through a cooling chamber which is positioned between a device adapted to emit electromagnetic radiation and a cooling plate. Other cooling systems and various components that may be used with systems and devices described herein are described and illustrated, for example, at Figs. 33-36 and pp. 40-45 of U.S. Provisional App. No. 60/912,899; and Figs. 11A-11B and pp. 21-24 of U.S. Provisional App. No. 61/013,274, also published as PCT/US08/60929, as well as illustrated and described, for example, in Figs. 33-36 and pp. 42-46 of Appendix 1 and Figs. 11A-11B and pp. 27-29 of Appendix 2.

### Temperature

In one cooling system, a cooling system is optimized to maintain the skin surface at a predetermined temperature. In one cooling system, a cooling system is optimized to maintain the skin surface at a temperature of less than 45°C. In one cooling system, a cooling system is optimized to maintain the skin surface at a temperature of less than 40°C. In one cooling system, a cooling system is optimized to maintain the skin surface at a temperature of approximately 22°C. In one cooling system, a cooling system is optimized to maintain a cooling plate at a temperature of less than 40°C. In one cooling system, a cooling system is optimized to maintain the skin surface at a temperature of less than 45°C. In one cooling system, cooling fluid is used remove heat from the cooling system.

### Cooling Fluid

In one system, moving cooling fluid is used to remove heat from the cooling system. In one system, cooling fluid has a temperature of between -5°C and 40°C as it enters a cooling chamber in the cooling system. In one system, cooling fluid has a temperature of between 10 and 25°C as it enters a cooling chamber in the cooling system. In one system, cooling fluid has a temperature of approximately 22°C as it enters a cooling chamber in the cooling system. In one system, cooling fluid has a flow rate of at least 100 milliliters per second as it moves through a cooling chamber. In one system, cooling fluid has a flow rate of between 250 and 450 milliliters per second as it moves through a cooling chamber. In one system, cooling fluid has a velocity of between 0.18 and 0.32 meters per second as it moves through a cooling chamber. In one system, coolant flow in a cooling chamber is non-laminar. In one system, coolant flow in a cooling chamber is turbulent to facilitate heat transfer. In one system, cooling fluid has a Reynolds number of between approximately 1232 and 2057 prior to entering a cooling chamber. In one system, cooling fluid has a Reynolds number of between approximately 5144 and 9256 prior to entering a cooling chamber.

In one system, cooling fluid is optimized to be substantially transparent to microwave energy. In one system, cooling fluid is optimized to minimize absorption of electromagnetic energy. In one system, cooling fluid is optimized to match an antenna to tissue. In one system, cooling fluid is optimized to facilitate the efficient transfer of microwave energy to tissue. In one system, cooling fluid is optimized to conduct heat away from the surface of skin. In one system, cooling fluid is comprised of a fluid having a high dielectric constant. In one system, a cooling fluid is optimized to have a high dielectric constant of between 70 and 90. In one system, a cooling fluid is optimized to have a high dielectric constant of approximately 80. In one system, a cooling fluid is optimized to have a low dielectric constant of between 2 and 10. In one system, a cooling fluid is optimized to have a low dielectric constant of approximately 2. In one system, a cooling fluid is optimized to have a dielectric constant of approximately 80. In one system, cooling fluid is comprised, at least in part, of de-ionized water. In one system, cooling fluid is comprised, at least in part, of alcohol. In one system, cooling fluid is comprised, at least in part, of ethylene glycol. In one system, cooling fluid is comprised, at least in part, of glycerol. In one system, cooling fluid is comprised, at least in part, of a germicide. In one system, cooling fluid is comprised, at least in part of vegetable oil. In one system, cooling fluid is comprised of a fluid having a low electrical conductivity. In one system, cooling fluid is comprised of a fluid having an electrical conductivity of less than approximately 0.5 siemens per meter.

### Cooling Plate

In the systems, a cooling plate may be, for example configured: to contact skin; cool skin tissue; to physically separate skin tissue from a microwave antenna; to conform to the hair bearing region of the axilla of a human; to constitute a thermoelectric cooler; to be thermally conductive; to be substantially transparent to microwave energy; to be thin enough to minimize microwave reflection; to be composed of ceramic; or to be composed of alumina.

In one system, a cooling plate is optimized to conduct electromagnetic energy to tissue. In one system, a cooling plate is optimized to conduct heat from the surface of skin into a cooling fluid. In one system, a cooling plate is optimized to have a thickness of between .0035" (0.0889 mm) and .025' (0.635 mm)', and may include thickness of up to .225" (0.5715mm). In one system, a cooling plate is optimized to have a dielectric constant of between 2 and 15. In one system, a cooling plate is optimized to have a dielectric constant of approximately 10. In one system, a cooling plate is optimized to have a low electrical conductivity. In one system, a cooling plate is optimized to have an electrical conductivity of less than 0.5 siemens per meter. In one system, a cooling plate is optimized to have a high thermal conductivity. In one system, a cooling plate is optimized to have a thermal conductivity of between 18 and 50 Watts per meter-Kelvin at room temperature. In one system, a cooling plate is optimized to have a thermal conductivity of between 10 and 100 Watts per meter-Kelvin at room temperature. In one system, a cooling plate is optimized to have a thermal conductivity of between .1 and 5 Watts per meter-Kelvin at room temperature. In one system, a cooling plate is comprised, at least in part, of a ceramic material. In one system, a cooling plate is comprised, at least in part, of alumina.

In one system, a cooling plate may be, for example, a thin film polymer material. In one system, a cooling plate may be, for example, a polyimide material. In one system, a cooling plate may be, for example, a material having a conductivity of approximately .12 Watts per meter-Kelvin and a thickness of between approximately .002" (0.051mm) and (0.25mm)

### Cooling Chamber

In one cooling chamber system, a cooling chamber has a thickness which is optimized for the electromagnetic radiation frequency, cooling fluid composition and cooling plate composition. In one cooling chamber system, a cooling chamber has a thickness which is optimized for a high dielectric cooling fluid. In one cooling chamber system, a cooling chamber has a thickness which is optimized for a cooling fluid having a dielectric constant of approximately 80, such as, for example, de-ionized water. In one cooling chamber system, a cooling chamber has a thickness of between 0.5 and 1.5 millimeters. In one cooling chamber system, a cooling chamber has a thickness of approximately 1.0 millimeters. In one cooling chamber system, a cooling chamber has a thickness which is optimized for a low dielectric cooling fluid. In one cooling chamber system, a cooling chamber has a thickness which is optimized for a cooling fluid which has a dielectric constant of approximately 2, such as, for example, vegetable oil. Low dielectric, low conductivity cooling fluids may be advantageous where it is desirable to limit the losses or to match elements. In one cooling chamber system, a cooling chamber is optimized such that eddy currents are minimized as fluid flows through the cooling chamber. In one cooling chamber system, a cooling chamber is optimized such that air bubbles are minimized as fluid flows through the cooling chamber. In one cooling chamber system, field spreaders located in the cooling chamber are positioned and designed to optimize laminar flow of cooling fluid through the cooling chamber. In one cooling chamber system, field spreaders located in the cooling chamber are substantially oval in shape. In one cooling chamber system, field spreaders located in the cooling chamber are substantially round in shape. In one cooling chamber system, field spreaders located in the cooling chamber are substantially rectangular in shape.

### Thermoelectric Module

In one cooling system, a cooling system optimized to maintain the skin surface at a predetermined temperature may be, for example a thermoelectric module. In one cooling system, a cooling system is optimized to maintain the skin surface at a predetermined temperature by attaching the cold plate side of a thermoelectric cooler(s) (TEC) to a face of the cooling plate adjacent to the waveguide antenna(s). The hot side of the TEC(s) is attached to a finned heat sink(s) that is acted upon by an axial fan(s) in order to maintain the hot side of the TEC(s) at a low temperature to optimize the cooling performance of the TEC(s). The attachment of the TEC(s) to the cooling plate and heat sink(s) utilizes ceramic thermal adhesive epoxy. For example, the TEC(s) may be part number 06311-5L31-03CFL, available from Custom Thermoelectric, the heat sink(s) may be part number 655-53AB, available from Wakefield Engineering, the ceramic thermal adhesive epoxy may be available from Arctic Silver and the axial fans(s) may be part number 1608KL-04W-B59-L00 available from NMB-MAT.

In one cooling system, a cooling system is optimized to maintain the skin surface at a predetermined temperature by constructing the cold plate side of a thermoelectric cooler(s) (TEC) as the cooling plate adjacent to or surrounding the waveguide antenna(s) with an opening(s) in the hot side of the TEC(s) where the waveguide antenna(s) exist. The hot side of the TEC(s) is attached to a finned heat sink(s) that is acted upon by an axial fan(s) in order to maintain the hot side of the TEC(s) at a low temperature to optimize the cooling performance of the TEC(s). The attachment of the TEC(s) to the heat sink(s) utilizes ceramic thermal adhesive epoxy. For example, the TEC(s) may be available from Laird Technology, the heat sink(s) may be part number 655-53AB, available from Wakefield Engineering, the ceramic thermal adhesive epoxy may be available from Arctic Silver and the axial fans(s) may be part number 1608KL-04W-B59-L00 available from NMB-MAT.

In one cooling system, a cooling system is optimized to maintain the skin surface at a predetermined temperature by attaching the cold plate side of a thermoelectric cooler(s) (TEC) to a side(s) of the waveguide antenna(s). The hot side of the TEC(s) is attached to a finned heat sink(s) that is acted upon by an axial fan(s) in order to maintain the hot side of the TEC(s) at a low temperature to optimize the cooling performance of the TEC(s). The attachment of the TEC(s) to the waveguide antenna(s) and heat sink(s) utilizes ceramic thermal adhesive epoxy. For example, the TEC(s) may be part number 06311-5L31-03CFL, available from Custom Thermoelectric, the heat sink(s) may be part number 655-53AB, available from Wakefield Engineering, the ceramic thermal adhesive epoxy may be available from Arctic Silver and the axial fans(s) may be part number 1608KL-04W-B59-L00 available from NMB-MAT.

### Energy

In one system, energy is delivered to the skin for a period of time which optimizes the desired tissue effect. In one esystem, energy is delivered to the skin for a period of between 3 and 4 seconds. In one system, energy is delivered to the skin for a period of between 1 and 6 seconds. In one system, energy is delivered to a target region in tissue. In one systemenergy delivered to the target region for a time sufficient to result in an energy density at the target tissue of between 0.1 and 0.2 Joules per cubic millimeter. In one systemenergy delivered to the target region for a time sufficient to heat the target tissue to a temperature of at least 75°C. In one systemenergy delivered to the target region for a time sufficient to heat the target tissue to a temperature of between 55 and 75°C. In one systemenergy delivered to the target region for a time sufficient to heat the target tissue to a temperature of at least 45°C.

### Cooling

In one system, the skin surface is cooled for a period of time which optimizes the desired tissue effect. In one system, the skin surface is cooled during the time energy is delivered to the skin. In one system, the skin surface is cooled for a period of time prior to the time energy is delivered to the skin. In one system, the skin surface is cooled for a period of between 1 and 5 seconds prior to the time energy is delivered to the skin. In one system, the skin surface is cooled for a period of approximately 2 seconds prior to the time energy is delivered to the skin. In one system, the skin surface is cooled for a period of time after to the time energy is delivered to the skin. In one system, the skin surface is cooled for a period of between 10 and 20 seconds after the time energy is delivered to the skin. In one system, the skin surface is cooled for a period of approximately 20 seconds after the time energy is delivered to the skin.

### Output power

In one system, power is delivered to a device adapted to radiate electromagnetic energy. In one system, power is delivered to an input to an antenna, such as, for example, the feed to a waveguide antenna. In one system, the power available at the antenna's input port is between 50 and 65 Watts. In one system, the power available at the antenna's input port is between 40 and 70 Watts. In one system, power available at the antenna's input port varies over time.

### Tissue Acquisition

In one system, skin is held in an optimal position with respect to an energy delivery device. In one system, skin is held in an optimal position with respect to an energy delivery device using vacuum pressure. In one system, skin is held in an optimal position with respect to an energy delivery device using vacuum pressure of between 400 and 750 millimeters of mercury. In one system, skin is held in an optimal position with respect to an energy delivery device using vacuum pressure of approximately 650 millimeters of mercury. Other tissue acquisition systems, methods, and devices that can be used with the system to hold the skin in place and/or protect non-target tissue structures can be found, for example, at Figs. 38-52C and pp. 46-57 of U.S. Provisional App. No. 60/912,899; and Figs. 12-16B and pp. 24-29 of U.S. Provisional App. No. 61/013,274 also published as PCT/US08/60929, as well as illustrated and described, for example, in Figs. 38-52C and pp. 46-55 of Appendix 1 and Figs. 12-16B and pp. 29-33 of Appendix 2.

### TISSUE INTERFACE

### Tissue Chamber

In one tissue chamber system, a tissue chamber may be, for example, a suction chamber. In one tissue chamber system, a tissue chamber may be configured to acquire at least a portion of the skin tissue. In one tissue chamber system, a tissue chamber may be operatively coupled to a vacuum source. In one tissue chamber system, a tissue chamber may be configured with at least one tapered wall. In one tissue chamber system, a tissue chamber may be configured to at least partially acquire skin tissue and bring skin tissue in contact with cooling plate. In one tissue chamber system, tissue chamber 338 may be configured to include at least one suction element. In one tissue chamber system, tissue chamber 338 may be configured to elevate skin and placing skin in contact with a cooling element. In one tissue chamber system, tissue chamber 338 may be configured to elevate skin and placing skin in contact with a cooling element. In one tissue chamber system, tissue chamber 338 may be configured to elevate skin and placing skin in contact with a suction chamber. In one tissue chamber system, tissue chamber 338 may be configured to elevate skin and placing skin in contact with suction openings. In one tissue chamber system, suction openings may include at least one channel wherein the channel may have rounded edges. In one tissue chamber system, tissue chamber 338 may have an ovoid or racetrack shape wherein the tissue chamber includes straight edges perpendicular to direction of cooling fluid flow. In one tissue chamber system, tissue chamber 338 may be configured to elevate skin separating skin tissue from underlying muscle tissue. In one tissue chamber system, tissue chamber 338 may be configured to include at least one temperature sensor. In one tissue chamber system, tissue chamber 338 may be configured to include at least one temperature sensor wherein the temperature sensor may be a thermocouple. In one tissue chamber system, tissue chamber 338 may be configured to include at least one temperature sensor wherein the temperature sensor is configured to monitor the temperature at skin surface. In one tissue chamber system, tissue chamber 338 may be configured to include at least one temperature sensor wherein the temperature sensor is configured such that it dose not significantly perturb a microwave signal.

In one system, a tissue interface may comprise a tissue chamber which is optimized to separate skin from underlying muscle. In one system, a tissue interface may comprise a vacuum chamber which is optimized to separate skin from underlying muscle when skin is pulled into a tissue chamber by, for example, vacuum pressure. In one system, a tissue chamber may be optimized to have a depth of between approximately 1 millimeter and approximately 30 millimeters. In one system, a tissue chamber may be optimized to have a depth of approximately 7.5 millimeters. In one system, walls of a tissue chamber may be optimized to have an angle of between approximately 2 and 45 degrees. In one system, walls of a tissue chamber may be optimized to have a chamber angle Z of between approximately 5 and 20 degrees. In one system, walls of a tissue chamber may be optimized to have a chamber angle Z of approximately 20°. In one tissue chamber system, a tissue chamber may be optimized to have an ovoid shape. In one tissue chamber system, a tissue chamber may be optimized to have a racetrack shape. In one tissue chamber system, a tissue chamber may be optimized to have an aspect ratio wherein the aspect ratio may be defined as the minimum dimension of a tissue interface surface to the height of the vacuum chamber. In the tissue chamber systemillustrated in Figure 8, the aspect ratio may be, for example the ratio between minimum dimension 10 and tissue depth Y. In one tissue chamber system, a tissue chamber may be optimized to have an aspect ratio of between approximately 1:1 and approximately 3:1. In one tissue chamber system, a tissue chamber may be optimized to have an aspect ratio of approximately 2:1.

### Staged Treatments

In some systems, it may be desirable to perform the treatment in stages. Additionally, the treatment may be patterned such that sections of target tissue are treated in the initial stage while other sections are treated in subsequent stages. Treatments using systems and devices disclosed herein may, for example, be treated in stages as disclosed in, for example, at Figs. 54-57 and pp. 61-63 of U.S. Provisional App. No. 60/912,899; and Figs. 17-19 and pp. 32-34 of U.S. Provisional App. No. 61/013,274..

### Diagnosis

Systems of the present invention also include methods and apparatuses for identifying and diagnosing patients with hyperhidrosis. Such diagnosis can be made based on subjective patient data (e.g., patient responses to questions regarding observed sweating) or objective testing. In one system of objective testing, an iodine solution can be applied to the patient to identify where on a skin surface a patient is sweating and not sweating. Moreover, particular patients can be diagnosed based on excessive sweating in different parts of the body in order to specifically identify which areas to be treated. Accordingly, the treatment may be applied only selectively to different parts of the body requiring treatment, including, for example, selectively in the hands, armpits, feet and/or face.

### Quantifying Treatment Success

Following completion of any of the treatments described above, or any stage of a treatment, the success can be evaluated qualitatively by the patient, or may be evaluated quantitatively by any number of ways. For example, a measurement can be taken of the number of sweat glands disabled or destroyed per surface area treated. Such evaluation could be performed by imaging the treated area or by determining the amount of treatment administered to the treated area (e.g. the quantity of energy delivered, the measured temperature of the target tissue, etc.). The aforementioned iodine solution test may also be employed to determine the extent of treatment effect. In addition, a treatment can be initiated or modified such that the amount of sweating experienced by a patient may be reduced by a desired percentage as compared to pre-treatment under defined testing criteria. For example, for a patient diagnosed with a particularly severe case of hyperhidrosis, the amount of sweating may be reduced by about 10%, 20%, 30%, 40%, 50%, 60%, 70%, 80%, 90% or more. For a patient diagnosed with a less severe or more normal sweating profile, a stepwise reduction of sweating may be achieved, but with less resolution. For example, such a patient may only be able to achieve partial anhidrosis in 25% increments.

### Overview of Systems, Methods, and Devices

One method of applying energy to tissue is described. One method includes the step of generating a radiation pattern with a region of localized high power loss density in skin. One method includes the step of generating a radiation pattern with a region of localized high power loss density in a region of the dermis adjacent a critical interface. One method includes the step of generating a radiation pattern with a region of localized high power loss density in a glandular layer. One method includes the step of generating a radiation pattern in skin with first and second regions of localized high power loss density wherein the first and second regions are separated by a region of low power loss density. One method includes the step of generating a radiation pattern with a plurality of regions of localized high power loss density in skin wherein the first and second regions are separated by a region of low power loss density. One method includes the step of generating a radiation pattern with a plurality of regions of localized high power loss density in skin wherein adjacent regions of high power loss density are separated by regions of low power loss density.

One method of applying energy to tissue is described. One method includes the step of generating a radiation pattern with a region of localized high specific absorption rate (SAR) in skin. One method includes the step of generating a radiation pattern with a region of localized high specific absorption rate (SAR) in a region of the dermis adjacent a critical interface. One method includes the step of generating a radiation pattern with a region of localized high specific absorption rate (SAR) in a glandular layer. One method includes the step of generating a radiation pattern in skin with first and second regions of localized specific absorption rate (SAR) wherein the first and second regions are separated by a region of low specific absorption rate (SAR). One method includes the step of generating a radiation pattern with a plurality of regions of localized high specific absorption rate (SAR) in skin wherein the first and second regions are separated by a region of low specific absorption rate (SAROne method includes the step of generating a radiation pattern with a plurality of regions of localized specific absorption rate (SAR) in skin wherein adjacent regions of high specific absorption rate (SAR) are separated by regions of low specific absorption rate (SAR).

One method of applying energy to tissue is described. One method includes the step of generating a radiation pattern with a region of localized high temperature in skin. One method includes the step of generating a radiation pattern with a region of localized high temperature in a region of the dermis adjacent a critical interface. One method includes the step of generating a radiation pattern with a region of localized high temperature in a glandular layer. One method includes the step of generating a radiation pattern in skin with first and second regions of localized temperature wherein the first and second regions are separated by a region of low temperature. One method includes the step of generating a radiation pattern with a plurality of regions of localized high temperature in skin wherein the first and second regions are separated by a region of low temperature. One method includes the step of generating a radiation pattern with a plurality of regions of localized temperature in skin wherein adjacent regions of high temperature are separated by regions of low temperature.

In one method, a method of aligning electromagnetic field to preferentially treat tissue having a relatively high water content is described. One method includes the steps of irradiating tissue with an electromagnetic Electric field aligned with a surface of the skin. One method includes irradiating tissue with electromagnetic radiation in the TE₁₀ mode. One method includes irradiating tissue with electromagnetic radiation having a minimal E-field in a direction perpendicular to at least a portion of a skin surface. One method includes aligning an E-field component of an electromagnetic wave to preferentially heat tissue having a high water content by irradiating with transverse electric (TE) or transverse electromagnetic (TEM) waves.

One method for controlling the delivery of energy to tissue is described. One method of delivering energy includes the step of delivering energy at a frequency of approximately 5.8GHz. One method of delivering energy includes the step of delivering energy having a power of greater than approximately 40 Watts. One method of delivering energy includes the step of delivering energy for a period of between approximately 2 seconds and approximately 10 seconds. One method of delivering energy includes the step of pre-cooling skin surface for a period of approximately 2 seconds. One method of delivering energy includes the step of post cooling for a period of approximately 20 seconds. One method of delivering energy includes the step of maintaining tissue engagement for a period of more than approximately 22 seconds. One method of delivering energy includes the step of engaging tissue using a vacuum pressure of approximately 600 millimeters of mercury. One method of delivering energy includes the step of measuring skin temperature. One method of delivering energy includes the step of adjusting energy delivery duration; pre-cooling duration; post-cooling duration; output power; frequency; vacuum pressure as a result of feedback of tissue parameters such as, for example, skin temperature. One method of delivering energy includes the step of adjusting energy delivery duration; pre-cooling duration; post-cooling duration; output power; frequency; vacuum pressure as a result of feedback of tissue parameters such as, for example, cooling fluid temperature.

One method of removing heat from tissue is described. One method of cooling tissue is described, the method including engaging the surface of the skin. One method includes the step of positioning a cooling element in contact with the skin surface. One method includes the step of conductively cooling the skin surface. One method includes the step of convectively cooling the skin surface. One method includes the step of conductively and convectively cooling the skin surface

Onemethod of damaging or destroying tissue structures is described. Onemethod of damaging or destroying glands is described. One method includes the step of inducing hyperthermia in the tissue structures. In one method, hyperthermia may be accomplished by mild heating of tissue to a temperature of, for example, between approximately 42°C and 45°C. One method includes the step of ablating tissue structures may be accomplished by heating of tissue to temperatures in excess of approximately 47°C.

One method of treating tissue using electromagnetic radiation is described. Onemethod of treating tissue includes creating a secondary effect in tissue. One method of treating tissue includes creating a secondary effect in tissue wherein the secondary effect includes, for example, reducing bacterial colonization. One method of treating tissue includes creating a secondary effect in tissue wherein the secondary effect includes clearing or reducing skin blemishes. One method of treating tissue includes creating a secondary effect in tissue wherein the secondary effect includes clearing or reducing skin blemishes resulting from, for example, acne vulgaris. One a method of treating tissue includes damaging sebaceous glands. One method of treating tissue includes disabling sebaceous glands. One method of treating tissue includes temporarily disabling sebaceous glands.

One method of delivering energy to selected tissue is described. One method includes delivering energy via a microwave energy delivery applicator. One method involves delivering energy sufficient to create a thermal effect in a target tissue within the skin tissue. One method includes the step of delivering energy to tissue which is subject to dielectric heating. One method includes the step of delivering energy to tissue having a high dielectric moment. One method includes delivering energy to target tissue within the skin tissue selected from the group consisting of collagen, hair follicles, cellulite, eccrine glands, apocrine glands, sebaceous glands, spider veins and combinations thereof. In one system, target tissue within the skin tissue comprises the interface between the dermal layer and subcutaneous layer of the skin tissue. In one method, creating a thermal effect in the target tissue comprises thermal alteration of at least one sweat gland. In one method, creating a thermal effect in the target tissue comprises ablation of at least one sweat gland.

One method of delivering microwave energy to tissue is described. One method includes the step of applying a cooling element to the skin tissue. One method includes the step of applying microwave energy to tissue at a power, frequency and duration and applying cooling at a temperature and a duration sufficient to create a lesion proximate interface between the dermis layer and subcutaneous layer in the skin tissue while minimizing thermal alteration to non-target tissue in the epidermis and dermis layers of the skin tissue. One method includes the step of applying microwave energy to a second layer of skin containing sweat glands sufficient to thermally alter the sweat glands. One method includes the step of applying microwave energy while the first layer of skin is protectively cooled, the second layer being deeper than the first layer relative to the skin surface. One method includes the step of cooling via a cooling element.

One method includes the step of using one or more field spreaders to spread the MW energy as it emerges from an antenna. One method includes creating a contiguous lesion larger than a single waveguide lesion. One method includes the step of using multiple antennas. One method includes the step of creating a contiguous lesion larger than a single waveguide lesion. One method includes the step of using an array of waveguides. One method includes the step of activating a plurality of waveguides in series. One method includes the step of activating multiple antennas. One method includes the step of activating less than all antennas in an array. One method includes the step of continuously cooling under all antennas in an array.

One method of applying energy to tissue is described. One method includes the step of applying energy at a depth deeper than a skin surface. One method includes the step of applying energy but not as deep as nerve or muscle tissue. One method includes the step of applying electromagnetic radiation at a frequency which concentrates energy at target tissue

One method of selectively heating tissue is described. One method includes the step of selectively heating tissue by dielectric heating. One method includes the step of selectively heating glands. One method includes the step of selectively heating glandular fluid. One method includes the step of heating tissue to a temperature sufficient to damage a gland. One method includes the step of heating the gland to a temperature sufficient to result in morbidity. One method includes the step of heating the gland to a temperature sufficient to result in death. One method includes the step of heating the gland to a temperature sufficient to damage adjacent hair follicles. One method includes the step of heating the gland to a temperature sufficient to destroy adjacent hair follicles. One method includes the step of heating the gland to a temperature sufficient to induce hyperthermia in tissue at the skin / fat interface. One method includes the step of heating the gland to a temperature sufficient to induce hyperthermia in tissue at the skin / fat interface while minimizing hyperthermia in surrounding tissue. I One method includes the step of heating the gland to at least 50°C.

One method of generating a temperature profile in skin tissue is described. One method includes generating a temperature profile having a peak in region directly above skin - fat interface. One method includes the step of generating a temperature profile wherein the temperature declines towards the skin surface. One method includes the step of generating a temperature profile wherein the temperature declines towards the skin surface in the absence of cooling.

One method of positioning skin is described. One method includes the step of using suction, pinching or adhesive. One method includes the step of using suction, pinching or adhesive to lift a dermal and subdermal layer away from a muscle layer.

One method of applying energy to tissue is described. One method includes the step of placing a microwave energy delivery applicator over the skin tissue. In one system, the microwave applicator includes a microwave antenna. In one system, the microwave antenna is selected from the group consisting of: single slot, multiple slot, waveguide, horn, printed slot, patch, Vivaldi antennas and combinations thereof. One method includes the step of positioning the microwave energy delivery applicator over a region having more absorptive tissue elements. One method includes the step of positioning the microwave energy delivery applicator over a region having a concentration of sweat glands. One method includes the step of positioning the microwave energy delivery applicator over a hair bearing area. One method includes the step of positioning the microwave energy delivery applicator over an axilla. One method includes the step of acquiring skin within a suction chamber. One method includes the step of activating a vacuum pump. One method includes the step of deactivating a vacuum pump to release skin. One method includes the step of securing skin tissue proximate to the microwave energy delivery applicator. One method includes the step of securing skin tissue proximate to the microwave energy delivery applicator by applying suction to the skin tissue. One method includes the step of securing skin tissue proximate to the microwave energy delivery applicator includes the step of at least partially acquiring the skin tissue within a suction chamber adjacent to the energy delivery applicator. One method includes the step of using a lubricant to enhance vacuum. One method includes the step of securing skin tissue proximate to the microwave energy delivery applicator includes the step of elevating the skin tissue. One method includes the step of securing skin tissue proximate to the microwave energy delivery applicator includes the step of brining skin in contact with cooling. One method includes the step of activating a vacuum pump to acquire the skin within a suction chamber.

In one system, disclosed herein is a system for the application of microwave energy to a tissue, including a signal generator adapted to generate a microwave signal having predetermined characteristics; an applicator connected to the generator and adapted to apply microwave energy to tissue, the applicator comprising one or more microwave antennas and a tissue interface; a vacuum source connected to the tissue interface; a cooling source connected to said tissue interface; and a controller adapted to control the signal generator, the vacuum source, and the coolant source. In some systems, the microwave signal has a frequency in the range of between about 4 GHz and about 10 GHz, between about 5 GHz and about 6.5 GHz, or about 5.8 GHz. The system can further comprise an amplifier connected between the signal generator and the applicator. The microwave antenna may comprise an antenna configured to radiate electromagnetic radiation polarized such that an E-field component of the electromagnetic radiation is substantially parallel to an outer surface of the tissue. In some systems, the microwave antenna comprises a waveguide antenna. The antenna may comprise an antenna configured to radiate in TE10 mode, and /or TEM mode. The tissue interface is configured to engage and hold skin. The skin is of the axillary region in some systems. The microwave antenna may comprise an antenna configured to radiate electromagnetic radiation polarized such that an E-field component of the electromagnetic radiation is parallel to an outer surface of the tissue.

In some systems, the tissue interface comprises a cooling plate and a cooling chamber positioned between the cooling plate and the microwave antenna. In some systems, the cooling plate has a dielectric constant between about 2 and 15. The vacuum source can be configured to supply vacuum pressure to the tissue interface. In some systems, the vacuum pressure is between about 400 mmHg to about 750 mmHg, or about 650 mmHg in some systems. The cooling source can be configured to supply a coolant to the tissue interface. The coolant can be a cooling fluid, which in some systemshas a dielectric constant of between about 70 and 90, about 80, between about 2 and 10, or about 2. In some systems, the cooling fluid can have a temperature of between about -5°C and 40 °C, 10°C and 25 °C, or about 22 °C. In some systems, the cooling fluid has a flow rate through at least a portion of the tissue interface of between about 100mL and 600mL per second, or between about 250mL and 450mL per second. In some systems, the cooling fluid is configured to flow through the tissue interface at a velocity of between 0.18 and 0.32 meters per second. The cooling fluid can be selected from, e.g., glycerin, vegetable oil, isopropyl alcohol, water, water mixed with alcohol, or other combinations in some systems. The cooling source may comprise a thermoelectric module. In some systems, the tissue comprises a first layer and a second layer, the second layer below the first layer, wherein the controller is configured such that the system delivers energy such that a peak power loss density profile is created in the second layer.

In another system, disclosed is an apparatus for delivering microwave energy to target tissue, the apparatus comprising a tissue interface; a microwave energy delivery device; a cooling element positioned between the tissue interface and the microwave energy device, the cooling element comprising a cooling plate positioned at the tissue interface; and a cooling fluid positioned between the cooling element and the microwave delivery device, the cooling fluid having a dielectric constant greater than a dielectric constant of the cooling element. In some systems, the tissue interface comprises a tissue acquisition chamber, which can be a vacuum chamber in some systems. The cooling plate may be made of ceramic. In some systems, the cooling plate is configured to contact a skin surface about the target tissue, cool the skin tissue, and physically separate the skin tissue from the cooling fluid. In some systems, the microwave energy delivery device comprises a microwave antenna, which may be a waveguide antenna in some systems.

In another system, disclosed is an apparatus for delivering microwave energy to a target region in tissue, the apparatus comprising: a tissue interface having a tissue acquisition chamber; a cooling element having a cooling plate; and a microwave energy delivery device having a microwave antenna. In some systems, the tissue acquisition chamber comprises a vacuum chamber adapted to elevate tissue, including the target region, and bring the tissue in contact with the cooling element. In some systems, the vacuum chamber has a racetrack shape comprising a first side and a second side, the first and second sides parallel to each other, and a first end and a second end, the first and second ends having arcuate shapes. In some systems, the cooling plate is configured to contact a skin surface above the target tissue, cool the skin tissue, and physically separate the skin tissue from the microwave energy delivery device. The cooling plate may be substantially transparent to microwave energy. In some systems, the microwave antenna is configured to deliver sufficient energy to the target region to create a thermal effect. In some systems, the microwave antenna comprises a waveguide antenna.

Also disclosed, in one system, is an apparatus for delivering microwave energy to a target region in tissue, the apparatus comprising a vacuum chamber adapted to elevate tissue including the target region and bring the tissue into contact with a cooling plate, wherein the cooling plate is adapted to contact a skin surface above the target region, cool the skin surface, and physically separate the skin tissue from the microwave energy delivery device; and a microwave antenna configured to deliver sufficient energy to the target region to create a thermal effect. In some systems, the vacuum chamber may have a race track shape comprising a first side and a second side, the first and second sides parallel to each other; and a first end and a second end, the first and second ends having arcuate shapes. In some systems, the cooling plate is substantially transparent to microwave energy. In some systems, the microwave antenna is configured to deliver sufficient energy to the target region to create a thermal effect. In some systems, the microwave antenna comprises a waveguide antenna. In some systems, the microwave antenna is configured to generate a radiation pattern having a peak at the target region.

Also disclosed, in one system, is a system for coupling microwave energy into tissue, the system comprising a microwave antenna, a fluid chamber positioned between the microwave antenna and the tissue, and a cooling plate positioned between the cooling chamber and the tissue. In one system, the system further comprises at least one field spreader. The field spreader may be positioned within the fluid chamber between the waveguide and the cooling plate. The field spreader may be configured to facilitate laminar flow of fluid through the fluid chamber. In one system, the field spreader may be configured to prevent one or more of eddy currents or air bubbles within the cooling fluid. In one aspect, the system may further comprise a cooling fluid selected to maximize thermal transfer while minimizing microwave reflections. The cooling fluid may be selected from the group consisting of alcohol, glycerol, ethylene glycol, deionized water, a germicide, and vegetable oil. In one system, the microwave antenna may a waveguide including a dielectric filler selected to generate a field having a minimal electric field perpendicular to a surface of the tissue at a predetermined frequency. In one system, the fluid chamber has a shape configured to facilitate laminar flow of cooling fluid therethrough. The fluid chamber may be rectangular shaped. In some systems, the cooling plate is thermally conductive and substantially transparent to microwave energy.

In another system, a method of creating a tissue effect in a target tissue layer is disclosed, comprising the steps of: irradiating the target tissue layer and a first tissue layer through a skin surface with electromagnetic energy having predetermined frequency and electric field characteristics, wherein the first tissue layer is above the target tissue layer, the first tissue layer being adjacent to a surface of the skin; and generating a power loss density profile, wherein the power loss density profile has a peak power loss density in a region of the target tissue layer. In one system, the method further comprises the step of identifying a patient desiring a reduction in sweat production. In other systems, the method further comprises the step of identifying a patient desiring a reduction in cellulite, identifying a patient with hyperhidrosis, identifying a patient with telangiectasias, identifying a patient with varicose veins, or identifying a patient desiring hair removal. In another system, the method further comprises the step of removing heat from the first tissue layer. In one system, the method further comprises the step of removing heat from the tissue layer. In one system, the tissue effect comprises a lesion. The lesion may have an origin in the target tissue layer. In one system, the origin of the lesion is in the region of the target tissue layer having the peak power loss density. In one system, the method further comprises the step of removing sufficient heat from the first layer to prevent the lesion from growing into the first layer, wherein the step of removing heat from the first tissue layer comprises cooling the skin surface. In one system, the target tissue layer comprises the dermis, a deep layer of the dermis, or a glandular layer. In one system, the electromagnetic energy has an electric field component which is substantially parallel to at least a portion of the skin surface. The electromagnetic energy has an electric field component which is parallel to at least a portion of the skin surface. In some systems, the electromagnetic energy radiates in a TE₁₀ mode or TEM mode. In some systems, the electromagnetic energy has a frequency in the range between about 4GHz and 10Ghz, between 5GHz and 6.5GHz, or approximately 5.8 GHz. In one system, the electromagnetic energy generates heat in the target tissue by dielectric heating. In one system, the power loss density is generated by a standing wave pattern in the target tissue layer and the first tissue layer. In one system, the standing wave pattern has a constructive interference peak in the region of the target tissue layer. The standing wave pattern has a constructive interference minimum in the first tissue layer.

In another system, disclosed is a method of creating a lesion in a target tissue layer in the absence of cooling, wherein the target tissue layer is below a first tissue layer, the first tissue layer being adjacent to a skin surface, the method comprising the steps of: irradiating the target tissue layer and a first tissue layer through a skin surface with electromagnetic energy having predetermined frequency and electric field characteristics, wherein the first tissue layer is above the target tissue layer, the first tissue layer being adjacent to a surface of the skin; and generating a power loss density profile, wherein the power loss density profile has a peak power loss density in a region of the target tissue layer. In one system, the lesion has an origin in the target tissue layer. In some systems, the target tissue layer comprises the dermis, a deep layer of the dermis, or a glandular layer. In one system, the electromagnetic energy has an electric field component which is substantially parallel to at least a portion of the skin surface. In one system, the electromagnetic energy has an electric field component which is substantially parallel to at least a portion of the skin surface. In one system, the electromagnetic energy has an electric field component which is parallel to at least a portion of the skin surface. In some systems, the electromagnetic energy radiates in a TE₁₀ mode or a TEM mode. In some systems, the electromagnetic energy has a frequency in the range of between about 4GHz and 10GHz, 5GHz and 6.5GHz, or approximately 5.8GHz. The electromagnetic energy may generate heat in the target tissue by dielectric heating. In one system, the power loss density is generated by a standing wave pattern in the target tissue layer and the first tissue layer. The standing wave pattern has a constructive interference peak in the region of the target tissue layer or in the first tissue layer. In one system, the origin of the lesion is in the region of the target tissue layer having the peak power loss density.

In another system, disclosed is a method of generating heat in a target tissue layer wherein the heat is sufficient to create a lesion in or proximate to the target tissue layer, wherein the target tissue layer is below a first tissue layer, the first tissue layer being adjacent to a skin surface, the method comprising the steps of: irradiating the target tissue layer and the first tissue layer through the skin surface with electromagnetic energy having predetermined frequency and electric field characteristics; and generating a power loss density profile wherein the power loss density profile has a peak power loss density in a region of the target tissue layer. In one system, the lesion has an origin in the target tissue layer. In some systems, the target tissue layer comprises the dermis, a deep layer of the dermis or a glandular layer. In one system, the method further comprises the step of removing heat from the first tissue layer. In one system, the method further comprises the step of removing sufficient heat from the first layer to prevent the lesion from growing into the first layer, wherein the step of removing heat from the first tissue layer comprises cooling the skin surface. In some systems, the electromagnetic energy has an electric field component which is substantially parallel to at least a portion of the skin surface, while in other systems, the electric field component is parallel to at least a portion of the skin surface. In some systems, the electromagnetic energy radiates in a TE₁₀ mode or TEM mode. In some systems, the electromagnetic energy has a frequency in the range of between about 4GHz and 10GHz, 5GHz and 6.5GHz, or approximately 5.8 GHz. In one system, the electromagnetic energy generates heat in the target tissue by dielectric heating. In one system, the power loss density is generated by a standing wave pattern in the target tissue layer and the first tissue layer. In one system, the power loss density is generated by a standing wave pattern in the target tissue layer and the first tissue layer. In some systems, the standing wave pattern has a constructive interference peak in the region of the target tissue layer or in the first tissue layer. In one system, the origin of the lesion is in the region of the target tissue layer having the peak power loss density. In one system, the heat is sufficient to destroy bacteria within the target tissue. In some systems, the method further comprises the step of identifying a patient with acne or identifying a patient desiring a reduction of sweat production.

In another system, disclosed is a method of generating heat in a target tissue layer in the absence of cooling wherein the heat is sufficient to create a tissue effect in or proximate to the target tissue layer, wherein the target tissue layer is below a first tissue layer, the first tissue layer being adjacent to a skin surface, the method comprising the steps of: irradiating the target tissue layer and the first tissue layer through the skin surface with electromagnetic energy having predetermined frequency and electric field characteristics; and generating a power loss density profile wherein the power loss density profile has a peak power loss density in a region of the target tissue layer. In one system, the heat is sufficient to generate a lesion having an origin in the target tissue layer. In some systems, the target tissue layer comprises the dermis, deep layer of the dermis or glandular layer. In one system, the electromagnetic energy has an electric field component which is substantially parallel to at least a portion of the skin surface, while in another system, the electric field component is parallel to at least a portion of the skin surface. In some systems, the electromagnetic energy radiates in a TE₁₀ mode or a TEM mode. In some systems, the electromagnetic energy has a frequency in the range of between about 4GHz and 10GHz, 5GHz and 6.5GHz, or about 5.8 GHz. In one system, the electromagnetic energy generates heat in the target tissue by dielectric heating. In one system, the power loss density is generated by a standing wave pattern in the target tissue layer and the first tissue layer. In some systems, the standing wave pattern has a constructive interference peak in the region of the target tissue layer or in the first tissue layer. In one system, the standing wave pattern has a constructive interference minimum in the first tissue layer. In one system, the origin of the lesion is in the region of the target tissue layer having the peak power loss density.

Also disclosed herein, in another system is a method of generating a temperature profile in tissue wherein the temperature profile has a peak in a target tissue layer, wherein the target tissue layer is below a first tissue layer, the first tissue layer being adjacent to a skin surface, the method comprising the steps of: irradiating the target tissue layer and the first tissue layer through the skin surface with electromagnetic energy having predetermined frequency and electric field characteristics; and generating a power loss density profile wherein the power loss density profile has a peak power loss density in a region of the target tissue layer. In some systems, the target tissue layer comprises the dermis, a deep layer of the dermis or a glandular layer. In one system, the method further comprises the step of removing heat from the first tissue layer. In one system, the electromagnetic energy has an electric field component which is substantially parallel to at least a portion of the skin surface. In one system, the electromagnetic energy has an electric field component which is parallel to at least a portion of the skin surface. In some systems, the electromagnetic energy radiates in a TE₁₀ mode or TEM mode. In some systems, the electromagnetic energy has a frequency in the range of between about 4GHz and 10GHz, between about 5 GHz and 6.5GHz, or of approximately 5.8GHz. In one system, the electromagnetic energy generates heat in the target tissue by dielectric heating. In one system, the power loss density is generated by a standing wave pattern in the target tissue layer and the first tissue layer. The standing wave pattern has a constructive interference peak in the region of the target tissue layer. The standing wave pattern may have a constructive interference minimum in the first tissue layer. In one system, the peak temperature is in the region of the target tissue layer having the peak power loss density.

In another system, disclosed herein is a method of generating a temperature profile in tissue in the absence of cooling wherein the temperature profile has a peak in a target tissue layer, wherein the target tissue layer is below a first tissue layer, the first tissue layer being adjacent to a skin surface, the method comprising the steps of: irradiating the target tissue layer and the first tissue layer through the skin surface with electromagnetic energy having predetermined frequency and electric field characteristics; and generating a power loss density profile wherein the power loss density profile has a peak power loss density in a region of the target tissue layer. In some systems, the target tissue layer comprises the dermis, a deep layer of the dermis or a glandular layer. In some systems, the electromagnetic energy has an electric field component which is substantially parallel to at least a portion of the skin surface or which is parallel to at least a portion of the skin surface. In some systems, the electromagnetic energy radiates in a TE₁₀ mode or a TEM mode. In some systems, the electromagnetic energy has a frequency in the range of between about 4GHz and 10GHz, 5GHz and 6.5GHz or approximately 5.8GHz. In one system, the electromagnetic energy generates heat in the target tissue by dielectric heating. In one system, the power loss density is generated by a standing wave pattern in the target tissue layer and the first tissue layer. The standing wave pattern has a constructive interference peak in the region of the target tissue layer. The standing wave pattern may have a constructive interference minimum in the first tissue layer. In one system, the peak temperature is in the region of the target tissue layer having the peak power loss density.

In another system, disclosed is a method of creating a lesion in a first layer of tissue, the first layer having an upper portion adjacent an external surface of the skin and a lower portion adjacent a second layer of the skin, the method comprising the steps of: exposing the external surface of the skin to microwave energy having a predetermined power, frequency, and electric field orientation; generating an energy density profile having a peak in the lower portion of the first layer; and continuing to expose the external surface of the skin to the microwave energy for a time sufficient to create a lesion, wherein the lesion begins in the peak energy density region. In one system, the first layer of skin has a first dielectric constant and the second layer of skin has a second dielectric constant, wherein the first dielectric constant is greater than the second dielectric constant. In one system, the first layer has a dielectric constant greater than about 25 and the second layer has a dielectric constant less than or equal to about 10. In one system, the first layer comprises at least a portion of a dermis layer. In some systems, the second layer comprises at least a portion of a hypodermis layer or at least a portion of a glandular layer.

Also disclosed herein is a method of creating a lesion in the skin wherein the skin has at least an external surface, a first layer below the external surface and a second layer, the method comprising the steps of: positioning a device adapted to radiate electromagnetic energy adjacent the external surface; radiating electromagnetic energy from the device, the microwave energy having an electric field component which is substantially parallel to a region of the external surface; and generating a standing wave pattern in the first layer, the standing wave pattern having a constructive interference peak in the first layer, wherein a distance from the constructive interference peak to the skin surface is greater than a distance from the constructive interference peak to an interface between the first layer and the second layer. In one system, the electromagnetic energy comprises microwave energy. In one system, the constructive interference peak is adjacent the interface. In one system, the first layer has a first dielectric constant and the second layer has a second dielectric constant, wherein the first dielectric constant is greater than the second dielectric constant. In one system, the first layer has a dielectric constant greater than about 25 and the second layer has a dielectric constant less than or equal to about 10. In one system, the first layer comprises at least a portion of a dermis layer. In some systems, the second layer comprises at least a portion of a hypodermis layer or at least a portion of a glandular layer.

In another system, disclosed is a method of creating a temperature gradient in the skin wherein the skin has at least an external surface, a first layer below the external surface and a second layer, the method comprising the steps of: positioning a device adapted to radiate electromagnetic energy adjacent the external surface; radiating electromagnetic energy from the device, the microwave energy having an electric field component which is substantially parallel to a region of the external surface; and generating a standing wave pattern in the first layer, the standing wave pattern having a constructive interference peak in the first layer, wherein a distance from the constructive interference peak to the skin surface is greater than a distance from the constructive interference peak to an interface between the first layer and the second layer.

In another system, disclosed is a method of creating a lesion in a dermal layer of the skin, the dermal layer having an upper portion adjacent an external surface of the skin and a lower portion adjacent a subdermal layer of the skin, the method comprising the steps of: exposing the external surface to microwave energy having a predetermined power, frequency, and electric field orientation; generating a peak energy density region in the lower portion of the dermal layer; and continuing to radiate the skin with the microwave energy for a time sufficient to create a lesion, wherein the lesion begins in the peak energy density region.

In another system, disclosed is a method of creating a lesion in a dermal layer of the skin wherein the skin has at least a dermal layer and a subdermal layer, the method comprising the steps of: positioning a device adapted to radiate microwave energy adjacent an external surface of the skin; and radiating microwave energy having an electric field component which is substantially parallel to a region of the external surface of the skin above the dermal layer, wherein the microwave energy has a frequency which generates a standing wave pattern in the dermal layer, the standing wave pattern having a constructive interference peak in the dermal layer proximal to an interface between the dermal layer and the subdermal layer.

In another system, disclosed herein is a method of creating a lesion in a dermal layer of the skin wherein the skin has at least a dermal layer and a subdermal layer, the method comprising the steps of: positioning a device adapted to radiate microwave energy adjacent an external surface of the skin; radiating microwave energy having an electric field component which is substantially parallel to a region of the external surface of the skin above the dermal layer, wherein the microwave energy has a frequency which generates a standing wave pattern in the dermal layer, the standing wave pattern having a constructive interference peak in the dermal layer proximal to an interface between the dermal layer and the subdermal layer; and heating the lower portion of the dermal region using the radiated microwave energy to create the lesion. In one system, a center of the lesion is positioned at the constructive interference peak.

In another system, disclosed is a method of heating a tissue structure located in or near a target tissue layer, wherein the target tissue layer is below a first tissue layer, the first tissue layer being adjacent a skin surface, the method comprising the steps of: irradiating the target tissue layer and the first tissue layer through the skin surface with electromagnetic energy having predetermined frequency and electric field characteristics; and generating a power loss density profile wherein the power loss density profile has a peak power loss density in a region of the target tissue layer. In one system, the tissue structure comprises a sweat gland. In one system, heating the tissue structure is sufficient to destroy a pathogen located in or near the tissue structure. The pathogen may be bacteria. In some systems, the tissue structure is a sebaceous gland or at least a portion of a hair follicle. In some systems, the tissue structure may be selected from the group consisting of: telangiectasias, cellulite, varicose veins, and nerve endings. In one system, heating the tissue structure is sufficient to damage the tissue structure. In one system, the heat generates a lesion having an origin in the target tissue layer. The lesion grows to include the tissue structure. In one system, the method further comprises the step of removing sufficient heat from the first layer to prevent the lesion from growing into the first layer. Removing sufficient heat from the first layer may comprise cooling the skin surface. In some systems, the target tissue layer may comprise a deep layer of the dermis or a glandular layer. In some systems, the electromagnetic energy has an electric field component which is substantially parallel to at least a portion of the skin surface or is parallel to at least a portion of the skin surface. In some systems, the electromagnetic energy radiates in a TE₁₀ mode or TEM mode. In some systems, the electromagnetic energy has a frequency in the range of between about 4GHz and 10GHz, 5GHz and 6.5GHz, or approximately 5.8GHz. In one system, the electromagnetic energy generates heat in the target tissue by dielectric heating. In one system, the power loss density is generated by a standing wave pattern in the target tissue layer and the first tissue layer. The standing wave pattern has a constructive interference peak in the region of the target tissue layer. The standing wave pattern may have a constructive interference minimum in the first tissue layer. In one system, the origin of the lesion is in the region of the target tissue layer having the peak power loss density. In one system, the lesion continues to grow through conductive heating after electromagnetic energy is no longer applied. In one system, the target tissue structure is heated primarily as a result of the conductive heating.

In another system, disclosed herein is a method of raising the temperature of at least a portion of a tissue structure located below an interface between a dermal layer and subdermal layer in skin, the dermal layer having an upper portion adjacent an external surface of the skin and a lower portion adjacent a subdermal region of the skin, the method comprising the steps of: radiating the skin with microwave energy having a predetermined power, frequency and e-field orientation; generating a peak energy density region in the lower portion of the dermal layer; initiating a lesion in the peak energy density region by dielectric heating of tissue in the peak energy density region; enlarging the lesion, wherein the lesion is enlarged, at least in part, by conduction of heat from the peak energy density region to surrounding tissue; removing heat from the skin surface and at least a portion of the upper portion of the dermal layer; and continuing to radiate the skin with the microwave energy for a time sufficient to extend the lesion past the interface and into the subdermal layer. In one system, the tissue structure comprises a sweat gland.

Also disclosed herein in another system is a method of raising the temperature of at least a portion of a tissue structure located below an interface between a dermal layer and a subdermal layer of skin, wherein the dermal layer has an upper portion adjacent an external surface of the skin and a lower portion adjacent a subdermal region of the skin, the method comprising the steps of: positioning a device adapted to radiate microwave energy adjacent the external surface of the skin; radiating microwave energy having an electric field component which is substantially parallel to a region of the external surface above the dermal layer, wherein the microwave energy has a frequency which generates a standing wave pattern in the dermal layer, the standing wave pattern having a constructive interference peak in the lower portion of the dermal layer; creating a lesion in the lower portion of the dermal region by heating tissue in the lower portion of the dermal region using the radiated microwave energy; removing heat from the skin surface and at least a portion of the upper portion of the dermal layer to prevent the lesion from spreading into the upper portion of the dermal layer; and ceasing the radiating after a first predetermined time, the predetermined time being sufficient to raise the temperature of the tissue structure. In some systems, the first predetermined time comprises a time sufficient to deposit enough energy in said lower portion of the dermal layer to enable said lesion to spread into the subdermal region or a time sufficient to enable heat generated by said radiation to spread to the tissue structure. In one system, the step of removing heat further comprises continuing to remove heat for a predetermined time after the step of ceasing said radiating. In one system, the constructive interference peak is located on a dermal side of the interface between the dermal layer and the subdermal layer. In one system, the lesion starts at the constructive interference peak.

In another system, disclosed herein is a method of controlling the application of microwave energy to tissue, the method comprising the steps of: generating a microwave signal having predetermined characteristics; applying the microwave energy to tissue, through a microwave antenna and a tissue interface operably connected to the microwave antenna; supplying a vacuum pressure to the tissue interface; and supplying cooling fluid to the tissue interface. In some systems, the microwave signal has a frequency in the range of between about 4GHz and 10GHz, between about 5GHz and 6.5GHz, or approximately 5.8 GHz. In one system, the microwave antenna comprises an antenna configured to radiate electromagnetic radiation polarized such that an E-field component of the electromagnetic radiation is substantially parallel to an outer surface of the tissue. The microwave antenna may comprise a waveguide antenna. In some systems, the microwave antenna comprises an antenna configured to radiate in TE₁₀ mode or in TEM mode. In one system, the tissue interface is configured to engage and hold skin. The skin may be in the axillary region. In one system, the microwave antenna comprises an antenna configured to radiate electromagnetic radiation polarized such that an E-field component of the electromagnetic radiation is parallel to an outer surface of the tissue. In one system, the tissue interface comprises a cooling plate and a cooling chamber positioned between the cooling plate and the microwave antenna. In one system, the cooling plate has a dielectric constant between about 2 and 15. In one system, the vacuum source is configured to supply vacuum pressure to the tissue interface. In some systems, the vacuum pressure is between about 400 mmHg to about 750mmHg, or about 650 mmHg. In one system, the cooling source is configured to supply a coolant to the tissue interface. In one system, the coolant is a cooling fluid. In some systems, the cooling fluid has a dielectric constant of between about 70 and 90, or about 80, or between about 2 and 10, or about 2. In some systems, the cooling fluid has a temperature of between about -5°C and 40 °C or between about 10°C and 25 °C. In one system, the cooling fluid has a temperature of about 22 °C. In some systems, the cooling fluid has a flow rate through at least a portion of the tissue interface of between about 100mL and 600mL per second or between about 250mL and 450mL per second. In one system, the cooling fluid is configured to flow through the tissue interface at a velocity of between about 0.18 and 0.32 meters per second. In one system, the cooling fluid is selected from the group consisting of glycerin, vegetable oil, isopropyl alcohol, and water, while in another system, the cooling fluid is selected from the group consisting of water and water mixed with an alcohol.

Also disclosed, in another system, is a method of positioning tissue prior to treating the tissue using radiated electromagnetic energy, the method comprising positioning a tissue interface adjacent a skin surface; engaging the skin surface in a tissue chamber of the tissue interface; substantially separating a layer comprising at least one layer of the skin from a muscle layer below the skin; and holding the skin surface in the tissue chamber. In one system, the tissue interface comprises a tissue chamber, the tissue chamber having at least one wall and a tissue-contacting surface. In one system, at least a portion of the tissue surface comprises a cooling plate positioned in the tissue chamber. In one system, the tissue chamber has an aspect ratio in the range of between about 1:1 and 3:1, while in another system, the tissue chamber has an aspect ratio of about 2:1. In one system, the tissue chamber has a tissue acquisition angle between the wall and the tissue surface, the tissue acquisition angle being in the range of between about 2 degrees and approximately 45 degrees, while in another system, the tissue acquisition angle is in the range of between about 5 degrees and approximately 20 degrees. In one system, the tissue chamber has a tissue acquisition angle between the wall and the tissue surface, the tissue acquisition angle is about 20 degrees.

The various systems described herein can also be combined to provide further systems. Related methods, apparatuses and systems utilizing microwave and other types of therapy, including other forms of electromagnetic radiation, and further details on treatments that may be made with such therapies, are described in the above-referenced provisional applications to which this application claims priority, the entireties of each of which are: U.S. Provisional Patent Application Ser. No. 61/013,274, also published as PCT/US08/60929 entitled "Methods, Delivery and Systems for Non-Invasive Delivery of Microwave Therapy," filed December 12, 2007, and U.S. Provisional Patent Application Ser. No. 61/045,937, also published as PCT/US08/60922 entitled "Systems and Methods for Creating an Effect Using Microwave Energy in Specified Tissue," filed April 17, 2008.

While this invention has been particularly shown and described with references to systems thereof, it will be understood by those skilled in the art that various changes in form and details may be made therein without departing from the scope of the invention. For all of the systems described above, the steps of the methods need not be performed sequentially.

## Claims

1. A system for the application of microwave energy to skin, comprising:
a signal generator (304) adapted to generate a microwave signal having predetermined characteristics;
an applicator (320) connected to the signal generator (304), adapted to apply microwave energy to skin and to be positioned adjacent an outer surface (1306) of the skin, the applicator (320) comprising one or more microwave antennas and a tissue interface;
a vacuum source (308) connected to the tissue interface (336), the tissue interface (336) having a tissue chamber (338) coupled to the vacuum source (308) to engage the skin therein;
a cooling source connected to said tissue interface (336);
a controller (302) adapted to control the signal generator (304), the vacuum source (308), and the coolant source (310); wherein
the one or more microwave antenna (358) is configured to radiate the microwave energy into the skin with an electric field component substantially parallel to the outer surface (1306) of the skin and having a frequency and electric field orientation which generates a standing wave pattern having a constructive interference peak in a target tissue, thereby creating a peak temperature at the target tissue, creating a lesion core (1321) by heating the target tissue using the radiated microwave energy, the target tissue being either in the lower portion of a dermal skin layer (1305) and the standing wave pattern is created by the reflection of at least a portion of the microwave energy off an interface (1308) between the dermal skin layer (1305) and a sub-dermal skin layer (1303) or in a glandular layer (1331) and the standing wave pattern is created by the reflection of at least a portion of the microwave energy off an interface (1333) between the glandular layer (1331) and a sub-dermal skin layer (1303).

2. A system according to claim 1, wherein the one or more microwave antenna (358) is configured to radiate the microwave energy into the skin with an electric field component substantially parallel to at least one interface between tissue layers within the skin.

3. A system according to any preceding claim, wherein the tissue interface (336) comprises a cooling plate (340) the cooling source being configured to supply a coolant to the cooling plate (340).

4. A system according to claim 3, wherein the tissue interface (336) comprises a tissue chamber (338) configured to acquire at least a portion of skin tissue and bring a skin surface into thermal contact with the cooling plate (340) in the tissue interface.

5. A system according to any preceding claim, wherein the cooling source removes heat from the outer surface of the skin (1306) and at least an upper portion of a dermal skin layer (1305) to prevent the heat from spreading into an upper portion of a dermal skin layer (1305).

6. A system of any preceding claim, wherein the microwave signal has a frequency in the range of: between about 4 GHz and about 10 GHz; or between 5 GHz and about 6.5 GHz; or about 5.8 GHz.

7. A system of any preceding claim, wherein the controller (302) is configured such that the system delivers energy such that a power loss density peak profile is created in the target tissue.

8. A system of any preceding claim, wherein the glandular layer (1331) comprises sweat glands (1341).

9. A system of any preceding claim, wherein the target tissue comprises at least one of axillary tissue, collagen, hair follicles (1344), cellulite, eccrine glands, apocrine glands, sebaceous glands, wound tissue and spider veins.

## Patentansprüche

1. System zur Anwendung von Mikrowellenenergie auf die Haut, umfassend:
einen Signalgenerator (304), ausgelegt zum Erzeugen eines Mikrowellensignals, das vorbestimmte Merkmale aufweist;
eine Anwendungsvorrichtung (320), die mit dem Signalgenerator (304) verbunden und ausgelegt ist, um Mikrowellenenergie auf die Haut anzuwenden und angrenzend an eine äußere Hautoberfläche (1306) angeordnet zu werden, wobei die Anwendungsvorrichtung (320) eine oder mehrere Mikrowellenantennen und eine Gewebegrenzfläche umfasst;
eine Unterdruckquelle (308), die mit der Gewebegrenzfläche (336) verbunden ist, wobei die Gewebegrenzfläche (336) eine Gewebekammer (338) aufweist, die mit der Unterdruckquelle (308) verbunden ist, um die Haut darin in Eingriff zu nehmen;
eine Kühlquelle, verbunden mit der Gewebegrenzfläche (336);
eine Steuerung (302), ausgelegt zum Steuern des Signalgenerators (304), der Unterdruckquelle (308) und der Kühlmittelquelle (310); wobei
die eine oder mehreren Mikrowellenantennen (358) konfiguriert sind, um die Mikrowellenenergie in die Haut auszustrahlen, wobei eine elektrische Feldkomponente im Wesentlichen parallel ist zur äußeren Hautoberfläche (1306) und eine Frequenz und Ausrichtung des elektrischen Felds aufweist, die ein Stehwellenmuster mit einer konstruktiven Interferenzspitze in einem Zielgewebe hat, wodurch eine Spitzentemperatur am Zielgewebe erzeugt wird, die einen Läsionskern (1321) erzeugt durch das Erwärmen des Zielgewebes unter Verwendung der ausgestrahlten Mikrowellenenergie, wobei das Zielgewebe entweder im unteren Abschnitt einer dermalen Hautschicht (1305) ist und das Stehwellenmuster durch die Reflexion mindestens eines Anteils der Mikrowellenenergie von einer Grenzfläche (1308) zwischen der dermalen Hautschicht (1305) und einer subdermalen Hautschicht (1303) erzeugt wird, oder in einer glandulären Schicht (1331) ist und das Stehwellenmuster durch die Reflexion mindestens eines Anteils der Mikrowellenenergie von einer Grenzfläche (1333) zwischen der glandulären Schicht (1331) und einer subdermalen Hautschicht (1303) erzeugt wird.

2. System nach Anspruch 1, wobei die eine oder die mehreren Mikrowellenantennen (358) konfiguriert sind, um die Mikrowellenenergie mit einer elektrischen Feldkomponente in die Haut auszustrahlen, die im Wesentlichen parallel ist zu mindestens einer Grenzfläche zwischen Gewebeschichten innerhalb der Haut.

3. System nach einem der vorhergehenden Ansprüche, wobei die Gewebegrenzfläche (336) eine Kühlplatte (340) umfasst, wobei die Kühlmittelquelle konfiguriert ist, der Kühlplatte (340) ein Kühlmittel zuzuführen.

4. System nach Anspruch 3, wobei die Gewebegrenzfläche (336) eine Gewebekammer (338) umfasst, die konfiguriert ist, um mindestens einen Abschnitt des Hautgewebes aufzunehmen und eine Hautoberfläche in thermischen Kontakt mit der Kühlplatte (340) in der Gewebegrenzfläche zu bringen.

5. System nach einem der vorhergehenden Ansprüche, wobei die Kühlquelle Wärme von der äußeren Hautoberfläche (1306) und mindestens einem oberen Abschnitt einer dermalen Hautschicht (1305) entfernt, um zu verhindern, dass die Wärme sich in einen oberen Abschnitt der dermalen Hautschicht (1305) verbreitet.

6. System nach einem der vorhergehenden Ansprüche, wobei das Mikrowellensignal eine Frequenz im folgenden Bereich aufweist: zwischen etwa 4 GHz und etwa 10 GHz; oder zwischen 5 GHz und etwa 6,5 GHz; oder etwa 5,8 GHz.

7. System nach einem der vorhergehenden Ansprüche, wobei die Steuerung (302) derart konfiguriert ist, dass das System derart Energie zuführt, dass ein Profil der Leistungsverlustdichte-Spitze in dem Zielgewebe erzeugt wird.

8. System nach einem der vorhergehenden Ansprüche, wobei die glanduläre Schicht (1331) Schweißdrüsen (1341) umfasst.

9. System nach einem der vorhergehenden Ansprüche, wobei das Zielgewebe mindestens eines von Axillargewebe, Collagen, Haarfollikeln (1344), Cellulite, ekkrinen Drüsen, apokrinen Drüsen, Talgdrüsen, Wundgewebe und Besenreisern umfasst.

## Revendications

1. Système d'application d'énergie micro-ondes à la peau, comprenant:
un générateur de signal (304) adapté pour générer un signal micro-ondes ayant des caractéristiques prédéterminées;
un applicateur (320) connecté au générateur de signal (304), adapté pour appliquer l'énergie micro-ondes à la peau et pour être positionné adjacent à une surface externe (1306) de la peau, l'applicateur (320) comprenant une ou plusieurs antennes micro-ondes et une interface tissulaire ;
une source de vide (308) connectée à l'interface tissulaire (336), l'interface tissulaire (336) ayant une chambre pour tissu (338) couplée à la source de vide (308) pour y engager la peau ;
une source réfrigérante connectée à ladite interface tissulaire (336)
un contrôleur (302) adapté pour contrôler le générateur de signal (304), la source de vide (308), et la source réfrigérante (310), dans lequel
l'une ou les plusieurs antennes micro-ondes (358) sont configurées pour émettre le rayonnement d'énergie micro-ondes dans la peau avec une composante de champ électrique substantiellement parallèle à la surface externe (1306) de la peau et ayant une fréquence et une orientation de champ électrique qui génèrent un modèle d'ondes stationnaires ayant une pointe d'interférence constructive dans un tissu cible, créant ainsi une température de pointe dans le tissu cible, créant un centre de lésion (1321) en chauffant le tissu cible en utilisant l'énergie micro-ondes émise, le tissu cible étant soit dans la partie inférieure d'une couche dermique de la peau (1305) et le modèle d'onde stationnaire est créé par la réflexion d'au moins une partie de l'énergie micro-ondes d'une interface (1308) entre la couche dermique de peau (1305) et la couche hypodermique de peau (1303) ou dans une couche glandulaire (1331) et le modèle d'onde stationnaire est créé par la réflexion d'au moins une partie de l'énergie micro-ondes d'une interface (1333) entre la couche glandulaire (1331) et une couche hypodermique de peau (1303).

2. Système selon la revendication 1, dans lequel l'une ou les plusieurs antennes micro-ondes (358) sont configurées pour émettre l'énergie micro-ondes dans la peau avec une composante de champ électrique substantiellement parallèle à au moins une interface entre les couches de tissu dans la peau.

3. Système selon l'une quelconque des revendications précédentes, dans lequel l'interface tissulaire (336) comprend une plaque réfrigérante (340), la source réfrigérante étant configurée pour fournir un réfrigérant à la plaque réfrigérante (340).

4. Système selon la revendication 3, dans lequel l'interface tissulaire (336) comprend une chambre pour tissu (338) configurée pour acquérir au moins une partie du tissu de la peau et mettre une surface de la peau en contact thermique avec la plaque réfrigérante (340) dans l'interface tissulaire.

5. Système selon l'une quelconque des revendications précédentes, dans lequel la source réfrigérante supprime la chaleur de la surface externe de la peau (1306) et d'au moins une partie supérieure de la couche dermique de peau (1305) pour empêcher la chaleur de se répandre dans une partie supérieure de la couche dermique de peau (1305).

6. Système selon l'une quelconque des revendications précédentes, dans lequel le signal de micro-ondes a une fréquence dans la plage d'entre environ 4 GHz et environ 10 GHz; ou d'entre 5 GHz et environ 6,5 GHz; ou d'environ 5,8 GHz.

7. Système selon l'une quelconque des revendications précédentes, dans lequel le contrôleur (302) est configuré de telle sorte que le système fournit de l'énergie de telle sorte qu'une pointe de densité de perte de puissance est créée dans le tissu cible.

8. Système selon l'une quelconque des revendications précédentes, dans lequel la couche glandulaire (1331) comprend des glandes sudoripares (1341).

9. Système selon l'une quelconque des revendications précédentes, dans lequel le tissu cible comprend au moins l'un des éléments suivants : tissus axillaires, collagène, follicules pileux (1344), cellulite, glandes eccrines, glandes apocrines, glandes sébacées, tissu de cicatrisation, varicosités.
